(19) Europäisches Patentamt
European Patent Office
Office européen des brevets

(11) EP 4 772 186 A1

(12) **EUROPEAN PATENT APPLICATION**
published in accordance with Art. 153(4) EPC

(43) Date of publication:
**08.07.2026 Bulletin 2026/28**

(21) Application number: **24858371.8**

(22) Date of filing: **19.08.2024**

(51) International Patent Classification (IPC):
***A61K 35/747*** *(2015.01)* ***A61K 38/14*** *(2006.01)*
***A61K 31/7084*** *(2006.01)* ***A61K 38/08*** *(2019.01)*
***A61K 31/192*** *(2006.01)* ***A23C 9/13*** *(2006.01)*
***A23F 3/14*** *(2006.01)* ***A23F 5/14*** *(2025.01)*
***A23G 3/36*** *(2006.01)* ***A23G 4/12*** *(2006.01)*
***A61Q 11/00*** *(2006.01)* ***A61P 1/02*** *(2006.01)*

(52) Cooperative Patent Classification (CPC):
**A23C 9/13; A23F 3/14; A23F 5/14; A23G 3/36; A23G 4/12; A61K 31/192; A61K 31/7084; A61K 35/747; A61K 38/08; A61K 38/14; A61P 1/02; A61Q 11/00**

(86) International application number:
**PCT/CN2024/113210**

(87) International publication number:
**WO 2025/044828 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**01.09.2023 CN 202311130603**
**01.09.2023 CN 202311130641**
**01.09.2023 CN 202311130582**
**01.09.2023 CN 202311130653**
**01.09.2023 CN 202311130620**
**07.02.2024 CN 202410174811**

(71) Applicant: **Inner Mongolia Yili Industrial Group Co., Ltd.**
**Hohhot City, Inner Mongolia 010000 (CN)**

(72) Inventors:
- **LAN, Hanglian**
  **Huhhot, Inner Mongolia 010000 (CN)**
- **HUNG, Wei-Lian**
  **Huhhot, Inner Mongolia 010000 (CN)**
- **HE, Jian**
  **Huhhot, Inner Mongolia 010000 (CN)**
- **ZHAO, Wen**
  **Huhhot, Inner Mongolia 010000 (CN)**
- **ZENG, Zhaozhong**
  **Huhhot, Inner Mongolia 010000 (CN)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

# (54) USE AND COMPOSITION OF POSTBIOTIC OF LACTOBACILLUS PARACASEI ET-22

(57) Use and composition of a postbiotic of *Lactobacillus paracasei* ET-22, and use of a lysate derived from *Lactobacillus paracasei* ET-22 in freshening breath and/or alleviating and inhibiting halitosis. The lysate is derived from heat-inactivated bacterial cells of *Lactobacillus paracasei* ET-22, the lysate at least includes 2-hydroxy-3-phenylpropionic acid, NAD and a polypeptide, and the polypeptide has a structure represented by Formula 1. The postbiotic of and the lysate derived from *Lactobacillus paracasei* ET-22 can significantly reduce the content of volatile sulfur compounds (VSCs) in breath and volatile organic compounds (VOCs) in saliva within the oral cavity, and lower the content of characteristic flavor compound such as indole, propanoic acid and hexanoic acid, and can alter the oral flora and inhibit the growth of oral biofilms and pathogenic bacteria, thus having the effects of freshening breath and alleviating and inhibiting halitosis.



Processed by Luminess, 75001 PARIS (FR)

(Cont. next page)

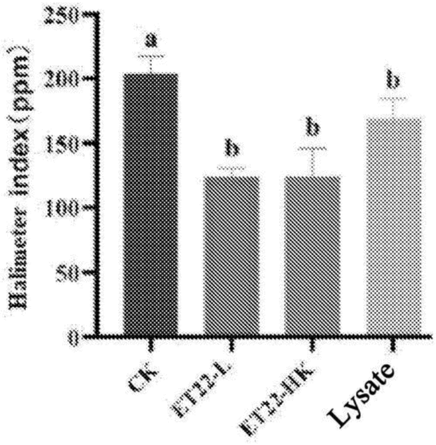
Halimeter index (ppm)
250
200
150
100
50
0
a
b
b
b
CK
ET22-L
ET22-HK
Lysate

FIG. 1

## Description

### TECHNICAL FIELD

This application relates to the use and composition of postbiotic from *Lactobacillus paracasei* ET-22, and pertains to the field of microbial technology.

### BACKGROUND

Currently, postbiotics, as a hot research field, have attracted much attention. In 2021, the International Scientific Association for Probiotics and Prebiotics (ISAPP) published a consensus statement on postbiotics, defining postbiotics as a preparation of inanimate microorganisms and/or their components that confers a health benefit on a host. As the derivative of prebiotics and probiotics, postbiotics have multiple potential benefits. They can regulate the intestinal flora, enhance intestinal barrier function, modulate intestinal inflammatory responses, etc., thereby exerting positive effects on human intestinal health. Furthermore, the biological activity of postbiotics is not limited to the gut; they also possess biological activities such as inhibition of oral pathogenic bacteria and regulation of pulmonary inflammatory responses. With the deepening understanding of gut health and the microbiome, postbiotics are considered a potential functional food and health management tool, receiving particular attention from the industry. Currently, reports on the biological activity of postbiotics mainly refer to the biological activity of probiotics or bacterial cells. *Lactobacillus paracasei* ET-22 belongs to a relatively typical postbiotic strain. Preliminary studies have found that *Lactobacillus paracasei* ET-22 has potential in regulating blood pressure and sleep quality, but its specific product form and preparation method can affect its performance, such as thermal stability.

Halitosis is a common oral health concern, affecting not only personal health levels but also psychological and social life. Studies have shown that some probiotics and their postbiotics possess functions of inhibiting the growth of oral pathogenic bacteria and regulating immune. For example, *Lactobacillus paracasei* L9 and *Bifidobacterium animalis* A6 can effectively alleviate gingival bleeding, periodontal inflammatory infiltration, and alveolar bone resorption, and reduce the relative proportion of pathogenic genera such as *Streptococcus, Fusobacterium,* and *Veillonella,* thereby preventing periodontitis; other studies have proven that *Lactobacillus paracasei* ET-22 extracted from infant intestines has a function of inhibiting the growth of *Streptococcus mutans* biofilm and the synthesis of bacterial water-soluble and water-insoluble polysaccharides, effectively preventing dental caries.

The efficacy of *Lactobacillus paracasei* ET-22 viable bacteria and their postbiotics in improving halitosis has also been confirmed. These viable bacteria can adhere to and colonize in the oral cavity, competing with halitosis-causing pathogenic bacteria for adhesion sites, thereby further inhibiting the growth of pathogenic bacteria and improving halitosis. However, postbiotics cannot adhere or colonize, and their active components remain unknown.

### SUMMARY

The present invention provides the use and composition of postbiotic from *Lactobacillus paracasei* ET-22, which can significantly reduce the content of volatile sulfur compounds (VSCs) in breath and volatile organic compounds (VOCs) in saliva within the oral cavity, and lower the content of characteristic flavor compounds such as indole, propanoic acid, and hexanoic acid; and can alter the oral flora, and inhibit the growth of oral biofilm and pathogenic bacteria, thereby having the effect of freshening breath and alleviating and inhibiting halitosis.

The present invention provides the use of a lysate derived from *Lactobacillus paracasei* ET-22 in freshening breath and/or alleviating and inhibiting halitosis. The lysate is derived from heat-inactivated bacterial cells of *Lactobacillus paracasei* ET-22. The lysate at least includes 2-hydroxy-3-phenylpropionic acid, NAD, and a polypeptide. The polypeptide has the structure shown in Formula 1:

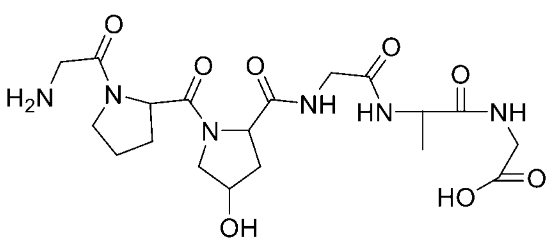

Formula 1.

Optionally, based on the total volume of the lysate, the mass of 2-hydroxy-3-phenylpropionic acid is not less than 145 mg/L, the mass of NAD is not less than 0.17 mg/L, and the mass of the polypeptide is not less than 0.24 mg/L.

Optionally, the lysate further includes one or more selected from 6-hydroxyhexanoic acid, 3-aminoglutaric acid, 2-hydroxy-3-phenylpropionic acid, 2-hydroxy-3-(4-hydroxyphenyl)propionic acid, and Asp-Phe.

Optionally, the preparation method of the lysate includes the following steps: performing liquid culture of *Lactobacillus paracasei* ET-22, stopping fermentation upon reaching a logarithmic growth phase, and collecting bacterial cells; resuspending the collected bacterial cells in sterile water, performing heat inactivation, and collecting supernatant to obtain the lysate.

Optionally, the heat inactivation is performed at a temperature of 95-150°C for 1-15 min.

The present invention also provides a peptide having breath-freshening and/or halitosis-alleviating and inhibiting effects. The peptide has the structure shown in Formula 1:

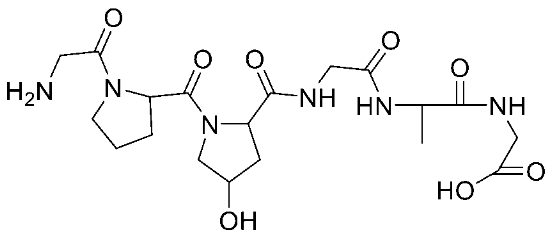

Formula 1.

Optionally, the peptide is derived from a postbiotic of *Lactobacillus paracasei* ET-22.

The present invention also provides a composition for freshening breath and/or alleviating and inhibiting halitosis, including a lysate, where the lysate is derived from heat-inactivated bacterial cells of *Lactobacillus paracasei* ET-22, the lysate at least includes 2-hydroxy-3-phenylpropionic acid, NAD, and a polypeptide, and the polypeptide has the structure shown in Formula 1:

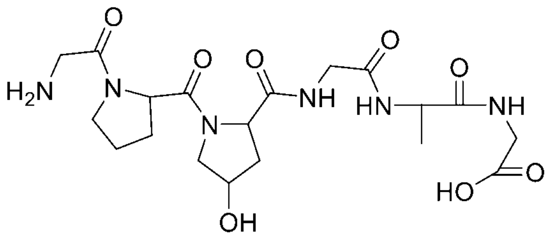

Formula 1;

or, including nicotinamide adenine dinucleotide; or, including 2-hydroxy-3-phenylpropionic acid; or, including the peptide as described above; or, including peptidoglycan, where the peptidoglycan is derived from heat-inactivated bacterial cells of *Lactobacillus paracasei* ET-22.

Optionally, the composition is one or more of a food composition, a pharmaceutical composition, and an oral cleaning composition.

Optionally, the food composition includes one or more of dairy beverage, tea, coffee, chewing gum, tooth-cleaning candy, tablet candy, and pet jerky.

Optionally, the oral cleaning composition includes one or more of toothpaste, tooth-cleaning powder, mouthwash, breath freshening spray, fluoride varnish, denture cleaner, toothbrush, interdental brush, dental floss, oral swab, pet dental gel, pet hairball paste, and pet dental chew.

Optionally, in the composition, the content of nicotinamide adenine dinucleotide is not less than 100 μg/mL; or, the content of 2-hydroxy-3-phenylpropionic acid is not less than 100 μg/mL; or, the content of the peptide as described above is not less than 100 μg/mL.

Optionally, the composition further includes one or more of an excipient, a diluent, a carrier, and an additive.

The present invention also provides the use of nicotinamide adenine dinucleotide in freshening breath and/or alleviating and inhibiting halitosis.

Optionally, the nicotinamide adenine dinucleotide is derived from a postbiotic of *Lactobacillus paracasei* ET-22.

The present invention also provides the use of nicotinamide adenine dinucleotide in reducing the content of volatile sulfur compounds in breath and volatile organic compounds in saliva within an oral cavity.

The present invention also provides the use of nicotinamide adenine dinucleotide in reducing the content of characteristic flavor compounds.

The present invention also provides the use of 2-hydroxy-3-phenylpropionic acid in freshening breath and/or alleviating and inhibiting halitosis.

Optionally, the 2-hydroxy-3-phenylpropionic acid is derived from a postbiotic of *Lactobacillus paracasei* ET-22.

The present invention also provides the use of 2-hydroxy-3-phenylpropionic acid in reducing the content of characteristic flavor compounds in the oral cavity.

The present invention also provides the use of 2-hydroxy-3-phenylpropionic acid in reducing the amount and thickness of biofilm in the oral cavity.

The present invention also provides the use of the peptide as shown in Formula 1 in freshening breath and/or alleviating and inhibiting halitosis.

The present invention also provides the use of the peptide as shown in Formula 1 in reducing the content of volatile organic compounds in the oral cavity and/or inhibiting the growth of microorganisms in the oral cavity.

The present invention also provides the use of peptidoglycan in freshening breath and/or alleviating and inhibiting halitosis, where the peptidoglycan is derived from heat-inactivated bacterial cells of *Lactobacillus paracasei* ET-22.

Optionally, the extraction method of the peptidoglycan includes the following steps: step 1: performing liquid culture of *Lactobacillus paracasei* ET-22, stopping fermentation upon reaching a logarithmic growth phase, and collecting bacterial cells; step 2: resuspending the collected cells in sterile water, performing heat inactivation, and collecting the heat-inactivated bacterial cells; step 3: resuspending the heat-inactivated bacterial cells in PBS buffer, performing ultrasonic disruption of the heat-inactivated bacterial cells, centrifuging to collect pellet, and extracting the peptidoglycan from the pellet.

Optionally, in step 2, the collected bacterial cells are resuspended in sterile water to prepare a bacterial suspension with a viable bacterial concentration of $1.0\times10^9$ CFU/ml.

Optionally, in step 2, the heat inactivation is performed at a temperature of 90-150°C for 5-15 min.

Optionally, in step 3, extracting the peptidoglycan from the pellet specifically includes: resuspending the heat-inactivated bacterial cell pellet in PBS buffer, performing ultrasonic disruption, then centrifuging to obtain a pellet; boiling the obtained pellet in 4% SDS solution for 30 minutes, then adding 10% TCA to remove teichoic acid and proteins from the heat-inactivated bacterial cells; then adding a mixed solvent comprising sodium acetate, chloroform, and methanol for degreasing; then treating with a composite enzyme solution of alkaline protease and trypsin at 37°C for 12 hours, then boiling in 1% SDS solution for 10 minutes, and washing to remove SDS to a trace level; then performing dialysis for 2 days with water changed every 8 hours, collecting sediment, and freeze-drying the sediment under vacuum to obtain peptidoglycan.

The present invention also provides *Lactobacillus paracasei* ET-22 bacteriocin, including exocytotic substance of *Lactobacillus paracasei* ET-22 and metabolites of *Lactobacillus paracasei* ET-22. The mass ratio of citric acid to L-methionine in the bacteriocin is greater than 15:1. The deposit number of *Lactobacillus paracasei* ET-22 is CGMCC No. 15077.

Optionally, the mass ratio of citric acid to L-methionine in the bacteriocin is 15-100:1; preferably, the mass ratio of citric acid to L-methionine in the bacteriocin is 35-45:1.

The present invention also provides a preparation method for the *Lactobacillus paracasei* ET-22 bacteriocin as described above, including the following steps: bacteriocin extraction, where the bacterial sludge separated from the *Lactobacillus paracasei* ET-22 fermentation broth is extracted with a solvent to obtain a mixed solution; sterilization, where the supernatant is separated from the mixed solution and subjected to heat sterilization to obtain the bacteriocin.

Optionally, the extraction temperature is 0-37°C; preferably, the extraction temperature is 20-30°C; and/or, the extraction time is 0.5-3 h; preferably, the extraction time is 30-120 min.

Optionally, the total colony count of *Lactobacillus paracasei* ET-22 in the mixed solution is $0.5\times10^{11}$ to $5x10^{11}$ CFU/ml; preferably, the total colony count of *Lactobacillus paracasei* ET-22 in the mixture is $2\times10^{11}$ to $4\times10^{11}$ CFU/ml.

Optionally, the heat sterilization temperature is 70-121°C; preferably, the heat sterilization temperature is 70-100°C.

Optionally, the heat sterilization time is 5-30 min; preferably, the heat sterilization time is 10-16 min.

Optionally, the method further includes a fermentation step: performing liquid culture of *Lactobacillus paracasei* ET-22, and stopping fermentation upon reaching the logarithmic growth phase.

Preferably, the fermentation step includes: inoculating a fermentation seed solution of *Lactobacillus paracasei* ET-22 into a culture medium, and fermenting at a temperature of 30-40°C, a rotational speed of 60-80 rpm, and a pH of 5.5-6.0 for 12-14 h to obtain a *Lactobacillus paracasei* ET-22 fermentation broth.

Preferably, the preparation of the fermentation seed solution of *Lactobacillus paracasei* ET-22 includes: subjecting *Lactobacillus paracasei* ET-22 strain to activation, purification, primary seed solution culture, secondary seed solution expansion, tertiary seed solution culture, and fermentation seed preparation, and obtaining the fermentation seed solution of *Lactobacillus paracasei* ET-22.

Preferably, the culture medium is MRS liquid medium.

Preferably, the solvent is water; preferably, the solvent is sterile water.

Preferably, after the sterilization step, the bacteriocin is freeze-dried to obtain a freeze-dried bacteriocin.

The present invention also provides the use of *Lactobacillus paracasei* ET-22 bacteriocin as described above in preparing food, where the food includes at least one of dairy product and beverage.

Optionally, the composition includes an antioxidant composition; preferably, the composition is selected from at least one of pharmaceutical, health food, and feed.

The present invention provides the use and composition of postbiotic from *Lactobacillus paracasei* ET-22, which can significantly reduce the content of volatile sulfur compounds (VSCs) in breath and volatile organic compounds (VOCs) in salvia within the oral cavity, and lower the content of characteristic flavor compounds such as indole, propanoic acid, and hexanoic acid; and can alter the oral flora, and inhibit the growth of oral biofilm and pathogenic bacteria, thereby having the effect of freshening breath and alleviating and inhibiting halitosis, and also having antioxidant effects.

## BRIEF DESCRIPTION OF DRAWINGS

FIG. 1 shows an improvement effect of the lysate isolated from ET-22 heat-inactivated bacterial cells on VSC concentration.
FIG. 2 shows a composition of volatile organic compounds after lysate intervention.
FIG. 3 shows a composition of volatile flavor-active compounds after lysate intervention.
FIG. 4 is a diagram showing a change in content of characteristic flavor-active compounds in the oral cavity after lysate intervention.
FIG. 5 is a diagram showing a change in biofilm biomass after lysate intervention.
FIG. 6a is an SEM image of the biofilm of a control group without intervention in a lysate observation group.
FIG. 6b is an SEM image of the biofilm after ET-22 viable bacteria intervention in the lysate observation group.
FIG. 6c is an SEM image of the biofilm after ET-22 heat-inactivated bacterial cells intervention in the lysate observation group.
FIG. 6d is an SEM image of the biofilm after lysate intervention.
FIG. 7a is a CLSM image of the biofilm of the control group without intervention in the lysate observation group.
FIG. 7b is a CLSM image of the biofilm after ET-22 viable bacteria intervention in the lysate observation group.
FIG. 7c is a CLSM image of the biofilm after ET-22 heat-inactivated bacterial cells intervention in the lysate observation group.
FIG. 7d is a CLSM image of the biofilm after lysate intervention.
FIG. 8 is a diagram showing the change in biofilm thickness after lysate intervention.
FIG. 9 is a diagram showing a change in relative abundance of halitosis-causing pathogenic bacteria after lysate intervention.
FIG. 10 is a diagram showing a change in relative proportion of a halitosis virulence factor after lysate intervention.
FIG. 11 shows an improvement effect of two nucleotides isolated from the lysate of ET-22 heat-inactivated bacterial cells on VSC concentration at different concentrations.
FIG. 12 shows a composition of volatile organic compounds after NAD intervention.
FIG. 13 shows a composition of volatile flavor-active compounds after NAD intervention.
FIG. 14 is a diagram showing the change in content of characteristic flavor-active compounds in the oral cavity after NAD intervention.
FIG. 15 is a diagram showing the change in biofilm biomass after NAD intervention.
FIG. 16a is an SEM image of the biofilm in the control group without intervention in the NAD observation group.
FIG. 16b is an SEM image of the biofilm after ET-22 viable bacteria intervention in the NAD observation group.
FIG. 16c is an SEM image of the biofilm after ET-22 heat-inactivated bacterial cells intervention in the NAD observation group.
FIG. 16d is an SEM image of the biofilm after NAD intervention.
FIG. 17a is a CLSM image of the biofilm in the control group without intervention in the NAD observation group.
FIG. 17b is a CLSM image of the biofilm after ET-22 viable bacteria intervention in the NAD observation group.
FIG. 17c is a CLSM image of the biofilm after ET-22 heat-inactivated bacterial cells intervention in the NAD observation group.
FIG. 17d is a CLSM image of the biofilm after NAD intervention.
FIG. 18 is a diagram showing the change in biofilm thickness after NAD intervention.
FIG. 19 is a diagram showing the change in relative abundance of halitosis-causing pathogenic bacteria after NAD intervention.
FIG. 20 is a diagram showing the change in relative proportion of a halitosis virulence factor after NAD intervention.
FIG. 21 shows the improvement effect of various organic acids isolated from the lysate of ET-22 heat-inactivated bacterial cells on VSC concentration at different concentrations.
FIG. 22 shows the composition of volatile organic compounds after 2-hydroxy-3-phenylpropionic acid intervention.
FIG. 23 is a diagram showing the composition of volatile flavor-active compounds after 2-hydroxy-3-phenylpropionic

acid intervention.

FIG. 24 is a diagram showing the change in content of characteristic flavor-active compounds in the oral cavity after 2-hydroxy-3-phenylpropionic acid intervention.

FIG. 25 is a diagram showing the change in biofilm biomass after 2-hydroxy-3-phenylpropionic acid intervention.

FIG. 26a is an SEM image of the biofilm in the control group without intervention in the 2-hydroxy-3-phenylpropionic acid observation group.

FIG. 26b is an SEM image of the biofilm after ET-22 viable bacteria intervention in the 2-hydroxy-3-phenylpropionic acid observation group.

FIG. 26c is an SEM image of the biofilm after ET-22 heat-inactivated bacteria intervention in the 2-hydroxy-3-phenylpropionic acid observation group.

FIG. 26d is an SEM image of the biofilm after 2-hydroxy-3-phenylpropionic acid intervention.

FIG. 27a is a CLSM image of the biofilm in the control group without intervention in the 2-hydroxy-3-phenylpropionic acid observation group.

FIG. 27b is a CLSM image of the biofilm after ET-22 viable bacteria intervention in the 2-hydroxy-3-phenylpropionic acid observation group.

FIG. 27c is a CLSM image of the biofilm after ET-22 heat-inactivated bacteria intervention in the 2-hydroxy-3-phenylpropionic acid observation group.

FIG. 27d is a CLSM image of the biofilm after 2-hydroxy-3-phenylpropionic acid intervention.

FIG. 28 is a diagram showing the change in biofilm thickness after 2-hydroxy-3-phenylpropionic acid intervention.

FIG. 29 is a diagram showing the change in relative abundance of halitosis-causing pathogenic bacteria after 2-hydroxy-3-phenylpropionic acid intervention.

FIG. 30 is a diagram showing the change in relative proportion of a halitosis virulence factor after 2-hydroxy-3-phenylpropionic acid intervention.

FIG. 31 shows an improvement effect of various polypeptide components isolated from the lysate of ET-22 heat-inactivated bacterial cells on VSC concentration at different concentrations.

FIG. 32 shows a composition of volatile organic compounds after polypeptide GP(HYP)GAG intervention.

FIG. 33 shows a composition of volatile flavor-active compounds after polypeptide GP(HYP)GAG intervention.

FIG. 34 shows an effect of polypeptide GP(HYP)GAG on key flavor-active compounds in the oral cavity.

FIG. 35 shows an SEM image of the biofilm after polypeptide GP(HYP)GAG intervention.

FIG. 36 shows a CLSM image of the biofilm after polypeptide GP(HYP)GAG intervention.

FIG. 37 is a diagram showing the change in biofilm thickness after polypeptide GP(HYP)GAG intervention.

FIG. 38 is a diagram showing the change in relative abundance of halitosis-causing pathogenic bacteria after polypeptide GP(HYP)GAG intervention.

FIG. 39 is a diagram showing the change in relative abundance of halitosis virulence factor after polypeptide GP(HYP) GAG intervention.

FIG. 40 shows an improvement effect of peptidoglycan isolated from ET-22 heat-inactivated bacterial cells on VSC concentration.

FIG. 41 shows a composition of volatile organic compounds after peptidoglycan intervention.

FIG. 42 shows a composition of volatile flavor-active compounds after peptidoglycan intervention.

FIG. 43 is a diagram showing the change in content of characteristic flavor-active compounds in the oral cavity after peptidoglycan intervention.

FIG. 44 is a diagram showing the change in biofilm biomass after peptidoglycan intervention.

FIG. 45a is an SEM image of the biofilm in the control group without intervention in the peptidoglycan observation group.

FIG. 45b is an SEM image of the biofilm after ET-22 viable bacteria intervention in the peptidoglycan observation group.

FIG. 45c is an SEM image of the biofilm after ET-22 heat-inactivated bacterial cells intervention in the peptidoglycan observation group.

FIG. 45d is an SEM image of the biofilm after peptidoglycan intervention.

FIG. 46a is a CLSM image of the biofilm in the control group without intervention in the peptidoglycan observation group.

FIG. 46b is a CLSM image of the biofilm after ET-22 viable bacteria intervention in the peptidoglycan observation group.

FIG. 46c is a CLSM image of the biofilm after ET-22 heat-inactivated bacterial cells intervention in the peptidoglycan observation group.

FIG. 46d is a CLSM image of the biofilm after peptidoglycan intervention.

FIG. 47 is a diagram showing the change in biofilm thickness after peptidoglycan intervention.

FIG. 48 is a diagram showing the change in relative abundance of *Prevotella intermedia* after peptidoglycan

intervention.

FIG. 49 is a diagram showing the change in relative proportion of halitosis virulence factor after peptidoglycan intervention.

FIG. 50 is a flow chart for preparing *Lactobacillus paracasei* ET-22 bacteriocin in Example 3.

FIG. 51 is a physical image of the *Lactobacillus paracasei* ET-22 bacteriocin in Example 3.

FIG. 52 is a liquid chromatogram of a bacteriocin sample obtained in Example 3.

FIG. 53 shows the hydroxyl-radical scavenging ability of *Lactobacillus paracasei* ET-22 bacteriocin under different extraction conditions.

FIG. 54 shows the DPPH-radical scavenging ability of *Lactobacillus paracasei* ET-22 bacteriocin under different extraction conditions.

FIG. 55 shows the hydroxyl-radical scavenging ability and DPPH-radical scavenging ability of inactivated bacteria at different heat sterilization temperatures in Comparative Example 1.

## DESCRIPTION OF EMBODIMENTS

To make the objectives, technical solutions, and advantages of the present invention clearer, the embodiments of the present invention will be described clearly and completely below with reference to the embodiments of the present invention. It is apparent that the described embodiments are only a part of the embodiments of the present invention, and not all of them. All other embodiments obtained by a person of ordinary skill in the art based on the embodiments of the present invention without creative efforts shall fall within the protection scope of the present invention.

The *Lactobacillus paracasei* ET-22 strain in the embodiments of the present invention has been deposited at the China General Microbiological Culture Collection Center (CGMCC) with the deposit number of CGMCC No. 15077.

In the embodiments of the present invention, the postbiotic refers to bacterial cell components of inactivated microorganisms and/or microorganisms, including metabolites produced during their fermentation, active small molecules released by decomposition of fermentation substrates, dead cells, and cell components after lysis.

The structure of 2-hydroxy-3-phenylpropionic acid provided in the embodiments of the present invention is shown in Formula 1-1:

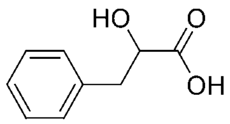

Formula 1-1.

Example 1: Extraction, isolation, and identification of lysate from ET-22 heat-inactivated bacterial cells (*Lactobacillus paracasei* ET-22 lysate), nucleotides, organic acids, polypeptides, and peptidoglycan from ET-22 postbiotic

1.1 Strain cultivation

The *Lactobacillus paracasei* ET-22 strain was purchased from the China General Microbiological Culture Collection Center, with a deposit number CGMCC No. 15077. MRS medium was used for strain resuscitation in a 37°C incubator for 12 hours, followed by dilution and plating on MRS solid medium, and culturing at 37°C for 48 hours. Single colonies were picked and cultured in the MRS medium at 37°C for 12 hours. Morphological and staining examinations were performed to confirm a pure culture. Then, the strain was subcultured consecutively for 2 passages. The third-generation bacterial suspension was taken for experiments and subjected to dilution and plate-counting.

MRS medium: 10 g/L peptone, 5 g/L beef extract powder, 4 g/L yeast powder, 2 g/L glucose, 1 mL/L Tween 80, 2 g/L dipotassium hydrogen phosphate, 5 g/L sodium acetate, 2 g/L triammonium citrate, 0.2 g/L magnesium sulfate, 0.05 g/L manganese sulfate, and distilled water.

1.2 Isolation and purification of lysate (preparation of postbiotic components)

The resuscitated bacterial suspension from 1.1 was centrifuged for 10 minutes at 4500 r/min. The bacterial pellet was washed three times with sterile water and resuspended in sterile water. Then, it was sterilized at 100°C for 10 minutes. The sterilized bacterial suspension was then centrifuged at a high speed of 10000 r/min for 10 minutes. The obtained pellet was the heat-inactivated bacterial cells, and the supernatant was filtered through a 0.22 μm sterile filter membrane. The filtered liquid was the lysate (lysate supernatant).

1.3 Sample preparation

The above lysate supernatant was freeze-dried and reconstituted in 1.5 mL of sterile water. 100 μL of liquid sample was pipetted into a 1.5 mL centrifuge tube, and 400 μL of extraction solution (acetonitrile:methanol=1:1) was added. The mixture was vortexed for 30 s and then subjected to low-temperature ultrasonic extraction for 30 min (5°C, 40 KHz). The sample was allowed to stand at -20°C for 30 min, then centrifuged at 4°C under 13000 g for 15 min. The supernatant was taken and dried under nitrogen gas, reconstituted in 100 μL of reconstitution solution (acetonitrile:water=1:1), followed by low-temperature ultrasonic extraction for 5 min (5°C, 40 KHz), and then centrifuged at 4°C under 13000 g for 5 min. The supernatant was transferred to a sampler vial with an insert for instrumental analysis.

LC-MS/MS analysis: The LC-MS analysis platform was a Thermo Fisher Scientific's Ultra High Performance Liquid Chromatography-Tandem Fourier Transform Ion Spectrometry UHPLC-Q Exactive HF-X system. Chromatographic conditions: 2 μL sample was separated by an HSS T3 column (100 mm×2.1 mm i.d., 1.8 μm) and then subjected to the mass spectrometer detection. Mobile phase A was 95% water + 5% acetonitrile (containing 0.1% formic acid). Mobile phase B was 47.5% acetonitrile + 47.5% isopropanol + 5% water (containing 0.1% formic acid). Separation gradient: 0-3.5 min, the mobile phase B increased from 0% to 24.5%, and its flow rate was 0.40 mL/min; 3.5-5 min, the mobile phase B increased from 24.5% to 65%, and the flow rate was 0.4 mL/min; 5-5.5 min, the mobile phase B increased from 65% to 100%, and its flow rate was 0.4 mL/min; 5.5-7.4 min, the mobile phase B maintained at 100%, and its flow rate increased from 0.4 mL/min to 0.6 mL/min; 7.4-7.6 min, the mobile phase B decreased from 100% to 51.5%, and its flow rate was 0.6 mL/min; 7.6-7.8 min, the mobile phase B decreased from 51.5% to 0%, and its flow rate decreased from 0.6 mL/min to 0.5 mL/min; 7.8-9 min, the mobile phase B maintained at 0%, and its flow rate decreased from 0.5 mL/min to 0.4 mL/min; 9-10 min, the mobile phase B maintained at 0%, and its flow rate 0.4 mL/min. The column temperature was 40°C.

Mass spectrometry conditions: Mass spectrometric signal acquisition of the sample was performed using positive and negative ion scanning modes, with a mass scan range of 70-1050 m/z. Sheath gas flow rate was 50 psi, auxiliary gas flow rate was 13 psi, auxiliary gas heating temperature was 425°C, ion spray voltage was set to 3500 V in positive mode, ion spray voltage was set to -3500 V in negative mode, ion transfer tube temperature was 325°C, normalized collision energy was 20-40-60 V cyclic collision energy. Primary mass spectrometry resolution was 60000, secondary mass spectrometry resolution was 7500, and data were collected in DDA mode.

The composition of small molecule active substances in the *Lactobacillus paracasei* ET-22 lysate was determined by a non-targeted metabolomics LC-MS, finding that it mainly consisted of three categories: polypeptides, organic acids, and nucleotides. Details are shown in Table 1.

Table 1 Core components in the lysate

| Substance name | Chemical formula | Peak area | Content(mg/L ) |
|---|---|---|---|
| 6-hydroxyhexanoic acid | $C_6H_{12}O3$ | 205812745.6 4 | 292.725 |
| 3-aminoglutaric acid | $C_5H_9NO_4$ | 88405084.65 | 54.23 |
| Nonanedioic acid | $C_9H_{16}O_4$ | 3050976.28 | 2.58 |
| 2-hydroxy-3-(4-hydroxyphenyl)propanoic acid | $C_9H_{10}O4$ | 13352475.46 | 8.16 |
| 2-hydroxybutanedioic acid | $C_{19}H_{34}O_3$ | 8997474.54 | 9.5 |
| 2-hydroxy-3-phenylpropionic acid | $C_9H_{10}O_3$ | 46462822.43 | 145.97 |
| Nicotinamide adenine dinucleotide (NAD) | $C_{21}H_{28}N_7O_{14}P$ 2 | 33616876.13 | 0.17 |
| Flavin adenine dinucleotide (FAD) | $C_{27}H_{33}N_9O_{15}P$ 2 | 13457779.69 | 102.83 |
| GPRPK | $C_{24}H_{43}N_9O_6$ | 3298839.11 | 0.21 |
| Asp-Leu | $C_{10}H_{19}N_3O_4$ | 25101954.11 | 31.44 |
| Asp-Phe | $C_{13}H_{16}N_2O_5$ | 68591468.76 | 14.85 |
| GP(Hyp)GAG | $C_{19}H_{30}N_6O_8$ | 21764593.42 | 0.24 |

According to Table 1, the polypeptides in the ET-22 lysate include GPRPK, Asp-Leu, Asp-Phe, and GP(Hyp)GAG (structure as shown in Formula 1); organic acids include 6-hydroxyhexanoic acid, 3-aminoglutaric acid, nonanedioic acid, 2-hydroxy-3-(4-hydroxyphenyl)propionic acid, 2-hydroxybutanedioic acid, and 2-hydroxy-3-phenylpropionic acid; nucleotides include nicotinamide adenine dinucleotide (NAD) and flavin adenine dinucleotide (FAD). Formula 1 is shown below:

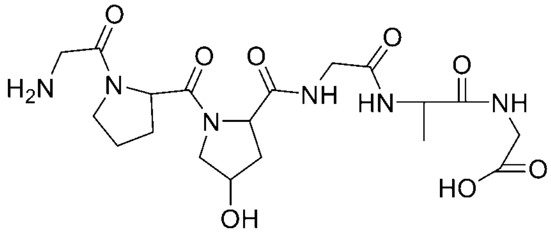

Formula 1.

1.4 Isolation and purification of peptidoglycan

Peptidoglycan was isolated from the heat-inactivated bacterial cells of *Lactobacillus paracasei* ET-22 after the bacterial cells were inactivated by high-temperature heating.

In one specific embodiment, *Lactobacillus paracasei* ET-22 could be first inoculated onto a solid medium for cultivation and resuscitation. Then, single colonies were inoculated into a liquid medium for fermentation. When the bacterial cells reached the logarithmic growth phase, the fermentation broth and bacterial cells were separated and the solid bacterial cells were collected.

Subsequently, the bacterial cells collected by filtration were resuspended in sterile water for heat inactivation, with the heat inactivation temperature being 90-150°C. After inactivation for 5-15 min, the obtained solution was taken out and cooled to room temperature, and the heat-inactivated bacterial cells were collected by filtration.

Finally, peptidoglycan was isolated and extracted from the heat-inactivated bacterial cells. The heat-inactivated bacterial cell pellet was resuspended in PBS buffer, subjected to ultrasonic disruption, and centrifuged to obtain a pellet. The pellet was boiled in 4% SDS solution for 30 minutes, then 10% TCA was added to remove teichoic acid and proteins from the heat-inactivated bacterial cells. Then, a mixed solvent including sodium acetate, chloroform, and methanol was added for degreasing. Then, degreased bacterial cells were treated with a composite enzyme solution of alkaline protease and trypsin at 37°C for 12 hours, followed by boiling in 1% SDS solution for 10 minutes, and washing to remove SDS to a trace level. Then, dialysis was performed for 2 days, with water changed every 8 hours. The obtained sediment was collected and vacuum freeze-dried to obtain peptidoglycan.

Specifically, the resuscitated bacterial suspension from 1.1 was centrifuged for 10 minutes at 4500 r/min. The bacterial pellet was washed three times with sterile water and resuspended in sterile water. Then, it was sterilized at 100°C for 10 minutes. The sterilized bacterial suspension was then centrifuged at high speed of 10000 r/min for 10 minutes. The obtained pellet was the heat-inactivated bacterial cells.

The heat-inactivated bacterial cell pellet was resuspended in PBS buffer, subjected to ultrasonic disruption at 240W for 30 minutes, and then centrifuged to obtain a precipitate. Then, the precipitate was boiled in 4% SDS solution for 30 minutes, followed by the addition of 10% TCA and standing for a period. A mixed solvent (sodium acetate:chloroform:methanol = 4:5:10 by volume) was added. Then, the obtained solution was treated with a composite enzyme solution of alkaline protease and trypsin at 37°C for 12 hours, followed by boiling in 1% SDS solution for 10 minutes, and washing to remove SDS to a trace level. Then, dialysis was performed for 2 days, with water changed every 8 hours. The sediment was vacuum freeze-dried. The obtained white or pale yellow powder was peptidoglycan.

Example 2: Respective study and validation of the effects of lysate, two nucleotides (nicotinamide adenine dinucleotide (NAD) and flavin adenine dinucleotide (FAD)), five organic acids, four polypeptides, and peptidoglycan on halitosis

Fresh saliva was collected from 10 halitosis subjects (Halimeter index >120). Subjects were required not to consume any food or drink for 2 hours before donating saliva. Saliva was directly spat into a saliva collection tube, 5 ml per person, and mixed. The mixture was centrifuged at 4500 rpm for 10 minutes, and the supernatant was collected, aliquoted, and stored at -80°C for subsequent experimental studies.

(I) In-vitro determination of the effects of lysate, two nucleotides (nicotinamide adenine dinucleotide (NAD) and flavin adenine dinucleotide (FAD)), five organic acids, four polypeptides, and peptidoglycan on VSC concentration using Halimeter

Lysate observation group: In a 10 ml sample vial, 100 μl of saliva was added first, followed by the separate addition of ET-22 viable bacteria ($1.0\times10^9$ CFU/ml), ET-22 heat-inactivated bacterial cells (obtained by inactivating viable bacteria at $1.0\times10^9$ CFU/ml), and lysate (obtained by inactivating and then filtering viable bacteria at $1.0\times10^9$ CFU/ml), each at 100μl. Then, 1.8 ml of SHI medium was added. After filling with nitrogen, anaerobic cultivation was performed for 10 hours. Then, the obtained culture was placed in a 60°C water bath for 30-min incubation. VSC concentration was

measured using the in vitro Halimeter method.

Group of two nucleotides: In a 10 ml sample vial, 100 μl of saliva was added first, followed by the separate addition of ET-22 viable bacteria ($1.0\times10^9$ CFU/ml), ET-22 heat-inactivated bacterial cells (obtained by inactivating viable bacteria at $1.0\times10^9$ CFU/ml), NAD, and FAD (500 μg/ml, 100 μg/ml, 10 μg/ml), each at 100μl. Then, 1.8 ml of SHI medium was added. After filling with nitrogen, anaerobic cultivation was performed for 10 hours. Then, the obtained culture was placed in a 60°C water bath for 30-min incubation. VSC concentration was measured using the in vitro Halimeter method.

Group of five organic acids: In a 10 ml sample vial, 100 μl of saliva was added first, followed by the separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and five organic acid components (500 μg/ml, 100 μg/ml, 10 μg/ml), each at 100μl. Then, 1.8 ml of SHI medium was added. After filling with nitrogen, anaerobic cultivation was performed for 10 hours. Then, the obtained culture was placed in a 60°C water bath for 30-min incubation. VSC concentration was measured using the in vitro Halimeter method.

Observation group of four polypeptides: In a 10 ml sample vial, 100 μl of saliva was added first, followed by the separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and components of four polypeptides (500 μg/ml, 100 μg/ml, 10 μg/ml), each at 100μl. Then, 1.8 ml of SHI medium was added. After filling with nitrogen, anaerobic cultivation was performed for 10 hours. Then, the obtained culture was placed in a 60°C water bath for 30-min incubation. VSC concentration was measured using the in vitro Halimeter method.

Peptidoglycan group: In a 10 ml sample vial, 100 μl of saliva was added first, followed by the separate addition of ET-22 viable bacteria ($1.0\times10^9$ CFU/ml), ET-22 heat-inactivated bacterial cells (obtained by inactivating viable bacteria at $1.0\times10^9$ CFU/ml), and peptidoglycan (peptidoglycan extracted from viable bacteria at $1.0\times10^9$ CFU/ml). Then, 1.8 ml of SHI medium was added. After filling with nitrogen, anaerobic cultivation was performed for 10 hours. Then, the obtained culture was placed in a 60°C water bath for 30-min incubation. VSC concentration was measured using the in vitro Halimeter method.

In the embodiments of the present invention, the above lysate group, two nucleotides group, five organic acids group, four polypeptides group, and peptidoglycan group all used SHI medium and the composition of the SHI medium was as follows: 10 g/L peptone, 5 g/L trypsin peptone, 5 g/L yeast extract, 2.5 g/L potassium chloride, 5 mg/L chlorhematin, 1 mg/L vitamin K, 0.06 g/L urea, 0.174 g/L arginine, 2.5 g/L porcine gastric mucin, 5% sterile defibrinated sheep blood, 10 mg/L N-acetylmuramic acid, 1% sucrose, and distilled water.

The test results of the lysate group are shown in FIG. 1. In the CK group, after simulation fermentation, the VSC concentration is the highest, exceeding 200 ppm, with a noticeable unpleasant smell. Compared with a control group (CK group), the VSC concentration significantly decreases ($p<0.05$) after intervention with ET-22 viable bacteria (ET22-L), ET-22 heat-inactivated bacteria (ET22-HK), and lysate.

The test results of the two nucleotides group are shown in FIG. 11. Under intervention with three concentrations of nucleotide, the VSC concentration in the NAD group is significantly lower than that in the CK group ($p<0.05$) at high and medium NAD concentrations, but there is no significant change between the NAD group and the CK group at low nucleotide concentration ($p>0.05$). The FAD group shows no significant change compared to the CK group at any FAD concentration ($p>0.05$). Therefore, NAD is determined to be the core active component for subsequent experiments.

The test results of the five organic acids group are shown in FIG. 21. Under intervention with three concentrations of organic acid components, the VSC concentration in the group of high-concentration 2-hydroxy-3-phenylpropionic acid is significantly lower than that in the control group ($p<0.05$); under medium concentration intervention, the VSC concentration in the group of 2-hydroxy-3-phenylpropionic acid is significantly lower than that in the control group ($p<0.05$); under low concentration intervention, none of the components show significant changes compared to the control group. Therefore, 2-hydroxy-3-phenylpropionic acid is determined to be the core active component for subsequent experiments.

The test results of the four polypeptides group are shown in FIG. 31. In FIG. 31, A represents the change of VSC concentration after intervention with 500 μg/ml polypeptide; B represents the change of VSC concentration after intervention with 100 μg/ml polypeptide, and C represents the change of VSC concentration after intervention with 10 μg/ml polypeptide. According to FIG. 31, it can be seen that under intervention with three concentrations of polypeptide components, only GP(HYP)GAG at high and medium concentrations significantly reduced VSC concentration compared to the CK group ($p<0.05$). Therefore, GP(HYP)GAG is determined to be the core active component.

The test results of the peptidoglycan group are shown in FIG. 40. In the CK group, after simulation fermentation, the VSC concentration was the highest, exceeding 200 ppm, with a noticeable unpleasant smell. Compared with the control group, the VSC concentration significantly decreases ($p<0.05$) after intervention with the ET-22 viable bacteria group (denoted as ET22-L), the ET-22 heat-inactivated bacteria group (denoted as ET22-HK), and peptidoglycan.

(II) Respective studies of the effects of lysate, NAD, 2-Hydroxy-3-phenylpropionic Acid, GP(HYP)GAG, and peptidoglycan on VOCs

2.1 Grouping and preparation of intervention samples

Lysate group: In a headspace vial, 100 μl of saliva was added first, followed by separate addition of ET-22 viable bacteria (1.0×$10^9$ CFU/ml), ET-22 heat-inactivated bacterial cells (obtained by inactivating viable bacteria at 1.0×$10^9$ CFU/ml), and lysate (obtained by inactivating and then filtering viable bacteria at 1.0×$10^9$ CFU/ml), each at 100μl. Then, 800 μl of SHI medium was added. After filling with nitrogen, anaerobic cultivation was performed for 10 hours for loading on machine, and then SPME-GC-MS was used to test the change in content of volatile organic compound.

NAD group: In a headspace vial, 100 μl of saliva was added first, followed by the separate addition of ET-22 viable bacteria (1.0×$10^9$ CFU/ml), ET-22 heat-inactivated bacterial cells (obtained by heat-inactivating viable bacteria at 1.0×$10^9$ CFU/ml), and NAD (100 μg/ml), each at 100μl. Then, 800 μl of SHI medium was added. After filling with nitrogen, anaerobic cultivation was performed for 10 hours for loading on machine, then SPME-GC-MS was used to test the changes in content of volatile organic compound.

2-hydroxy-3-phenylpropionic acid group: In a headspace vial, 100 μl of saliva was added first, followed by the separate addition of ET-22 viable bacteria (1.0×$10^9$ CFU/ml), ET-22 heat-inactivated bacterial cells (obtained by heat-inactivating viable bacteria at 1.0×$10^9$ CFU/ml), and 2-hydroxy-3-phenylpropionic acid (100 μg/ml), each at 100μl. Then, 800 μl of SHI medium was added. After filling with nitrogen, anaerobic cultivation was performed for 10 hours for loading on machine, and then SPME-GC-MS was used to test the changes in content of volatile organic compound.

GP(HYP)GAG group: In a headspace vial, 100 μl of saliva was added first, followed by the separate addition of ET-22 viable bacteria (1.0×$10^9$ CFU/ml), ET-22 heat-inactivated bacterial cells (obtained by heat-inactivating viable bacteria at 1.0×$10^9$ CFU/ml), and GP(HYP)GAG (100 μg/ml), each at 100μl. Then, 800 μl of SHI medium was added. After filling with nitrogen, anaerobic cultivation was performed for 10 hours for loading on machine, and then SPME-GC-MS was used to test the changes in content of volatile organic compound.

Peptidoglycan group: In a headspace vial, 100 μl of saliva was added first, followed by separate addition of ET-22 viable bacteria (1.0×$10^9$ CFU/ml), ET-22 heat-inactivated bacterial cells (same as Example 1), and peptidoglycan (same as Example 1), each at 100μl. Then, 800 μl of SHI medium was added. After filling with nitrogen, anaerobic cultivation was performed for 10 hours for loading on machine, and then SPME-GC-MS was used to test the changes in content of volatile organic compound.

Helium was used as a carrier gas, with a rate remaining at 1.1 mL min-1. The injector temperature was set at 250°C. Oven temperature program was as follows: holding at 40°C for 1 min, then ramping to 120°C at a rate of 8°C/min, followed by increasing to 230°C at a rate of 5°C/min and holding for 10 minutes. The mass spectrometer was operated in electron ionization (EI) mode, with ion source temperature of 230°C, interface temperature of 200°C, and M/Z scan range of 30-400. To identify specific compounds, their mass spectra were compared with those of true compounds given in the NIST 17 database (V.05). The minimum matching quality for forward and reverse matches was 75/100. The flavor characteristic of each compound was determined based on data from the Good Scents Company Information System (http://www.thegoodscentscompany.com).

A total of 241 volatile organic compounds (VOCs) were detected in this example, including 51 alkanes, 60 esters, 27 alcohols, 20 acids, 19 ketones, 20 alkenes, 9 aldehydes, 7 phenols, and 28 other types. Results of the lysate group: FIG. 1 shows the improvement effect of the lysate isolated from ET-22 heat-inactivated bacterial cells on VSC concentration; FIG. 2 shows the quantitative changes of various compounds under lysate intervention; and according to FIG. 2, it can be seen that under lysate intervention, the number of ester compounds significantly decreases, and the number of alcohols significantly increases ($p<0.001$). Results of the NAD group: FIG. 12 shows the quantitative changes of various compounds under NAD intervention, where ET22-L denotes a viable bacteria intervention group, and ET22-HK denotes a heat-inactivated bacteria intervention group; according to FIG. 12, it can be seen that under NAD intervention, the number of alkane compounds and acid compounds significantly decreases ($p<0.01$); studies have shown that alkane compounds are products of metabolism and decomposition in bacterial fatty acid pathway; other studies have indicated that alkane substances are closely related to microbial production, and one of the causes of halitosis may be related to fatty acid degradation. Results of the 2-hydroxy-3-phenylpropionic acid group: FIG. 22 shows the quantitative changes of various compounds after 2-hydroxy-3-phenylpropionic acid intervention, where ET22-L denotes the viable bacteria intervention group, and ET22-HK denotes the heat-inactivated bacteria intervention group; according to FIG. 22, it can be seen that after 2-hydroxy-3-phenylpropionic acid intervention, alkane compounds significantly decreases ($p<0.01$); studies have shown that alkane compounds are products of metabolism and decomposition in bacterial fatty acid pathway, and alkane substances are closely related to microbial production. Results of the GP(HYP)GAG group: FIG. 32 shows the quantitative changes of various compounds after GP(HYP)GAG intervention, where ET22-L denotes the viable bacteria intervention group, and ET22-HK denotes the heat-inactivated bacteria intervention group; according to FIG. 32, it can be seen that after GP(HYP)GAG intervention, acid compounds significantly decrease ($p<0.001$), and one of the causes of halitosis may be related to fatty acid degradation. Results of peptidoglycan: FIG. 41 shows the quantitative changes of various compounds under peptidoglycan intervention; according to FIG. 41, it can be seen that under peptidoglycan intervention, the number of alkane compounds significantly decreases ($p<0.05$), the number of alcohols

significantly increases ($p<0.001$), the number of alkene compounds also significantly increases ($p<0.001$), and the number of ester compounds also increases.

Among the 241 VOCs, 71 VOCs are identified as aroma-active VOCs and classified into 6 categories (esters, alcohols, acids, ketones, phenols, and other compounds). This example further detects the changes in the content of these 71 aroma-active VOCs under lysate intervention. The detection results are shown in FIG. 3. Esters and acids are the most flavor-active VOCs, and the number of flavor-active ester compounds significantly decreases ($p<0.05$) under intervention with ET22-L, ET22-HK, and lysate. This example also further detects the change in the content of these 71 aroma-active VOCs under NAD intervention. The detection results are shown in FIG. 13. The number of flavor-active acid compounds also significantly decreases ($p<0.001$) under NAD intervention, and acid compounds are the most flavor-active VOCs. This example also further detects the change in the content of these 71 aroma-active VOCs under 2-hydroxy-3-phenylpropionic acid intervention. The detection results are shown in FIG. 23. The number of flavor-active ester compounds and acid compounds also significantly decreases ($p<0.05$) after 2-hydroxy-3-phenylpropionic acid intervention. This example further detects the change in the content of these 71 aroma-active VOCs under GP(Hyp)GAG intervention. The detection results are shown in FIG. 33. The number of acid compounds also significantly decreases ($p<0.001$) after GP(Hyp)GAG intervention. This example also further detects the changes in the content of these 71 aroma-active VOCs under peptidoglycan intervention. The detection results are shown in FIG. 42. Esters are the most flavor-active VOCs, and the number of flavor-active ester compounds significantly decreases ($p<0.05$) under intervention with ET22-L, ET22-HK, and peptidoglycan.

2.2 Respective study the effects of lysate, NAD, 2-hydroxy-3-phenylpropionic acid, GP(Hyp)GAG, and peptidoglycan on characteristic flavor compounds

Since causes of oral malodor are not limited to VSCs, indole and short-chain fatty acids (propanoic acid, butyric acid, etc.) may also be causes of oral malodor. Indole is a compound with an unpleasant smell. Hexanoic acid is a compound with a sour, cheesy smell. Propanoic acid has been proven to cause halitosis and is also a compound with a sour, cheesy smell. Therefore, this example testes the effects of lysate, NAD, 2-hydroxy-3-phenylpropionic acid, GP(Hyp) GAG, and peptidoglycan on indole, hexanoic acid, and propanoic acid, respectively. In one specific embodiment, the above characteristic flavor compounds include one or more of indole, hexanoic acid, and propanoic acid.

The test results for lysate are shown in FIG. 4. In FIG. 4, A shows the effect of lysate on indole concentration, B shows the effect of lysate on propanoic acid concentration, and C shows the effect of lysate on hexanoic acid concentration. It can be seen that under intervention with ET-22 viable bacteria, ET-22 heat-inactivated bacterial cells and lysate, the concentrations of indole, hexanoic acid, and propanoic acid all significantly decrease ($p<0.01$), and the intervention effect of lysate on propanoic acid and hexanoic acid is better than that of ET-22 viable bacteria and its heat-inactivated bacteria.

The test results for NAD are shown in FIG. 14. In FIG. 14, A shows the effect of NAD on indole concentration, B shows the effect of NAD on propanoic acid concentration, and C shows the effect of NAD on hexanoic acid concentration. It can be seen that under intervention with ET-22 viable bacteria, ET-22 heat-inactivated bacterial cells and NAD, the concentrations of indole, hexanoic acid, and propanoic acid all significantly decrease ($p<0.01$), and the intervention effect of NAD is better than that of ET-22 viable bacteria and its heat-inactivated bacterial cells.

The test results for 2-hydroxy-3-phenylpropionic acid are shown in FIG. 24. In FIG. 24, A shows the effect of 2-hydroxy-3-phenylpropionic acid on indole concentration, B shows the effect of 2-hydroxy-3-phenylpropionic acid on propanoic acid concentration, and C shows the effect of 2-hydroxy-3-phenylpropionic acid on hexanoic acid concentration. It can be seen that under intervention with ET-22 viable bacteria, heat-inactivated bacteria, and 2-hydroxy-3-phenylpropionic acid, the concentrations of indole, hexanoic acid and propanoic acid all significantly decrease ($p<0.01$), and the inhibitory effect of 2-hydroxy-3-phenylpropionic acid is better than that of ET-22 viable bacteria and heat-inactivated bacteria.

The test results for GP(Hyp)GAG are shown in FIG. 34. In FIG. 34, A shows the effect of GP(Hyp)GAG on indole concentration, B shows the effect of GP(Hyp)GAG on propanoic acid concentration, and C shows the effect of GP(Hyp) GAG on hexanoic acid concentration. It can be seen that under intervention with ET-22 and its heat-inactivated bacteria, the concentrations of indole, hexanoic acid, and propanoic acid all significantly decrease ($p<0.01$). Under GP(Hyp)GAG intervention, hexanoic acid and propanoic acid are not detected, indicating that the polypeptide GP(Hyp)GAG has a good inhibitory effect on characteristic flavor compounds.

The test results for peptidoglycan are shown in FIG. 43. In FIG. 43, A shows the effect of peptidoglycan on indole concentration, B shows the effect of peptidoglycan on propanoic acid concentration, and C shows the effect of peptidoglycan on hexanoic acid concentration. It can be seen that under intervention with ET-22 viable bacteria, ET-22 heat-inactivated bacterial cells and peptidoglycan, the concentrations of indole, hexanoic acid and propanoic acid all significantly decrease ($p<0.01$), and the intervention effect of peptidoglycan on hexanoic acid is better than that of ET-22 viable bacteria and its heat-inactivated bacteria.

(III) Respective study of the effects of lysate, NAD, 2-hydroxy-3-phenylpropionic acid, and peptidoglycan on the biomass of multi-species biofilm

The association between biofilm biomass and halitosis degree were analyzed by evaluating biofilm formation under intervention with ET-22 viable bacteria, heat-inactivated bacteria, lysate, NAD, 2-hydroxy-3-phenylpropionic acid and peptidoglycan. Test method included the following.

(1) Grouping and culturing

Lysate Group: 10 μl of saliva was added to a 96-well plate, followed by separate addition of ET-22 viable bacteria ($1.0\times10^9$ CFU/ml), ET-22 heat-inactivated bacterial cells (obtained by inactivating viable bacteria at $1.0\times10^9$ CFU/ml), and lysate (obtained by inactivating and filtering viable bacteria at $1.0\times10^9$ CFU/ml), each at 100 μl. Then, 80 μl of SHI medium was added. The plate was placed in an anaerobic box and cultured anaerobically at 37°C for 10 hours. Six replicates were set for each group.

NAD Group: 10 μl of saliva was added to a 96-well plate, followed by separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and NAD (100 μg/ml), each at 100 μl. Then, 80 μl of SHI medium was added. The plate was placed in an anaerobic box and cultured anaerobically at 37°C for 10 hours. Six replicates were set for each group.

2-hydroxy-3-phenylpropionic acid group: 10 μl of saliva was added to a 96-well plate, followed by separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and 2-hydroxy-3-phenylpropionic acid (100 μg/ml), each at 100 μl. Then, 80 μl of SHI medium was added. The plate was placed in an anaerobic box and cultured anaerobically at 37°C for 10 hours. Six replicates were set for each group.

Peptidoglycan group: 10 μl of saliva was added to a 96-well plate, followed by separate addition of ET-22 viable bacteria ($1.0\times10^9$ CFU/ml), ET-22 heat-inactivated bacterial cells (same as Example 1), and peptidoglycan (same as Example 1), each at 100 μl. Then, 80 μl of SHI medium was added. The plate was placed in an anaerobic box and cultured anaerobically at 37°C for 10 hours. Six replicates were set for each group.

(2) The supernatant was gently aspirated with a pipette tip, and then the plate was washed 1-2 times with sterile PBS buffer.

(3) 100 μl of 0.5% crystal violet dye was added to the well plate, followed by standing at room temperature for 20 minutes.

(4) After complete aspirating of the dye, the plate was washed 3 times or more with sterile PBS buffer until the dye was thoroughly washed away.

(5) The well plate was placed in a microplate reader and the biofilm biomass was measured at 600 nm.

The test results of the lysate group are shown in FIG. 5. The biofilm of the CK group has the highest optical density reading at 595 nm. Compared with the CK group, the biofilm biomass decreases after intervention with ET22-L and ET22-HK ($p<0.05$), and the intervention effect of lysate on the biofilm biomass is better than that of ET-22 viable bacteria and its heat-inactivated bacteria.

The test results of the NAD group are shown in FIG. 15. The CK group biofilm has the highest optical density reading. Compared with the CK group, the biofilm biomass decreases after intervention with ET22-L and ET22-HK ($p<0.05$). The NAD group shows a slight decrease in biomass compared to the CK group.

The test results of the 2-hydroxy-3-phenylpropionic acid group are shown in FIG. 25. The CK group biofilm has the highest optical density reading. Compared with the CK group, the biofilm biomass decreases after intervention with ET22-L, ET22-HK, and 2-hydroxy-3-phenylpropionic acid. The 2-hydroxy-3-phenylpropionic acid group shows a significant decrease in biomass compared to the CK group ($p<0.05$), and its intervention effect is better than that of ET-22 viable bacteria and heat-inactivated bacteria.

The test results of the peptidoglycan group are shown in FIG. 44. The biofilm of the CK group has the highest optical density reading at 595 nm. Compared with the CK group, the biofilm biomass decreases after intervention with ET22-L and ET22-HK ($p<0.05$). The peptidoglycan group shows a slight decrease in biomass compared to the CK group.

(IV) Observation of the effects of ET-22 viable bacteria, lysate, heat-inactivated bacteria, NAD, 2-hydroxy-3-phenylpropionic acid, GP(Hyp)GAG, and peptidoglycan on biofilm by scanning electron microscopy (SEM).

Observation methods includes the following.

(1) Grouping and culturing

Lysate group: A hydroxyapatite (HA) disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (obtained by inactivating viable bacteria at $1.0\times10^9$ CFU/ml), and lysate (obtained by inactivating and filtering viable

bacteria at $1.0\times10^9$ CFU/ml), each at 10 μl. Then, 80 μl of SHI medium was added. The 96-well plate was placed in an anaerobic box and cultured anaerobically at 37°C for 10 hours.

NAD group: A hydroxyapatite (HA) disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and NAD (100 μg/ml), each at 10 μl. Then, 80 μl of SHI medium was added. The 96-well plate was placed in an anaerobic box and cultured anaerobically at 37°C for 10 hours.

2-hydroxy-3-phenylpropionic acid group: A hydroxyapatite (HA) disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and 2-hydroxy-3-phenylpropionic acid (100 μg/ml), each at 10 μl. Then, 80 μl of SHI medium was added. The 96-well plate was placed in an anaerobic box and cultured anaerobically at 37°C for 10 hours.

GP(Hyp)GAG group: A hydroxyapatite (HA) disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and GP(Hyp)GAG (100 μg/ml), each at 10 μl. Then, 80 μl of SHI medium was added. The 96-well plate was placed in an anaerobic box and cultured anaerobically at 37°C for 10 hours.

Peptidoglycan group: A hydroxyapatite (HA) disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (same as Example 1), and peptidoglycan (same as Example 1), each at 10 μl. Then, 80 μl of SHI medium was added. The 96-well plate was placed in an anaerobic box and cultured anaerobically at 37°C for 10 hours.

(2) The HA disc surface was washed gently with sterile PBS buffer to remove floating bacteria.

(3) 2.5% glutaraldehyde solution was added to the 96-well plate for fixation, followed by standing at 4°C overnight.

(4) The fixed samples were subjected to gradient dehydration by sequentially placing them in ethanol solutions at concentrations of 30%, 50%, 60%, 70%, 80%, 90%, and 100%, each concentration for 15 minutes.

(5) Critical point drying and gold sputtering were performed, followed by observation under a scanning electron microscope.

Observation results of the lysate observation group are shown in FIGS. 6a-6d. In the CK group, the biofilm structure is dense, with various forms of bacteria in the form of rods and spheres interwoven layer by layer; and there are many layers of bacterial cells and a relatively large bacterial count. In the ET-22.L group, the number of rod-shaped bacteria in the biofilm increases, the layer number of bacterial cells decreases, and the biofilm becomes thinner. In the ET-22.HK group, its biofilm is similar to that of the ET-22.L group, except that the bacterial morphology is mostly short rods and cocci, with almost no extracellular matrix production. In the lysate group, the biofilm structure is looser, with decreased layer number of bacterial cells, and a large amount of extracellular matrix is produced between most bacterial cells; and some streptococci also form a compact island-like biofilm covered by a large amount of mucus or reticular structures.

Observation results of the NAD observation group are shown in FIGS. 16a-16d. In the CK group, the biofilm structure is dense, with various forms of bacteria in the form of rods and spheres interwoven layer by layer; and there are many layers of bacterial cells and a relatively large bacterial count. In the ET-22.L group, the number of rod-shaped bacteria in the biofilm increases, the layer number of bacterial cells decreases, and the biofilm becomes thinner. In the ET-22.HK group, its biofilm is similar to that of the ET-22.L group, except that the bacterial morphology is mostly short rods and cocci, with almost no extracellular matrix production. The biofilm structure of the NAD group exhibits a large number of bacterial cell layers but a relatively low bacterial count.

Observation results of the 2-hydroxy-3-phenylpropionic acid observation group are shown in FIGS. 26a-26d. In the CK group, the biofilm structure is dense, with various forms of bacteria in the form of rods and spheres interwoven layer by layer; and there are many layers of bacterial cells and a relatively large bacterial count. In the ET-22.L group, the number of rod-shaped bacteria in the biofilm increases, the layer number of bacterial cells decreases, and the biofilm becomes thinner. In the ET-22.HK group, its biofilm is similar to that of the ET-22.L group, except that the bacterial morphology is mostly short rods and cocci, with almost no extracellular matrix production. The biofilm structure of the 2-hydroxy-3-phenylpropionic acid group shows a large amount of mucus-like structures, the amount of extracellular matrix significantly decreases, the number of bacterial cell layers decreases, and the biofilm becomes thinner. Observation results of GP(Hyp) GAG Group are shown in FIG. 35. In the CK group, the biofilm structure is dense, with various forms of bacteria in the form of rods and spheres interwoven layer by layer; and there are many layers of bacterial cells and a relatively large bacterial count. In the ET-22.L group, the number of rod-shaped bacteria in the biofilm increases, the layer number of bacterial cells decreases, and the biofilm becomes thinner. In the ET-22.HK group, its biofilm is similar to that of the ET-22.L group, except that the bacterial morphology is mostly short rods and cocci, with almost no extracellular matrix production. In the GP(Hyp)GAG group, the bacterial morphology of biofilm is almost invisible, the biofilm structure is relatively loose, consisting mostly of dense, tiny extracellular matrix and some mucus-like biofilm. This indicates that the polypeptide GP(Hyp)GAG can effectively improve biofilm thickness.

Observation results of the peptidoglycan observation group are shown in FIGS. 45a-45d. In the CK group, the biofilm structure is dense, with various forms of bacteria in the form of rods and spheres interwoven layer by layer; and there are many layers of bacterial cells and a relatively large bacterial count. In the ET-22.L group, the number of rod-shaped bacteria in the biofilm increases, the layer number of bacterial cells decreases, and the biofilm becomes thinner. In the ET-22.HK group, its biofilm is similar to that of the ET-22.L group, except that the bacterial morphology is mostly short rods and cocci, with almost no extracellular matrix production. In the peptidoglycan group, the bacterial morphology of biofilm is almost invisible, the biofilm structure is relatively loose, consisting mostly of dense, tiny extracellular matrix and some mucus-like biofilm.

(V) Observation of the effects of ET-22 viable bacteria, heat-inactivated bacterial cells, lysate, NAD, 2-hydroxy-3-phenylpropionic acid, polypeptide GP(Hyp)GAG (GP(Hyp)GAG), and peptidoglycan on the biofilm thickness by confocal laser scanning microscopy (CLSM).

Observation methods included the following.

(1) Grouping and culturing

Lysate group: A HA disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (obtained by inactivating viable bacteria at $1.0\times10^9$ CFU/ml), and lysate (obtained by inactivating and filtering viable bacteria at $1.0\times10^9$ CFU/ml), each at 10 μl. Then, 80 μl of SHI medium was added. The 96-well plate was placed in an anaerobic box and cultured anaerobically at 37°C for 10 hours.

NAD group: A HA disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and NAD (100 μg/ml), each at 10 μl. Then, 80 μl of SHI medium was added. The 96-well plate was placed in an anaerobic box and cultured anaerobically at 37°C for 10 hours.

2-hydroxy-3-phenylpropionic acid group: A HA disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and 2-hydroxy-3-phenylpropionic acid (100 μg/ml), each at 10 μl. Then, 80 μl of SHI medium was added. The 96-well plate was placed in an anaerobic box and cultured anaerobically at 37°C for 10 hours.

Polypeptide GP(Hyp)GAG group: A HA disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (obtained by heat-inactivating viable bacteria at $1.0\times10^9$ CFU/ml), and polypeptide GP(Hyp)GAG (100 μg/ml), each at 10 μl. Then, 80 μl of SHI medium was added. The 96-well plate was placed in an anaerobic box and cultured anaerobically at 37°C for 10 hours.

Peptidoglycan group: A HA disc was placed in a 96-well plate. 10 μl of saliva was added to the 96-well plate, followed by separate addition of ET-22 viable bacteria ($1.0\times10^9$ CFU/ml), ET-22 heat-inactivated bacterial cells (same as Example 1), and peptidoglycan (same as Example 1), each at 10 μl. Then, 80 μl of SHI medium was added. The 96-well plate was placed in an anaerobic box and cultured anaerobically at 37°C for 10 hours.

(2) The HA disc surface was washed gently with 0.85% NaCl solution to remove floating bacteria.

(3) A Live/Dead Bacterial Staining Kit was used for 646 staining. 100 μl of N01 staining solution A was first added, followed by incubation at room temperature in the dark for 15 minutes. After washing the disc surface once with 0.85% NaCl solution, PI staining solution B was added and incubated at room temperature in the dark for 15 minutes. Then, the disc surface was washed once more with 0.85% NaCl solution.

(4) The stained disc was placed upside down on a glass slide and observed with a ×60 oil immersion lens using confocal microscopy.

(5) Experimental results were photographed and plotted using ZEN Lite 2012 software.

Observation results of the lysate observation group are shown in FIGS. 7a-7d. Subsequently, biofilm thickness was measured at different positions on the HA disc, and the resulting data were plotted as a bar chart shown in FIG. 8. According to FIGS. 7a-7d and FIG. 8, compared with the CK group, ET-22 viable bacteria and ET-22 heat-inactivated bacteria can both significantly reduce the biofilm thickness ($p<0.0001$); and the lysate can also reduce the biofilm thickness ($p<0.05$), and its intervention effect on biofilm thickness is superior to that of ET-22 viable bacteria and ET-22 heat-inactivated bacteria.

Observation results of the NAD observation group are shown in FIGS. 17a-17d. Subsequently, biofilm thickness was measured at different positions on the HA disc, and the resulting data were plotted as a bar chart shown in FIG. 18. According to FIGS. 17a-17d and FIG. 18, compared with the CK group, ET-22 viable bacteria and ET-22 heat-inactivated bacteria can both significantly reduce the biofilm thickness ($p<0.0001$). The intervention effect of NAD is not obvious.

Observation results of the 2-hydroxy-3-phenylpropionic acid observation group are shown in FIGS. 27a-27d.

Subsequently, biofilm thickness was measured at different positions on the HA disc, and the resulting data were plotted as a bar chart shown in FIG. 28. According to FIGS. 27a-27d and FIG. 28, compared with the CK group, ET-22 viable bacteria, ET-22 heat-inactivated bacteria and 2-hydroxy-3-phenylpropionic acid can all significantly reduce the biofilm thickness ($p<0.0001$); and the intervention effect of 2-hydroxy-3-phenylpropionic acid is superior to that of ET-22 viable bacteria and ET-22 heat-inactivated bacteria.

For observation results of the polypeptide GP(Hyp)GAG group, according to FIGS. 36-37, compared with the CK group, ET-22 viable bacteria, ET-22 heat-inactivated bacteria, and polypeptide GP(Hyp)GAG can all significantly reduce biofilm thickness ($p<0.0001$).

Observation results of the peptidoglycan observation group are shown in FIGS. 46a-46d. Subsequently, biofilm thickness was measured at different positions on the HA disc, and the resulting data were plotted as a bar chart shown in FIG. 47. According to FIGS. 46a-46d and FIG. 47, it can be seen that, compared with the CK group, ET-22 viable bacteria and ET-22 heat-inactivated bacteria can both significantly reduce biofilm thickness ($p<0.0001$), and peptidoglycan can also reduce biofilm thickness ($p<0.05$).

(VI) Establishment of an in vitro oral biofilm model to investigate the effects of lysate, NAD, 2-hydroxy-3-phenylpropionic acid, polypeptide GP(Hyp)GAG, and peptidoglycan on biofilms, respectively.

6.1 Construction of in vitro oral biofilm model

100 μl of saliva was added to a 24-well plate, then 100 μl of the corresponding concentration solution was added respectively to the experimental groups, and finally 800 μl of SHI medium was added. The plate was placed in an anaerobic box and cultured anaerobically at 37°C for 10 hours. Then, the supernatant was discarded, and the multi-species biofilm adhered to the well plate was obtained. After discarding the supernatant from the incubated biofilm, the biofilm adhered to the bottom of the well plate was re-suspended with sterile water by pipetting, transferred to a 1.5 ml centrifuge tube, and stored until gene sequencing was performed.

6.2 Respective study of the effects of lysate, NAD, 2-hydroxy-3-phenylpropionic acid, polypeptide GP(Hyp)GAG, and peptidoglycan on halitosis-causing pathogenic bacteria in multi-species biofilm

Based on sequencing results, halitosis-causing pathogenic bacteria in the biofilm were identified. Four key halitosis-causing pathogenic bacteria were identified: *Fusobacterium nucleatum, Streptococcus mutans, Prevotella intermedia,* and *Solobacterium moorei.* All four bacteria had been proven to be positively correlated with VSC concentration. *Prevotella intermedia* is a periodontal pathogen; and it has also been demonstrated that *Prevotella intermedia* and *Fusobacterium nucleatum* can produce indole via the tryptophan metabolic pathway. Therefore, this example adopts lysate, NAD, 2-hydroxy-3-phenylpropionic acid, and polypeptide GP(Hyp)GAG to intervene in halitosis-causing pathogenic bacteria in the biofilm, evaluating changes in the relative abundance of pathogenic bacteria after intervention. Additionally, *Prevotella intermedia* is a periodontal pathogen that can produce indole via the tryptophan metabolic pathway. Therefore, in this example, peptidoglycan is also used to intervene in *Prevotella intermedia* in the biofilm, evaluating changes in its relative abundance after intervention.

Analysis results of the lysate group are shown in FIG. 9. In FIG. 9, A shows the effect of lysate intervention on the halitosis-causing pathogenic bacterium *Prevotella intermedia;* B shows the effect of lysate intervention on the halitosis-causing pathogenic bacterium *Streptococcus mutans;* C shows the effect of lysate intervention on the halitosis-causing pathogenic bacterium *Fusobacterium nucleatum;* and D shows the effect of lysate intervention on the halitosis-causing pathogenic bacterium *Solobacterium moorei.* In summary, ET-22.L, ET-22.HK, and lysate can all inhibit halitosis-causing pathogenic bacteria, and lysate has a significant inhibitory effect on the growth of *Streptococcus mutans.*

Analysis results of the NAD group are shown in FIG. 19. In FIG. 19, A is a diagram showing the change in relative abundance of halitosis-causing pathogenic bacteria *Prevotella intermedia* after NAD intervention, B is a diagram showing the change in relative abundance of halitosis-causing pathogenic bacteria *Streptococcus mutans* after NAD intervention, C is a diagram showing the change in relative abundance of halitosis-causing pathogenic bacteria *Fusobacterium nucleatum* after NAD intervention, and D is a diagram showing the change in relative abundance of halitosis-causing pathogenic bacteria *Solobacterium moorei* after NAD intervention. According to FIG. 19, it can be seen that, compared with the CK group, both ET-22 viable bacteria and ET-22 heat-inactivated bacteria can significantly inhibit the growth of halitosis-causing pathogenic bacteria, but the intervention effect of NAD is not obvious.

Analysis results of the 2-hydroxy-3-phenylpropionic acid group are shown in FIG. 29. In FIG. 29, A is a diagram showing the change in relative abundance of halitosis-causing pathogenic bacteria *Prevotella intermedia* after 2-hydroxy-3-phenylpropionic acid intervention, B is a diagram showing the change in relative abundance of halitosis-causing pathogenic bacteria *Streptococcus mutans* after 2-hydroxy-3-phenylpropionic acid intervention, C is a diagram showing the change in relative abundance of halitosis-causing pathogenic bacteria *Fusobacterium nucleatum* after 2-hydroxy-3-phenylpropionic acid intervention, and D is a diagram showing the change in relative abundance of halitosis-causing pathogenic bacteria *Solobacterium moorei* after 2-hydroxy-3-phenylpropionic acid intervention. According to FIG. 29, it can be seen that, 2-hydroxy-3-phenylpropionic acid can significantly inhibit the growth of *Solobacterium moorei* ($p<0.05$), and its inhibitory effect is better than that of ET-22 viable bacteria or ET-22 heat-inactivated bacterial cells.

Analysis results of the polypeptide GP(Hyp)GAG group are shown in FIG. 38. In FIG. 38, A shows the effect of polypeptide GP(Hyp)GAG intervention on halitosis-causing pathogenic bacteria *Prevotella intermedia,* B shows the effect of polypeptide GP(Hyp)GAG intervention on halitosis-causing pathogenic bacteria *Streptococcus mutans,* C shows the effect of polypeptide GP(Hyp)GAG intervention on halitosis-causing pathogenic bacteria *Fusobacterium nucleatum,* and D shows the effect of polypeptide GP(Hyp)GAG intervention on halitosis-causing pathogenic bacteria *Solobacterium moorei.* According to FIG. 38, it can be seen that, GP(Hyp)GAG can significantly inhibit the growth of *Solobacterium moorei* ($p<0.05$), and its inhibitory effect is better than that of ET-22 viable bacteria or ET-22 heat-inactivated bacteria.

Analysis results of the peptidoglycan group are shown in FIG. 48. Compared with the CK group, ET-22 viable bacteria, ET-22 heat-inactivated bacteria, and peptidoglycan all significantly inhibited the growth of the halitosis-causing pathogenic bacterium *Prevotella intermedia.*

6.3 Respective study of the effects of lysate, NAD, 2-hydroxy-3-phenylpropionic acid, polypeptide GP(Hyp)GAG, and peptidoglycan on virulence factors in multi-species biofilm

This example evaluated the effects of lysate, NAD, 2-hydroxy-3-phenylpropionic acid, polypeptide GP(Hyp) GAG, and peptidoglycan on halitosis virulence factors, including cdl, fadA, mgl, and gtfb. The gene cdl can regulate the expression of L-cysteine dehydrogenase, which can catalyze the degradation of protein substrate to produce VSC, thereby causing halitosis. *Fusobacterium nucleatum* secretes an amyloid-like adhesin (fadA) via the fap2-like autotransporter protein to enhance its virulence. The adhesin plays a key role in the pathogenicity of *Fusobacterium nucleatum,* and fadA refers to the mRNA encoding this adhesin. L-methionine-$\alpha$-deamino-$\gamma$-mercaptomethane-lyase (METase) can produce $\alpha$-ketobutyrate, ammonia, and $CH_3SH$ from L-methionine. Anaerobic bacteria can further produce large amounts of VSCs through METase encoded by mgl. gtfB is the most common virulence gene, encoding glucosyltransferases (Gtfs). Glycosyltransferases are involved in the adhesion of *Streptococcus mutans* and other bacteria to biofilm. Studies have shown that deletion of the gtfB gene in *Streptococcus mutans* can reduce bacterial accumulation.

Experimental methods include the following.

(1) Extraction of biofilm bacterial RNA: The extraction method for biofilm bacterial RNA (biofilm bacteria RNA) refers to the operation of RNAprep Pure Culture Bacteria/Cells Total RNA Extraction Kit from TIANGEN.

(2) Reverse Transcription

The reverse transcription system was shown in Table 2. The following reagents were added to a PCR tube in ice bath.

Table 2 RNA reverse transcription cDNA system

| Component | Usage amount |
|---|---|
| 5×FastKing-RT SuperMix | 4μL |
| RNA | 4μL |
| RNase-Free $ddH_2O$ | 12μL |

Reverse transcription reaction was performed on a PCR instrument according to the conditions in Table 3.

Table 3 PCR reaction program

| Reaction temperature | Reaction time | Description |
|---|---|---|
| 42°C | 15min | Genomic DNA removal and reverse transcription reaction |
| 95°C | 3min | Enzyme inactivation |

(3) RT-PCR detection

After the sample RNA was reverse-transcribing into cDNA, RT-PCR reaction was performed to detect biofilm-related virulence factors. The primer sequences were shown in Table 4. Experimental results were calculated using the $2^-$

$^{\triangle\triangle CT}$ method.

Table 4 RT-PCR primer sequence of biofilm-related virulence factor

| Target gene name | Sequence | Sequence No. | Tm(°C) |
|---|---|---|---|
| 16Sr DNA | F: AAGCGCGTCTAGGTTATGT | SEQ ID NO.1 | 60.2 |
| | R: TGTAGTTCCGCTTACCTCTCCAG | SEQ ID NO.2 | 62.4 |
| gtfb | F: ACGAACTTTGCCGTTATTGTCA | SEQ ID NO.3 | 56.5 |
| | R: AGCAATGCAGCCAATCTACAA | SEQ ID NO.4 | 55.9 |
| fadA | F: CACAAGCTGACGCTGCTAGA | SEQ ID NO.5 | 62 |
| | R: GTTACCAGCTCTTAAAGCTTG | SEQ ID NO.6 | 55.9 |
| Cdl | F: AATACAGGAATTGGGCTTGCT | SEQ ID NO.7 | 55.9 |
| | R: CTTCAGTTCCATAGGCTCTCATAA | SEQ ID NO.8 | 59.3 |
| mgl | F: TCGTGCTTATGAGCGATGTC | SEQ ID NO.9 | 60 |
| | R: GGAAGTCACCCTCGTGGATA | SEQ ID NO.10 | 62 |

Analysis results of the lysate group are shown in FIG. 10. In FIG. 10, A is a diagram showing the change in relative proportion of halitosis virulence factor cdl after lysate intervention, B is a diagram showing the change in relative proportion of halitosis virulence factor fadA after lysate intervention, C is a diagram showing the change in relative proportion of halitosis virulence factor mgl after lysate intervention, and D is a diagram showing the change in relative proportion of halitosis virulence factor gtfb after lysate intervention. According to FIG. 10, it can be seen that, compared with the CK group, ET-22 viable bacteria, ET-22 heat-inactivated bacteria, and lysate can all regulate the expression of virulence factors, and the inhibitory effect of lysate on the expression of virulence factor fadA is better than that of ET-22 viable bacteria and ET-22 heat-inactivated bacteria.

Analysis results of the NAD group are shown in FIG. 20. In FIG. 20, A is a diagram showing the change in relative proportion of halitosis virulence factor cdl after NAD intervention, B is a diagram showing the change in relative proportion of halitosis virulence factor fadA after NAD intervention, C is a diagram showing the change in relative proportion of halitosis virulence factor mgl after NAD intervention, and D is a diagram showing the change in relative proportion of halitosis virulence factor gtfb after NAD intervention. According to FIG. 20, it can be seen that, compared with the CK group, both ET-22 viable bacteria and ET-22 heat-inactivated bacteria can regulate the expression of virulence factors, but the intervention effect of NAD is not obvious. In summary, NAD mainly exerts breath-freshening and halitosis-alleviating and inhibiting effects by reducing volatile sulfur compounds (VSCs) in breath and volatile organic compounds (VOCs) in saliva within the oral cavity, and lowering the content of characteristic flavor compounds such as indole, propanoic acid, and hexanoic acid.

Analysis results of the 2-hydroxy-3-phenylpropionic acid group are shown in FIG. 30. In FIG. 30, A is a diagram showing the change in relative proportion of halitosis virulence factor cdl after 2-hydroxy-3-phenylpropionic acid intervention, B is a diagram showing the change in relative proportion of halitosis virulence factor fadA after 2-hydroxy-3-phenylpropionic acid intervention, C is a diagram showing the change in relative proportion of halitosis virulence factor mgl after 2-hydroxy-3-phenylpropionic acid intervention, and D is a diagram showing the change in relative proportion of halitosis virulence factor gtfb after 2-hydroxy-3-phenylpropionic acid intervention. According to FIG. 30, it can be seen that, 2-hydroxy-3-phenylpropionic acid can significantly regulate the four halitosis virulence factors ($p<0.05$), and its inhibitory effect on virulence factor fadA is better than that of ET22-L and ET22-HK.

Analysis results of the polypeptide GP(Hyp)GAG group are shown in FIG. 39. In FIG. 39, A shows the change in halitosis virulence factor cdl after polypeptide GP(Hyp)GAG intervention, B shows the change in halitosis virulence factor fadA after polypeptide GP(Hyp)GAG intervention, C shows the change in halitosis virulence factor mgl after polypeptide GP(Hyp)GAG intervention, and D shows the change in halitosis virulence factor gtfb after polypeptide GP(Hyp)GAG intervention. According to FIG. 39, it can be seen that, polypeptide GP(Hyp)GAG can significantly regulate the four halitosis virulence factors ($p<0.05$), and its inhibitory effects on virulence factors cdl and fadA are better than those of ET22-L and ET22-HK. Section 6.2 of the experiment has confirmed that GP(Hyp)GAG can inhibit the growth of *Fusobacterium nucleatum.* We speculate that GP(Hyp)GAG may inhibit the growth of *Fusobacterium nucleatum* by regulating the expression of cdl and fadA genes, thereby further reducing VSC production.

Analysis results of the peptidoglycan group are shown in FIG. 49. In FIG. 49, A is a diagram showing the change in relative proportion of halitosis virulence factor cdl after peptidoglycan intervention, B is a diagram showing the change in relative proportion of halitosis virulence factor fadA after peptidoglycan intervention, C is a diagram showing the change in

relative proportion of halitosis virulence factor mgl after peptidoglycan intervention, and D is a diagram showing the change in relative proportion of halitosis virulence factor gtfb after peptidoglycan intervention. According to FIG. 49, it can be seen that, compared with the CK group, ET-22 viable bacteria, ET-22 heat-inactivated bacteria, and peptidoglycan can all regulate the expression of virulence factors, but the regulatory effect is slightly weaker than that of ET-22 viable bacteria and ET-22 heat-inactivated bacteria. In summary, peptidoglycan mainly exerts breath-freshening and halitosis-alleviating and inhibiting effects by reducing volatile sulfur compounds (VSCs) in breath and volatile organic compounds (VOCs) in saliva within the oral cavity, and lowering the content of characteristic flavor compounds such as indole, propanoic acid, and hexanoic acid.

The following is indicated by the above research and analysis.

The embodiments of the present invention provides the use and composition of the lysate derived from *Lactobacillus paracasei* ET-22 in breath freshening and/or alleviating and inhibiting halitosis; the lysate can significantly reduce the content of volatile sulfur compounds (VSCs) in breath and volatile organic compounds (VOCs) in saliva within the oral cavity, and reduce the content of characteristic flavor compounds, and can alter the oral flora, inhibit the growth of oral biofilm and pathogenic bacteria, thereby having the effect of freshening breath and alleviating and inhibiting halitosis.

The embodiments of the present invention provides the use and composition of nicotinamide adenine dinucleotide in breath freshening; nicotinamide adenine dinucleotide can significantly reduce the content of volatile sulfur compounds (VSCs) in breath and volatile organic compounds (VOCs) in saliva within the oral cavity, and reduce the content of characteristic flavor compounds such as indole, propanoic acid, and hexanoic acid, having breath-freshening and halitosis-alleviating and inhibiting effects.

The embodiments of the present invention provides the use and composition of 2-hydroxy-3-phenylpropionic acid in breath freshening; 2-hydroxy-3-phenylpropionic acid can significantly inhibit the formation of characteristic flavor compounds, reduce the concentration of key volatile odor compounds of indole, propanoic acid, and hexanoic acid; and can alter the oral flora, especially reduce biofilm amount and thickness, and inhibit biofilm formation and pathogenic bacterial growth in the oral cavity, thereby having breath-freshening and halitosis-alleviating and inhibiting effects.

The embodiments of the present invention provides a breath-freshening peptide and its application and a composition including this peptide. This peptide (as shown in Formula 1) has the amino acid composition of Gly-Pro-Hyp-Gly-Ala-Gly. It can significantly inhibit the formation of volatile organic compounds in the oral cavity, and reduce the concentration of key volatile odor compounds of indole, propanoic acid, and hexanoic acid; and can alter the oral flora, and inhibit biofilm and pathogenic bacterial growth in the oral cavity, thereby having breath-freshening and halitosis-alleviating and inhibiting effects.

The embodiments of the present invention provides the use and composition of peptidoglycan derived from *Lactobacillus paracasei* ET-22 in breath freshening; peptidoglycan can significantly reduce the content of volatile sulfur compounds (VSCs) in breath and volatile organic compounds (VOCs) in saliva within the oral cavity, and reduce the content of characteristic flavor compounds such as indole, propanoic acid, and hexanoic acid, having effects of freshening breath and alleviating and inhibiting halitosis.

The embodiments of the present invention also provide *Lactobacillus paracasei* ET-22 bacteriocin, its preparation method and use, yielding a product with antioxidant property.

The *Lactobacillus paracasei* ET-22 bacteriocin in the embodiments of the present invention refers to the product obtained by extracting the water-soluble metabolites and exocytotic substance of *Lactobacillus paracasei* ET-22 from the bacterial sludge separated from the fermentation broth of *Lactobacillus paracasei* ET-22.

The embodiments of the present invention also provide a *Lactobacillus paracasei* ET-22 bacteriocin, including the exocytotic substance of *Lactobacillus paracasei* ET-22 and the metabolites of *Lactobacillus paracasei* ET-22, where the mass ratio of citric acid to L-methionine in the bacteriocin is greater than 15:1, and the deposit number of *Lactobacillus paracasei* ET-22 is CGMCC No. 15077.

In the embodiments of the present invention, the bacteriocin mainly includes components such as exocytotic substance and bacterial metabolites. As the extraction of the bacteriocin proceeds, the concentrations of citric acid and L-methionine in the bacteriocin gradually increase. Therefore, in the embodiments of the present invention, the bacteriocin is characterized by the concentrations of L-methionine and citric acid.

The *Lactobacillus paracasei* ET-22 bacteriocin in the embodiments of the present invention has antioxidant function and can be applied in food and pharmaceuticals. Compared to a mixture including intact dead cell + cell wall component + cell membrane component + cell-free supernatant, the bacteriocin in the embodiments of the present invention is a liquid or water-soluble powder, which has more advantages for use in some liquid beverages and pharmaceuticals.

In optional embodiments, the mass ratio of citric acid to L-methionine in the bacteriocin is 15-100:1. Specifically, it can be 15:1, 25:1, 35:1, 45:1, 55:1, 65:1, 75:1, 85:1, 100:1, or any value between 15-100:1, or any value >100:1.

Preferably, the mass ratio of citric acid to L-methionine in the bacteriocin is 35-45:1.

The embodiments of the present invention also provide a preparation method for the aforementioned *Lactobacillus paracasei* ET-22 bacteriocin, including the following steps: bacteriocin extraction, where the bacterial sludge

separated from the *Lactobacillus paracasei* ET-22 fermentation broth is extracted with a solvent to obtain a mixed solution; sterilization, where the supernatant is separated from the mixed solution and subjected to heat sterilization to obtain the bacteriocin.

In optional embodiments, the extraction temperature is 0-37°C, specifically, it may be 0°C, 2°C, 4°C, 6°C, 8°C, 10°C, 15°C, 20°C, 25°C, 30°C, 37°C, or any value between 0-37°C; preferably, the extraction temperature is 20-30°C.

In optional embodiments, the extraction time is 0.5-3 h, specifically, it may be 0.5 h, 1 h, 1.5 h, 2 h, 2.5 h, 3 h, or any value between 1-3 h; preferably, the extraction time is 30-150 min.

In optional embodiments, the total colony count of *Lactobacillus paracasei* ET-22 in the mixed solution is $0.5\times10^{11}$-$5\times10^{11}$, specifically it can be $0.5\times10^{11}$ CFU/ml, $0.7\times10^{11}$ CFU/ml, $0.9\times10^{11}$ CFU/ml, $1\times10^{11}$ CFU/ml, $2\times10^{11}$ CFU/ml, $3\times10^{11}$ CFU/ml, $4\times10^{11}$ CFU/ml, $5\times10^{11}$ CFU/ml, or any value between $0.5\times10^{11}$-$5\times10^{11}$ CFU/ml; preferably, the total colony count of *Lactobacillus paracasei* ET-22 in the mixed solution is $2\times10^{11}$-$4\times10^{11}$ CFU/ml.

In optional embodiments, the heat sterilization temperature is 70-121°C, specifically, it can be 70°C, 80°C, 90°C, 100°C, 110°C, 121°C, or any value between 70-121°C; preferably, the heat sterilization temperature is 70-100°C.

In optional embodiments, the heat sterilization time is 5-30 min, specifically, it can be 5 min, 10 min, 15 min, 20 min, 25 min, 30 min, or any value between 5-30 min; preferably, the heat sterilization time is 10-16 min.

In optional embodiments, the method further includes a fermentation step: performing liquid cultivation of *Lactobacillus paracasei* ET-22, and stopping fermentation when the logarithmic growth phase is reached.

Preferably, the fermentation step includes: inoculating *Lactobacillus paracasei* ET-22 fermentation seed solution into a culture medium, and fermenting at a temperature of 30-40°C, a rotational speed of 60-80 rpm, and a pH of 5.5-6.0 for 12-14 h to obtain the *Lactobacillus paracasei* ET-22 fermentation broth.

Preferably, the preparation of the *Lactobacillus paracasei* ET-22 fermentation seed solution includes: subjecting the *Lactobacillus paracasei* ET-22 strain to activation, purification, primary seed solution cultivation, secondary seed solution expansion, tertiary seed solution cultivation, and fermentation seed preparation, and obtaining the *Lactobacillus paracasei* ET-22 fermentation seed solution.

Preferably, the culture medium is MRS liquid medium.

In optional embodiments, the solvent is water; preferably, the solvent is sterile water, specifically, sterile purified water can be chosen.

Preferably, after the sterilization step, the bacteriocin is further freeze-dried to obtain freeze-dried bacteriocin.

In the embodiments of the present invention, the bacteriocin obtained after sterilization is a liquid, in which the mass ratio of citric acid to L-methionine is greater than 15:1, the concentration of L-methionine is greater than 0.05 mg/mL, and the concentration of citric acid is greater than 3 mg/mL. In some embodiments, the concentration of L-methionine is 0.05-0.2 mg/mL and the concentration of citric acid is 3-5 mg/mL.

Specifically, in the embodiments of the present invention, separation of bacterial sludge and supernatant can be achieved using existing separation methods, for example, centrifugation is selected to achieve solid-liquid separation. In some embodiments, the bacteriocin has an L-methionine concentration of 0.05 mg/ml, 0.07 mg/ml, 0.09 mg/ml, 0.11 mg/ml, 0.13 mg/ml, 0.15 mg/ml, 0.17 mg/ml, 0.19 mg/ml, 0.2 mg/ml, or any value between 0.05-0.2 mg/ml, or any value >0.2 mg/ml; citric acid concentration of 3 mg/ml, 3.5 mg/ml, 4 mg/ml, 4.5 mg/ml, 5 mg/ml, or any value between 3-5 mg/ml, or any value >5 mg/ml.

Preferably, the bacteriocin has an L-methionine concentration of 0.05-0.1 mg/ml and a citric acid concentration of 3-4 mg/ml.

After the sterilization step, the bacteriocin is freeze-dried to obtain a freeze-dried bacteriocin, where the solvent is removed. The mass ratio of citric acid to L-methionine in the freeze-dried bacteriocin is greater than 15:1.

Specifically, in some embodiments, the *Lactobacillus paracasei* ET-22 bacteriocin is prepared by the following steps.

1. Tertiary seed preparation

1.1 Standard cryovial

Prepared uniformly from purified strains, which are aliquoted into 1.5 mL centrifuge tubes, not less than 50 tubes, and stored in a -80°C freezer, the shelf life not exceeding 6 months.

1.2 Cryovial activation

One portion of strain stored at -80°C is taken and thawed at room temperature; aseptically, 200 μL of bacterial suspension is inoculated into 10 mL of seed liquid medium, followed by standing and incubating at 37°C for 8-10 h.

1.3 Primary purification

The cultured bacterial suspension is diluted and spread, with dilution of -4, -5, and -6. 2 MRS solid plates are prepared for each dilution, followed by inverted incubation at 37°C for 48h-72h until distinct colonies form on the plates. Single colonies of uniform size are picked with an inoculation loop and transferred into 5 tubes containing 10 mL MRS liquid medium, respectively, and then subjected to standing and incubating at 37°C for 18-20 h.

1.4 Secondary purification

The cultured primary-purified bacterial suspension is diluted and spread, with dilution of -4, -5, and -6. 2 MRS solid plates are prepared for each dilution, followed by inverted incubation at 37°C for 48h-72h until distinct colonies form on the plates. Single colonies of uniform size are picked with an inoculation loop and transferred into 5 tubes containing 10 mL MRS liquid medium, respectively, and then subjected to standing and incubating at 37°C for 18-20 h.

1.5 Primary seed preparation

One tube of cultured secondary-purified bacterial suspension is selected. Using a pipette, 200 μL of bacterial suspension is transferred into each of 5 tubes containing 10 mL MRS liquid medium, and subjected to standing and incubating at 37°C for 8-10 h.

1.6 Secondary seed preparation

4 tubes of cultured primary seed are selected, 4 mL of bacterial solution is injected into each of 4 bottles containing 80 mL MRS liquid medium, and subjected to standing and incubating at 37°C for 9-11 h.

1.7 Tertiary seed preparation

The cultured secondary seed is poured into each of 2 bottles containing 1.8 L MRS liquid medium, and subjected to standing and incubating at 37°C for 11-13 h.

1.8 Temporary storage of tertiary seed

The prepared tertiary seed can be stored at 4°C for no more than 6 h.

1.9 Process quality control

(1) Endpoint determination indicators for seed at all levels: pH 3.8-4.2, OD600 ≥ 2.0.
(2) Purity test: the bacterial cell morphology is observed under a microscope. Under 100× oil immersion lens, the bacterial cells are intact and in the shape of short rods, and present singly or in chains.
(3) Contaminant test: tertiary seed solution is tested for contaminants including *Escherichia coli* and non-lactic acid bacteria.

2. Fermentation seed preparation

2.1 Inoculation

(1) Turn on a stirrer and temperature control program, and set a rotational speed to 80 rpm and temperature to 36°C.
(2) Open a nitrogen inlet valve, and introduce a small nitrogen gas flow into a fermenter to maintain positive pressure.
(3) During inoculation, pour alcohol onto an inoculation ring and ignite to form a flame ring.
(4) Unscrew the upper port of the inoculation device, pour the cultured seed into the sterile area above the flame, with an inoculation amount of 2.5%.
(5) After inoculation, tighten the upper cover of the inoculation device, close the inoculation valve, and extinguish the alcohol flame ring.
(6) After completion of inoculation, continue to introduce a small nitrogen gas flow into the fermenter for 5-10 min.

(7) Close the nitrogen inlet and exhaust valves, control fermenter pressure at 0.01-0.03 MPa for pressurized fermentation.

2.2 Fermentation

(1) Set fermentation parameters: rotational speed of 80 rpm, and temperature of 36°C.
(2) Set fermentation time to 11h-13h, and monitor fermentation status every 2h, including pH, OD600, temperature, and rotational speed.
(3) After the ending of fermentation, proceed to inoculate the fermenter, or cool to 10-20°C and store for no more than 6 h.

2.3 Process quality control

(1) Seed tank fermentation endpoint determination indicators: pH 3.8-4.2, OD600 ≥ 2.0.
(2) Purity test: the bacterial cell morphology of seed tank endpoint fermentation broth is observed under a microscope. Under 100× oil immersion lens, the bacterial cells are intact and in the shape of short rods, and present singly or in chains.
(3) Contaminant test: the seed tank endpoint fermentation broth is tested for contaminants including *Escherichia coli* and non-lactic acid bacteria.

3. Fermentation broth cultivation

3.1 Inoculation

(1) Turn on the stirrer and temperature control program, and set a rotational speed to 80 rpm and temperature to 36°C.
(2) Sterilize the inoculation pipeline with steam for 30 min.
(3) Close the steam valve, and open the seed tank bottom valve and fermenter inoculation valve for inoculation, with an inoculation volume of 2%.
(4) After completion of inoculation, close the fermenter inoculation valve and clean the inoculation pipeline.

3.2 Fermentation

(1) Set fermentation parameters: temperature of 30-40°C, rotational speed of 60-80 rpm, and constant pH of 5.5-6.0 for fermentation.
(2) Set fermentation time to 12-14h, and monitor fermentation status every 2h, including pH, OD600, temperature, and rotational speed.
(3) After the ending of fermentation, proceed to perform centrifugation, or cool to 10-20°C and store for no more than 4 h.

3.3 Bacterial sludge separation

(1) Turn on the operating-water supply valve of a centrifuge, with total pressure of operating-water pipeline >3 bar, and mechanical seal water pressure maintained at 1.8-2.5 bar.
(2) Start the centrifuge until the centrifuge speed reaches 11600-11800 rpm, perform slag discharge operation automatically by a program, and keep the main interface drum indicator stay on.
(3) Open a feed valve, make the *Lactobacillus paracasei* ET-22 fermentation broth enter the centrifuge drum for centrifugation, and obtain the bacterial sludge.
(4) Set centrifugation process parameters: a feed rate of 600 L/h, and a slag discharge time of 200 s.

4. Bacteriocin extraction

4.1 Bacterial sludge transfer

The collected bacterial sludge is transferred to a sterile fermenter for extraction.

4.2 Extraction conditions

The bacterial sludge is extracted by mixing it with sterile purified water to obtain a mixed solution. The total colony count of *Lactobacillus paracasei* ET-22 in the mixed solution is $1\times10^{11}$-$3\times10^{11}$ CFU/ml, with the extraction temperature of 0-37°C, the extraction time of 0.5-3 h, and the extraction speed of 50-100 rpm.

It should be noted that in actual production, the OD600 value of the *Lactobacillus paracasei* ET-22 fermentation broth in step 3.2 can be adjusted, while controlling the amount of sterile purified water added in the extraction step, so that the volume of the mixed solution is 1% of the volume of the *Lactobacillus paracasei* ET-22 fermentation broth, so as to more easily adjust the total colony count of *Lactobacillus paracasei* ET-22 in the mixed solution.

5. Centrifugal separation

(1) Turn on the operating-water supply valve of a centrifuge, with total pressure of operating-water pipeline >3 bar, and mechanical seal water pressure maintained at 1.8-2.5 bar.
(2) Start the centrifuge, perform slag dischar operation automatically by a program when the centrifuge speed reaches 11600-11800 rpm, and keep the main interface drum indicator stay on.
(3) Open the feed valve, and make the mixed solution enter the centrifuge drum for centrifugation.
(4) Set centrifugation process parameters: a feed rate of 600 L/h, and a slag discharge time of 200 s.
(5) Transfer the centrifugal supernatant to a collection tank.

6. Packaging and sterilization

The filled supernatant in bottles is subjected to heat sterilization to obtain the bacteriocin. Sterilization conditions for each strain are as follows.

Sterilization conditions for *Lactobacillus paracasei* ET-22: 70-121°C, 5-30 min.

The embodiments of the present invention also provide the use of the *Lactobacillus paracasei* ET-22 bacteriocin as described in any of the preceding embodiments in preparing food, where the food includes at least one of dairy products and beverages.

The embodiments of the present invention also provide the use of the *Lactobacillus paracasei* ET-22 bacteriocin as described in any of the preceding embodiments in preparing a composition, where the composition includes an antioxidant composition; preferably, the composition is selected from at least one of pharmaceuticals, health foods, and feed.

The *Lactobacillus paracasei* ET-22 bacteriocin in the embodiments of the present invention has antioxidant function and can be applied in food and pharmaceuticals. Compared to a mixture including intact dead cell + cell wall component + cell membrane component + cell-free supernatant, the bacteriocin in the embodiments of the present invention is a liquid or water-soluble powder, which has more advantages for use in some liquid beverages and pharmaceuticals.

Example 3

This example provided a *Lactobacillus paracasei* ET-22 bacteriocin. As shown in FIG. 50, its preparation method included the following steps.

1. Tertiary seed preparation

1.1 Standard Cryovial: The standard cryovial was prepared uniformly from purified strains, which are aliquoted into 1.5 mL centrifuge tubes, not less than 50 tubes, and stored in a -80°C freezer, the shelf life not exceeding 6 months.

1.2 Cryovial activation: One portion of strain stored at -80°C was taken and thawed at room temperature; aseptically, 200 μL of bacterial suspension was inoculated into 10 mL of seed liquid medium, followed by standing and incubating at 37°C for 8-10 h.

1.3 Primary purification: The cultured bacterial suspension was diluted and spread, with dilution of -4, -5, and -6. 2 MRS solid plates were prepared for each dilution, followed by inverted incubation at 37°C for 48h-72h until distinct colonies form on the plates. Single colonies of uniform size were picked with an inoculation loop and transferred into 5 tubes containing 10 mL MRS liquid medium, respectively, and then subjected to standing and incubating at 37°C for 18-20 h.
1.4 Secondary purification: The cultured primary-purified bacterial suspension was diluted and spread, with dilution of -4, -5, and -6. 2 MRS solid plates were prepared for each dilution, followed by inverted incubation at 37°C for 48h-72h until distinct colonies form on the plates. Single colonies of uniform size were picked with an inoculation loop and transferred into 5 tubes containing 10 mL MRS liquid medium, respectively, and then subjected to standing and incubating at 37°C for 18-20 h.
1.5 Primary seed preparation: One tube of cultured secondary-purified bacterial suspension was selected. Using a pipette, 200 μL of bacterial suspension was transferred into each of 5 tubes containing 10 mL MRS liquid medium, and subjected to standing and incubating at 37°C for 8-10 h.
1.6 Secondary seed preparation: 4 tubes of cultured primary seed were selected, 4 mL of bacterial solution is injected into each of 4 bottles containing 80 mL MRS liquid medium, and subjected to standing and incubating at 37°C for 9-11 h.
1.7 Tertiary seed preparation: The cultured secondary seed was poured into each of 2 bottles containing 1.8 L MRS liquid medium, and subjected to standing and incubating at 37°C for 11-13 h.
1.8 Temporary storage of tertiary seed: The prepared tertiary seed can be stored at 4°C for no more than 6 h.

1.9 Process quality control

(1) Endpoint determination indicators for seed at all levels: pH 3.8-4.2, OD600 ≥ 2.0.
(2) Purity test: the bacterial cell morphology was observed under a microscope. Under 100× oil immersion lens, the bacterial cells were intact and in the shape of short rods, and present singly or in chains.
(3) Contaminant test: tertiary seed solution was tested for contaminants including *Escherichia coli* and non-lactic acid bacteria.

Formulation of tertiary seed fermentation medium

| Formulation 1 | |
|---|---|
| Name of raw and adjuvant materials | Amount (kg/100kg) |
| FP102 Yeast peptone | 1.0 |
| FM828 Yeast extract powder | 0.75 |
| Glucose (mixed sterilization) | 2.0 |
| Diammonium hydrogen phosphate | 0.15 |
| Potassium citrate | 0.25 |
| Sodium acetate (anhydrous) | 0.5 |
| Magnesium sulfate (heptahydrate) | 0.058 |
| Manganese sulfate (monohydrate) | 0.019 |
| Tween-80 | 0.1 |
| Agar | 1.5 (added only in solid medium) |

pH value: 6.2-6.4; sterilization conditions: 121°C, 15-20 min.

2. Fermentation seed preparation

2.1 Inoculation

(1) Turn on a stirrer and temperature control program, and set a rotational speed to 80 rpm and temperature to 36°C.
(2) Open a nitrogen inlet valve, and introduce a small nitrogen gas flow into a fermenter to maintain positive pressure.
(3) During inoculation, pour alcohol onto an inoculation ring and ignite to form a flame ring.
(4) Unscrew the upper port of the inoculation device, pour the cultured seed into the sterile area above the flame, with an inoculation amount of 2.5%.
(5) After inoculation, tighten the upper cover of the inoculation device, close the inoculation valve, and extinguish the alcohol flame ring.
(6) After completion of inoculation, continue to introduce a small nitrogen gas flow into the fermenter for 5-10 min.
(7) Close the nitrogen inlet and exhaust valves, control fermenter pressure at 0.01-0.03 MPa for pressurized fermentation.

2.2 Fermentation

(1) Set fermentation parameters: rotational speed of 80 rpm, and temperature of 36°C.
(2) Set fermentation time to 11h-13h, and monitor fermentation status every 2h, including pH, OD600, temperature, and rotational speed.
(3) After the ending of fermentation, proceed to inoculate the fermenter, or cool to 10-20°C and store for no more than 6 h.

2.3 Process quality control

(1) Seed tank fermentation endpoint determination indicators: pH 3.8-4.2, OD600 ≥ 2.0.
(2) Purity test: the bacterial cell morphology of seed tank endpoint fermentation broth is observed under a microscope. Under 100× oil immersion lens, the bacterial cells are intact and in the shape of short rods, and present singly or in chains.
(3) Contaminant test: the seed tank endpoint fermentation broth is tested for contaminants including *Escherichia coli* and non-lactic acid bacteria.

Formulation of seed tank fermentation medium

| Formulation 2 | |
|---|---|
| Name of raw and adjuvant materials | Amount (kg/100kg) |
| FP102 Yeast peptone | 1.0 |
| FM828 Yeast extract powder | 0.75 |
| Glucose (mixed sterilization) | 2.0 |
| Diammonium hydrogen phosphate | 0.15 |
| Potassium citrate | 0.25 |
| Sodium acetate (anhydrous) | 0.5 |
| Magnesium sulfate (heptahydrate) | 0.058 |
| Manganese sulfate (monohydrate) | 0.019 |
| Tween-80 | 0.1 |

pH value: 6.2-6.4; sterilization conditions: 121°C, 15-20 min.

3. Fermentation broth cultivation

3.1 Inoculation

(1) Turn on a stirrer and temperature control program, and set a rotational speed to 80 rpm and temperature to 36°C.
(2) Sterilize the inoculation pipeline with steam for 30 min.
(3) Close the steam valve, and open the seed tank bottom valve and fermenter inoculation valve for inoculation, with an inoculation volume of 2%.
(4) After completion of inoculation, close the fermenter inoculation valve and clean the inoculation pipeline.

3.2 Fermentation

(1) Set fermentation parameters: temperature of 36°C, rotational speed of 80 rpm, and constant pH of 5.75 for fermentation.
(2) Set fermentation time to 12-14h, and monitor fermentation status every 2h, including pH, OD600, temperature, and rotational speed.
(3) After the ending of fermentation, proceed to perform centrifugation, or cool to 10-20°C and store for no more than 4 h.

Formulation of fermenter fermentation medium

| Formulation 3 | |
|---|---|
| Name of raw and adjuvant materials | Amount (kg/100kg) |
| FP102 Yeast peptone | 2.0 |
| FM828 Yeast extract powder | 1.0 |
| Glucose (sterilization alone) | 6.0 |
| Diammonium hydrogen phosphate | 0.15 |
| Potassium citrate | 0.25 |
| Sodium acetate (anhydrous) | 0.5 |
| Magnesium sulfate (heptahydrate) | 0.058 |
| Manganese sulfate (monohydrate) | 0.019 |
| Tween-80 | 0.1 |

pH value: 6.2-6.4; sterilization conditions: 121°C, 15-20 min.

3.3 Bacterial sludge separation

(1) Turn on the operating-water supply valve of a centrifuge, with total pressure of operating-water pipeline >3 bar, and mechanical seal water pressure maintained at 1.8-2.5 bar.
(2) Start the centrifuge until the centrifuge speed reaches 11600-11800 rpm, perform slag discharge operation automatically by a program, and keep the main interface drum indicator stay on.
(3) Open a feed valve, make the *Lactobacillus paracasei* ET-22 fermentation broth enter the centrifuge drum for centrifugation, and obtain the bacterial sludge.
(4) Set centrifugation process parameters: a feed rate of 600 L/h, and a slag discharge time of 200 s.

4. Bacteriocin extraction

4.1 Bacterial sludge transfer

The collected bacterial sludge was transferred to a sterile fermenter for extraction.

4.2 Extraction conditions

The bacterial sludge was extracted by mixing it with sterile purified water and resuspending to obtain a mixed solution. The total colony counts of *Lactobacillus paracasei* ET-22 in the mixed solution were $1\times10^{11}$ CFU/ml, $1.5\times10^{11}$ CFU/ml, and $3\times10^{11}$ CFU/ml, respectively. Extraction temperatures were 4°C, 25°C, and 37°C, respectively. Extraction time were 1 h, 2 h, and 3 h, respectively. Extraction speed was 70 rpm.

5. Centrifugal separation

(1) Turn on the operating-water supply valve of a centrifuge, with total pressure of operating-water pipeline >3 bar, and mechanical seal water pressure maintained at 1.8-2.5 bar.
(2) Start the centrifuge, perform slag dischar operation automatically by a program when the centrifuge speed reaches 11600-11800 rpm, and keep the main interface drum indicator stay on.
(3) Open the feed valve, and make the mixed solution enter the centrifuge drum for centrifugation.
(4) Set centrifugation process parameters: a feed rate of 600 L/h, and a slag discharge time of 200 s.
(5) Transfer the centrifugal supernatant to a collection tank.

6. Packaging and sterilization

The filled supernatant in bottles was subjected to heat sterilization to obtain the bacteriocin. Sterilization conditions for *Lactobacillus paracasei* ET-22 includes 75°C, 10 min. The obtained *Lactobacillus paracasei* ET-22 bacteriocin was shown in FIG. 51.

7. Quantitative detection

The concentrations of L-methionine and citric acid in the product obtained in Example 3 were detected. The detection method included the following steps.

(1) Preparation of standard working solutions

Standard stock solution: an appropriate amount of each standard was weighed precisely and separately (i.e., L-methionine and citric acid, to an accuracy of 0.1 mg), dissolved in water, prepared into separate standard stock solution with a concentration of 5 mg/mL, and stored at -20°C.

Mixed standard intermediate solution: an appropriate volume of standard stock solution was pipetted accurately and separately, diluted to volume with water to prepare a mixed standard intermediate solution with a concentration of 500 μg/mL, and refrigerated at 4°C for storage.

Mixed standard working solution: the mixed standard intermediate solution was diluted serially with water as needed to prepare mixed standard working solutions with concentrations of 1 μg/mL, 5 μg/mL, 10 μg/mL, 20 μg/mL, 50 μg/mL, 100 μg/mL, and 200 μg/mL, respectively. These solutions were prepared freshly prior to use.

(2) Preparation of elution solution

0.1% phosphoric acid aqueous solution: 1 mL of phosphoric acid was taken and diluted to 1000 mL with water, followed by mixing well. The solution was prepared freshly prior to use.

0.1% phosphoric acid acetonitrile solution: 1 mL of phosphoric acid was taken and diluted to 1000 mL with acetonitrile, followed by mixing well. The solution was prepared freshly prior to use.

(3) Preparation of test sample solution

Liquid sample: the sample of *Lactobacillus paracasei* ET-22 bacteriocin was mixed well, directly pipetted 1 mL,

and centrifuged at 10000 rpm for 10 min at 4°C; and the supernatant was diluted to be within a linear range for column analysis.

(4) Detection and analysis

High-performance liquid chromatography (HPLC) was used to test the sample solution and standard working solution, and the corresponding chromatographic peak areas were determined. Using the concentration of the standard working solution as the horizontal coordinate and the chromatographic peak area as the vertical coordinate, a standard curve was drawn. Based on the detection result of the sample solutions from step (3) (as shown in FIG. 52, where 1 is L-methionine and 2 is citric acid), and combined with the standard curve, the concentrations of L-methionine and citric acid in the extraction step of the bacteriocin were calculated. FIG. 52 was the liquid chromatogram of the bacteriocin sample obtained in Example 3.

Chromatographic analysis conditions: an LC-20A analysis system was used, and the chromatographic column was Poroshell 120Aq-C18 column (4.6 mm × 150 mm, 2.7 μm); mobile phase A was 0.1% phosphoric acid aqueous solution, mobile phase B was 0.1% phosphoric acid acetonitrile solution, and gradient elution program was shown in Table 5; the flow rate was 0.7 mL/min, the detection wavelength was 210 nm, the column temperature was 30°C, and the injection volume was 5 μL.

Table 5 Gradient elution program

| Time (min) | Mobile phase A (%) | Mobile phase B (%) |
|---|---|---|
| 0 | 100 | 0 |
| 10 | 100 | 0 |
| 33 | 95 | 5 |
| 45 | 95 | 5 |

It was calculated that when the mixed solution had a total colony count of *Lactobacillus paracasei* ET-22 of $3\times10^{11}$ CFU/ml, the extraction temperature was 25°C and the extraction time was 1 h, the L-methionine concentration was 0.09 mg/100 ml and the citric acid concentration was 3.68 mg/100 ml in the bacteriocin.

(5) Calculation of colony count before inactivation of inactivated bacterial cells

Citric acid and L-methionine can be used as target substances to calculate the colony count in the fermentation broth before bacteriocin extraction.

When citric acid was used as the target substance to calculate the corresponding colony count in the fermentation broth before bacteriocin extraction, the regression equation used was y = 2.068x + 10.874; where x represented the concentration of the target substance, with a unit of mg/100g; y represented the colony count, with a unit of $10^9$ CFU/ml. Based on the calculated concentration of the target substance, it was substituted into the regression equation to calculate the colony count of the inactivated bacterial cells before inactivation.

When L-methionine was used as the target substance to calculate the corresponding colony count in the fermentation broth before bacteriocin extraction, the regression equation used was y = 74.439x - 1.822; where x represented the concentration of the target substance, with a unit of mg/100g; y represented the colony count, with a unit of $10^9$ CFU/ml. Based on the calculated concentration of the target substance, it was substituted into the regression equation to calculate the colony count of the inactivated bacterial cells before inactivation.

| Specialized strain | Dependent variable y | Independent variable x | Range of values | | Regression equation | Regression equation $R^2$ |
|---|---|---|---|---|---|---|
| | | | y (10^9 CFU/ml) | x (mg/100mL) | | |
| ET-22 | Colony count | L-methionine | 2-300 | 0.04~4.12 | y = 74.439 x - 1.822 | 0.9959 |
| | Colony count | Citric acid | 2-300 | 1.23~138.71 | v = 2.068 x - 10.874 | 0.9749 |

Comparative Example 1

This comparative example provided fermentation inactivated bacteria of *Lactobacillus paracasei* ET-22. Its preparation method included the following steps: 1. tertiary seed preparation, which was same as Example 3; 2.

fermentation seed preparation, which was same as Example 3; 3. fermentation broth cultivation, which was same as Example 3.

4. Bacterial cell inactivation

The bacterial sludge was mixed with sterile purified water and resuspended to obtain a mixed solution (its concentration same as Example 3). The mixed solution was subjected to heat sterilization, where the heat sterilization temperatures were 70°C, 80°C, 90°C, 100°C, and 121°C, respectively, and the heat sterilization time was 10 min.

Test Example 1: 2,2-diphenyl-1-picrylhydrazyl (DPPH) radical scavenging

200 μL of 0.2 mM DPPH solution was mixed with bacteriocin or inactivated bacteria that was prepared using 200 μL of the mixed solution ($10^9$ CFU/ml) as raw material, and incubated at 25°C in the dark for 30 min. For the control group, an equal volume of PBS (pH 7.4) was used. For the blank group, an equal amount of PBS (pH 7.4) was used instead of the DPPH radical solution. After centrifugation at 2,000×g for 10 minutes, the absorbance of the solution was measured at 517 nm. Calculation formula was as follows:

$$\text{Scavenging ratio }(\%) = [1 - \frac{A_{smple} - A_{blank}}{A_{control}}] \times 100$$

Test Example 2: Hydroxyl radical scavenging

Bacteriocin or inactivated bacteria prepared using a total of 1.0 mL of the mixed solution ($10^9$ CFU/ml) as raw material was added to a mixture containing 2.5 mM 1,10-phenanthroline (1.0 mL), 2.5 mM $FeSO_4$ (1.0 mL), and PBS (1.0 mL, pH 7.4). After addition of 20 mM $H_2O_2$ (1.0 mL), the mixture was incubated in a 37°C water bath for 90 min. Absorbance at 536 nm was obtained. Its hydroxyl radical scavenging activity was calculated as follows:

$$\text{Scavenging ratio }(\%) = (\frac{A_{sample} - A_{blank}}{A_{control} - A_{blank}}) \times 100$$

In the embodiments of the present invention, LC-MS is used to detect the components of the inactivated bacteria obtained in Comparative Example 1. Components with the peak area greater than $10^4$ in the detection result are screened to obtain target substance characterizing the inactivated bacteria components. Target substance screening criteria include: ① The component does not exist before heat sterilization, or its content before heat sterilization is far lower than that after heat sterilization; ② It can stably exist under different heat sterilization conditions within the experimental range (its content variation amplitude ≤ 20%). It is found that six types of substances can be used as potential detection target substances: Proly-Alanine, L-Methionine, Citric Acid, bAsp-Leu, bAsp-Phe, Antiarrhythmic peptide, GRPPK. Simultaneously, six detection targets in the bacteriocin are detected. It is found that only citric acid and L-methionine are present in the bacteriocin; the other four detection targets are not extracted or their content was below the detection limit. Therefore, in the embodiments of the present invention, the bacteriocin is characterized by citric acid and L-methionine as detection targets.

The test results for the hydroxyl radical scavenging ability of the bacteriocin obtained in Example 3 are shown in FIG. 53. It can be seen from FIG. 53 that, when the extraction concentration is $3\times10^{11}$ CFU/ml, the extraction temperature is 25°C, and the extraction time is 1 h, the bacteriocin has better hydroxyl radical scavenging ability. The test results for the DPPH radical scavenging ability of the bacteriocin obtained in Example 3 are shown in FIG. 54. In FIG. 54, K1-K9 extraction temperatures and times are: 4°C, 1h; 4°C, 2h; 4°C, 3h; 25°C, 1h; 25°C, 2h; 25°C, 3h; 37°C, 1h; 37°C, 2h; and 37°C, 3h, respectively. K10-K18 extraction temperatures and times are: 4°C, 1h; 4°C, 2h; 4°C, 3h; 25°C, 1h; 25°C, 2h; 25°C, 3h; 37°C, 1h; 37°C, 2h; and 37°C, 3h, respectively. K19-K27 extraction temperatures and times are: 4°C, 1h; 4°C, 2h; 4°C, 3h; 25°C, 1h; 25°C, 2h; 25°C, 3h; 37°C, 1h; 37°C, 2h; and 37°C, 3h, respectively. In FIG. 54, the effect of extraction conditions on DPPH radicals is relatively small.

The hydroxyl radical scavenging ability and DPPH radical scavenging ability of the postbiotics obtained in Comparative Example 1 are shown in FIG. 55. The DPPH radical scavenging ability of the bacteriocin is insensitive to temperature changes at first, but decreases significantly under high-temperature condition of 121°C.

Finally, it should be noted that: the above embodiments are only used to illustrate the technical solutions of the present invention and are not intended to limit them; although the present invention has been described in detail with

reference to the above-mentioned embodiments, those of ordinary skill in the art should understand that they can still modify the technical solutions described in the above-mentioned embodiments, or equivalently replace some or all of the technical features; and such modifications or replacements do not cause the essence of the corresponding technical solutions to depart from the scope of the technical solutions of the embodiments of the present invention.

## Claims

1. Use of a lysate derived from *Lactobacillus paracasei* ET-22 in freshening breath and/or alleviating and inhibiting halitosis, wherein the lysate is derived from heat-inactivated bacterial cells of *Lactobacillus paracasei* ET-22; the lysate at least comprises 2-hydroxy-3-phenylpropionic acid, NAD, and a polypeptide; the polypeptide has a structure shown in Formula 1;

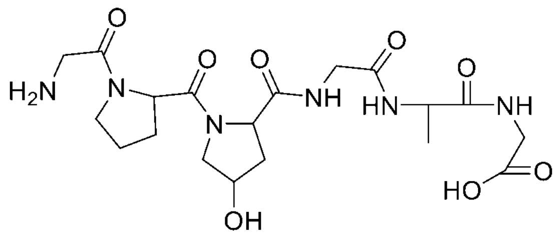

Formula 1.

2. The use according to claim 1, wherein based on a total volume of the lysate, a mass of 2-hydroxy-3-phenylpropionic acid is not less than 145 mg/L, a mass of NAD is not less than 0.17 mg/L, and a mass of the polypeptide is not less than 0.24 mg/L.

3. The use according to claim 1 or 2, wherein the lysate further comprises one or more of 6-hydroxyhexanoic acid, 3-aminoglutaric acid, 2-hydroxy-3-phenylpropionic acid, 2-hydroxy-3-(4-hydroxyphenyl)propanoic acid, and Asp-Phe.

4. The use according to any one of claims 1-3, wherein a preparation method of the lysate comprises the following steps:

   performing liquid culture of *Lactobacillus paracasei* ET-22, stopping fermentation upon reaching a logarithmic growth phase, and collecting bacterial cells;
   resuspending the collected bacterial cells in sterile water, performing heat inactivation, and collecting supernatant to obtain the lysate.

5. The use according to claim 4, wherein a heat inactivation temperature is 95-150°C and a heat inactivation time is 1-15 min.

6. A peptide having breath-freshening and/or halitosis-alleviating and inhibiting effects, wherein the peptide has a structure shown in Formula 1;

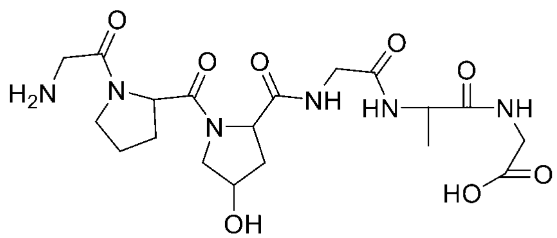

Formula 1.

7. The peptide according to claim 6, wherein the peptide is derived from postbiotic of *Lactobacillus paracasei* ET-22.

8. A composition for freshening breath and/or alleviating and inhibiting halitosis, comprising

   a lysate, wherein the lysate is derived from heat-inactivated bacterial cells of *Lactobacillus paracasei* ET-22, the lysate at least comprises 2-hydroxy-3-phenylpropionic acid, NAD, and a polypeptide, and the polypeptide has a structure shown in Formula 1;

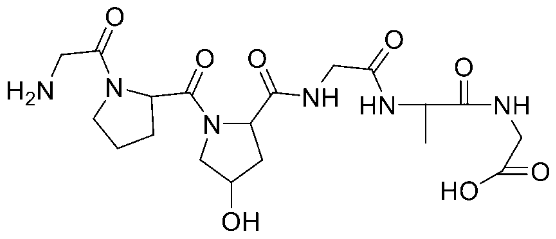

Formula 1;

or, comprising nicotinamide adenine dinucleotide;
or, comprising 2-hydroxy-3-phenylpropionic acid;
or, comprising the peptide according to claim 6 or 7;
or, comprising peptidoglycan, wherein the peptidoglycan is derived from heat-inactivated bacterial cells of *Lactobacillus paracasei* ET-22.

**9.** The composition according to claim 8, wherein the composition is one or more of a food composition, a pharmaceutical composition, and an oral cleaning composition.

**10.** The composition according to claim 9, wherein the food composition comprises one or more of dairy beverage, tea, coffee, chewing gum, tooth-cleaning candy, tablet candy, and pet jerky.

**11.** The composition according to claim 9, wherein the oral cleaning composition comprises one or more of toothpaste, tooth-cleaning powder, mouthwash, breath freshening spray, fluoride varnish, denture cleaner, toothbrush, interdental brush, dental floss, oral swab, pet dental gel, pet hairball paste, and pet dental chew.

**12.** The composition according to claim 8, wherein in the composition, a content of nicotinamide adenine dinucleotide is not less than 100 μg/mL; or,

a content of 2-hydroxy-3-phenylpropionic acid is not less than 100 μg/mL; or,
a content of the peptide according to claim 6 or 7 is not less than 100 μg/mL.

**13.** The composition according to any one of claims 8-12, wherein the composition further comprises one or more of an excipient, a diluent, a carrier, and an additive.

**14.** Use of nicotinamide adenine dinucleotide in freshening breath and/or alleviating and inhibiting halitosis.

**15.** The use according to claim 14, wherein the nicotinamide adenine dinucleotide is derived from postbiotic of *Lactobacillus paracasei* ET-22.

**16.** Use of nicotinamide adenine dinucleotide in reducing a content of volatile sulfur compound in breath and volatile organic compound in saliva within an oral cavity.

**17.** Use of nicotinamide adenine dinucleotide in reducing a content of characteristic flavor compound.

**18.** Use of 2-hydroxy-3-phenylpropionic acid in freshening breath and/or alleviating and inhibiting halitosis.

**19.** The use according to claim 18, wherein the 2-hydroxy-3-phenylpropionic acid is derived from postbiotic of *Lactobacillus paracasei* ET-22.

**20.** Use of 2-hydroxy-3-phenylpropionic acid in reducing a content of characteristic flavor compound in an oral cavity.

**21.** Use of 2-hydroxy-3-phenylpropionic acid in reducing amount and thickness of biofilm in an oral cavity.

**22.** Use of the peptide according to claim 6 or 7 in freshening breath and/or alleviating and inhibiting halitosis.

**23.** Use of the peptide according to claim 6 or 7 in reducing a content of volatile organic compound in an oral cavity and/or inhibiting growth of microorganism in an oral cavity.

**24.** Use of peptidoglycan in freshening breath and/or alleviating and inhibiting halitosis, wherein the peptidoglycan is derived from heat-inactivated bacterial cells of *Lactobacillus paracasei* ET-22.

**25.** The use according to claim 24, wherein an extraction method of the peptidoglycan comprises the following steps:

step 1: performing liquid culture of *Lactobacillus paracasei* ET-22, stopping fermentation upon reaching a logarithmic growth phase, and collecting bacterial cells;
step 2: resuspending the collected bacterial cells in sterile water, performing heat inactivation, and collecting to obtain the heat-inactivated bacterial cells;
step 3: resuspending the heat-inactivated bacterial cells in PBS buffer, performing ultrasonic disruption of the heat-inactivated bacterial cells, centrifuging to collect a pellet, and extracting the peptidoglycan from the pellet.

**26.** The use according to claim 25, wherein in step 2, the collected bacterial cells are resuspended in sterile water to prepare a bacterial suspension with a viable bacterial concentration of $1.0\times10^9$ CFU/ml.

**27.** The use according to claim 25, wherein in step 2, a heat inactivation temperature is 90-150°C and a heat inactivation time is 5-15 min.

**28.** The use according to claim 25, wherein in step 3, extracting the peptidoglycan from the pellet specifically comprises: resuspending the heat-inactivated bacterial cell pellet in PBS buffer, performing ultrasonic disruption, then centrifuging to obtain a pellet; boiling the obtained pellet in 4% SDS solution for 30 minutes, then adding 10% TCA to remove teichoic acid and proteins from the heat-inactivated bacterial cells; then adding a mixed solvent comprising sodium acetate, chloroform, and methanol for degreasing; then treating with a composite enzyme solution of alkaline protease and trypsin at 37°C for 12 hours, then boiling in 1% SDS solution for 10 minutes, and washing to remove SDS to a trace level; then performing dialysis for 2 days with water changed every 8 hours, collecting sediment, and freeze-drying the sediment under vacuum to obtain peptidoglycan.

**29.** *A Lactobacillus paracasei* ET-22 bacteriocin, comprising exocytotic substance of *Lactobacillus paracasei* ET-22 and metabolites of *Lactobacillus paracasei* ET-22, wherein a mass ratio of citric acid to L-methionine in the bacteriocin is greater than 15:1, and a deposit number of *Lactobacillus paracasei* ET-22 is CGMCC No. 15077.

**30.** The *Lactobacillus paracasei* ET-22 bacteriocin according to claim 29, wherein the mass ratio of citric acid to L-methionine in the bacteriocin is 15-100:1;
preferably, the mass ratio of citric acid to L-methionine in the bacteriocin is 35-45: 1.

**31.** A preparation method for the *Lactobacillus paracasei* ET-22 bacteriocin according to claim 29 or 30, comprising following steps:

bacteriocin extraction, wherein bacterial sludge separated from *Lactobacillus paracasei* ET-22 fermentation broth is extracted with a solvent to obtain a mixed solution;
sterilization, wherein a supernatant is separated from the mixed solution and subjected to heat sterilization to obtain the bacteriocin.

**32.** The preparation method for the *Lactobacillus paracasei* ET-22 bacteriocin according to claim 31, wherein extraction temperature is 0-37°C; preferably, the extraction temperature is 20-30°C; and/or, extraction time is 0.5-3 h; preferably, the extraction time is 30-120 min.

**33.** The preparation method for the *Lactobacillus paracasei* ET-22 bacteriocin according to claim 31, wherein a total colony count of *Lactobacillus paracasei* ET-22 in the mixed solution is $0.5\times10^{11}$-$5\times10^{11}$ CFU/ml; preferably, the total colony count of *Lactobacillus paracasei* ET-22 in the mixed solution is $2\times10^{11}$-$4\times10^{11}$ CFU/ml.

**34.** The preparation method for the *Lactobacillus paracasei* ET-22 bacteriocin according to claim 31, wherein heat sterilization temperature is 70-121°C; preferably, the heat sterilization temperature is 70-100°C.

**35.** The preparation method for the *Lactobacillus paracasei* ET-22 bacteriocin according to claim 31, wherein heat sterilization time is 5-30 min; preferably, the heat sterilization time is 10-16 min.

**36.** The preparation method for the *Lactobacillus paracasei* ET-22 bacteriocin according to claim 31, wherein the method

further comprises a fermentation step: performing liquid culture of *Lactobacillus paracasei* ET-22, and stopping fermentation upon reaching a logarithmic growth phase;

preferably, the fermentation step comprises: inoculating a fermentation seed solution of *Lactobacillus paracasei* ET-22 into a culture medium, and fermenting at a temperature of 30-40°C, a rotational speed of 60-80 rpm, and a pH of 5.5-6.0 for 12-14 h to obtain a *Lactobacillus paracasei* ET-22 fermentation broth;
preferably, the preparation of the fermentation seed solution of *Lactobacillus paracasei* ET-22 comprises: subjecting *Lactobacillus paracasei* ET-22 strain to activation, purification, primary seed solution culture, secondary seed solution expansion, tertiary seed solution culture, and fermentation seed preparation, and obtaining the fermentation seed solution of *Lactobacillus paracasei* ET-22;
preferably, the culture medium is MRS liquid medium;
preferably, the solvent is water; preferably, the solvent is sterile water;
preferably, after the sterilization step, the bacteriocin is freeze-dried to obtain a freeze-dried bacteriocin.

**37.** Use of the *Lactobacillus paracasei* ET-22 bacteriocin according to claim 29 or 30 in preparing food, wherein the food comprises at least one of dairy product and beverage.

**38.** Use of the *Lactobacillus paracasei* ET-22 bacteriocin according to claim 29 or 30 in preparing a composition, wherein the composition comprises an antioxidant composition; preferably, the composition is selected from at least one of pharmaceutical, health food, and feed.

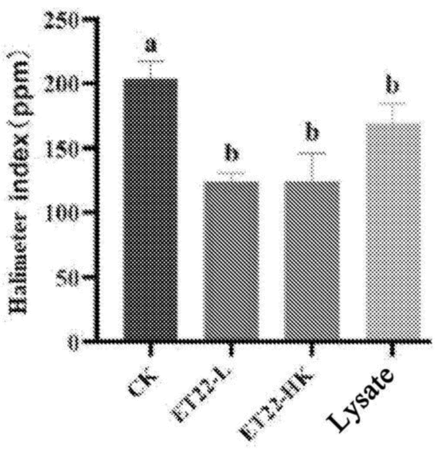


FIG. 1

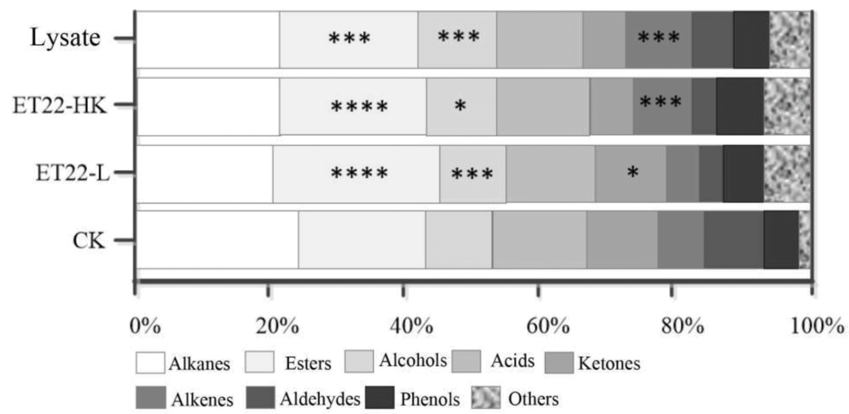


FIG. 2

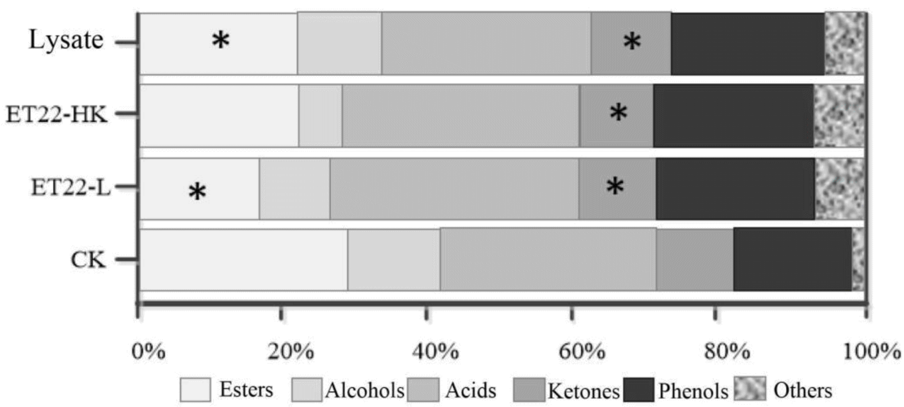


FIG. 3

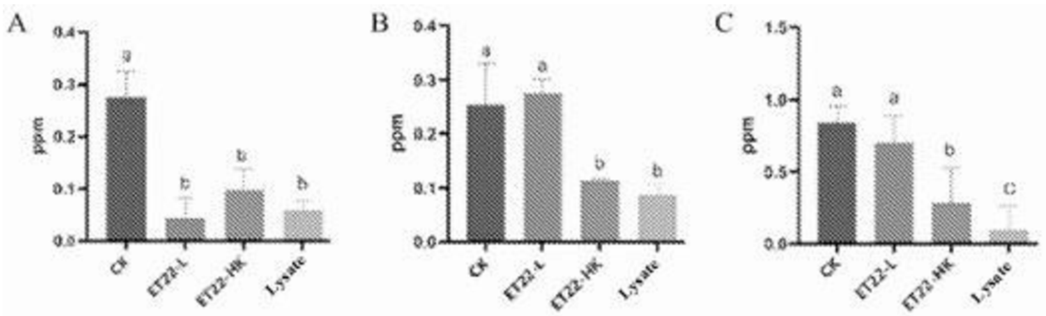


FIG. 4

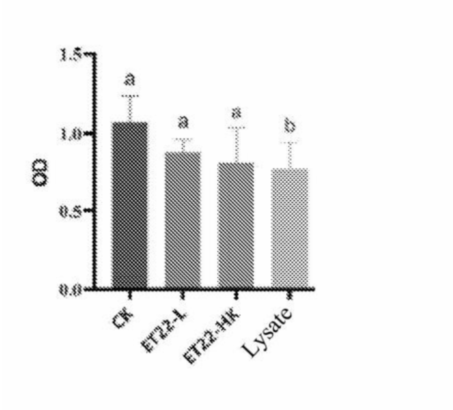


FIG. 5

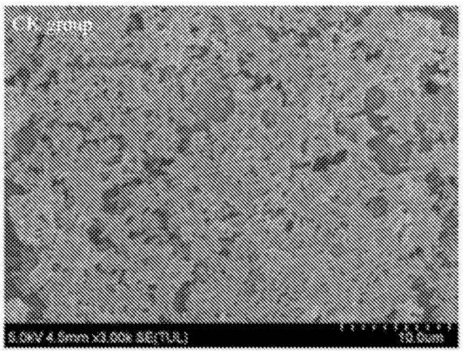


FIG. 6a

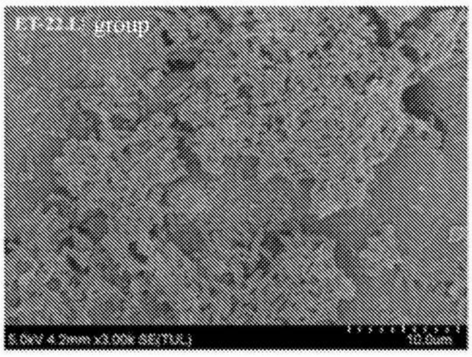

FIG. 6b

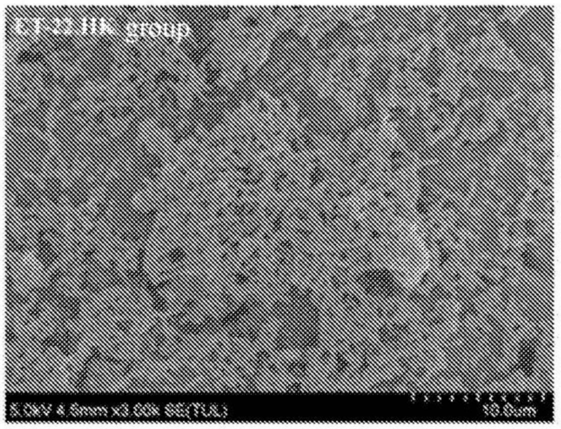

FIG. 6c

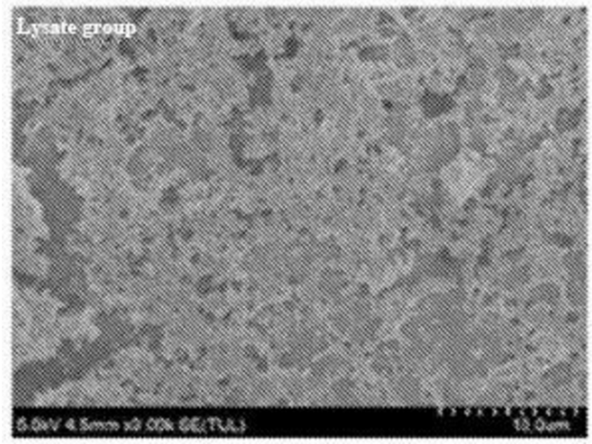


FIG. 6d

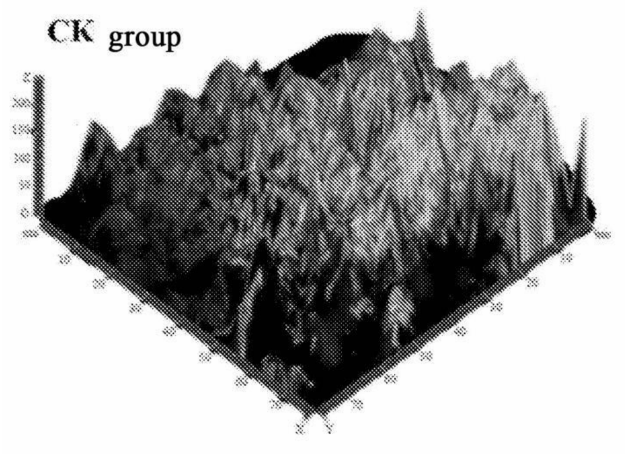


FIG. 7a

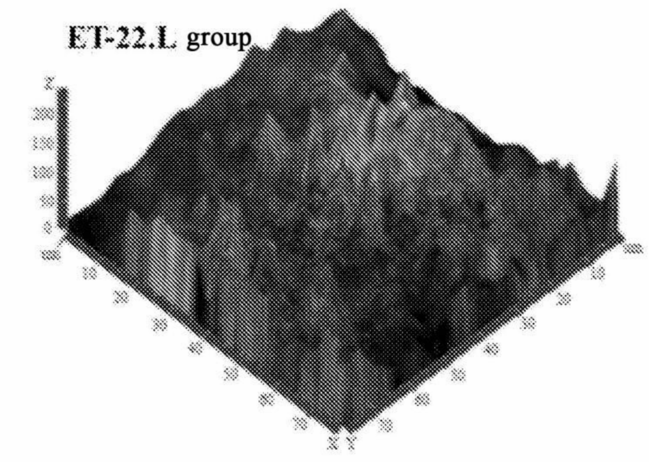


FIG. 7b

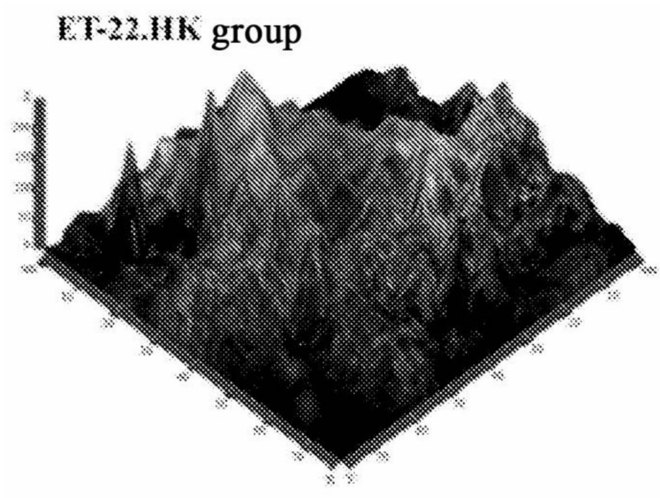


FIG. 7c

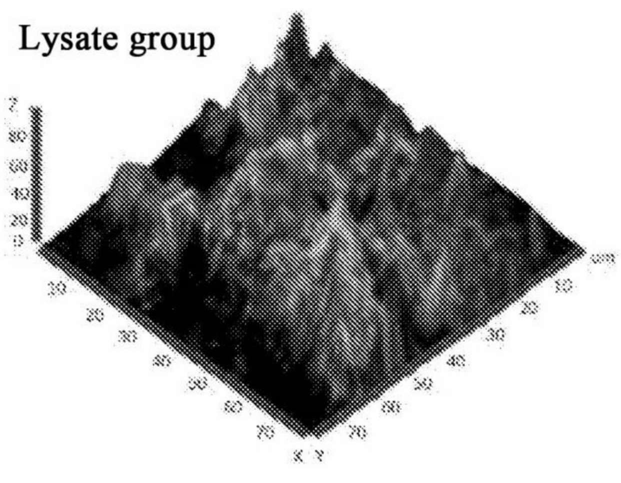


FIG. 7d

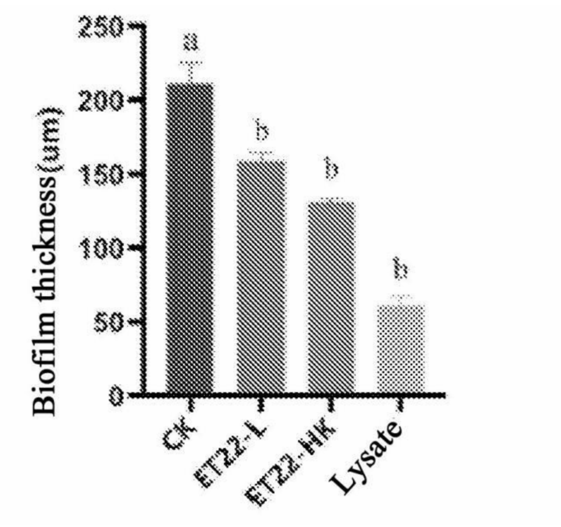
Biofilm thickness(um)
250
200
150
100
50
0
a
b
b
b
CK
ET22-L
ET22-HK
Lysate

FIG. 8

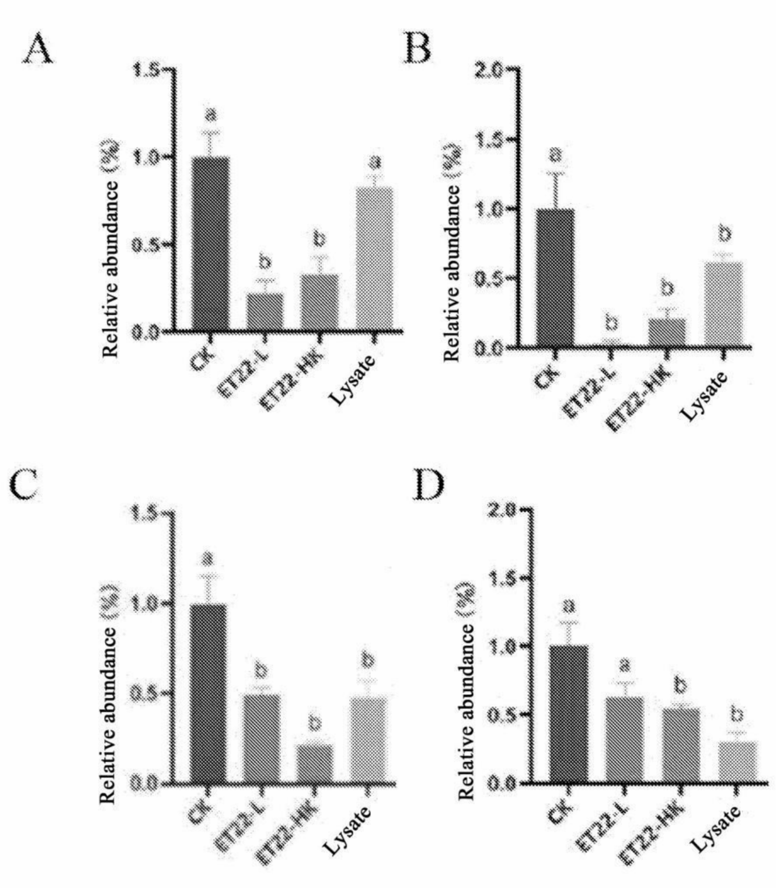
A
B
C
D
Relative abundance (%)
CK
ET22-L
ET22-HK
Lysate

FIG. 9

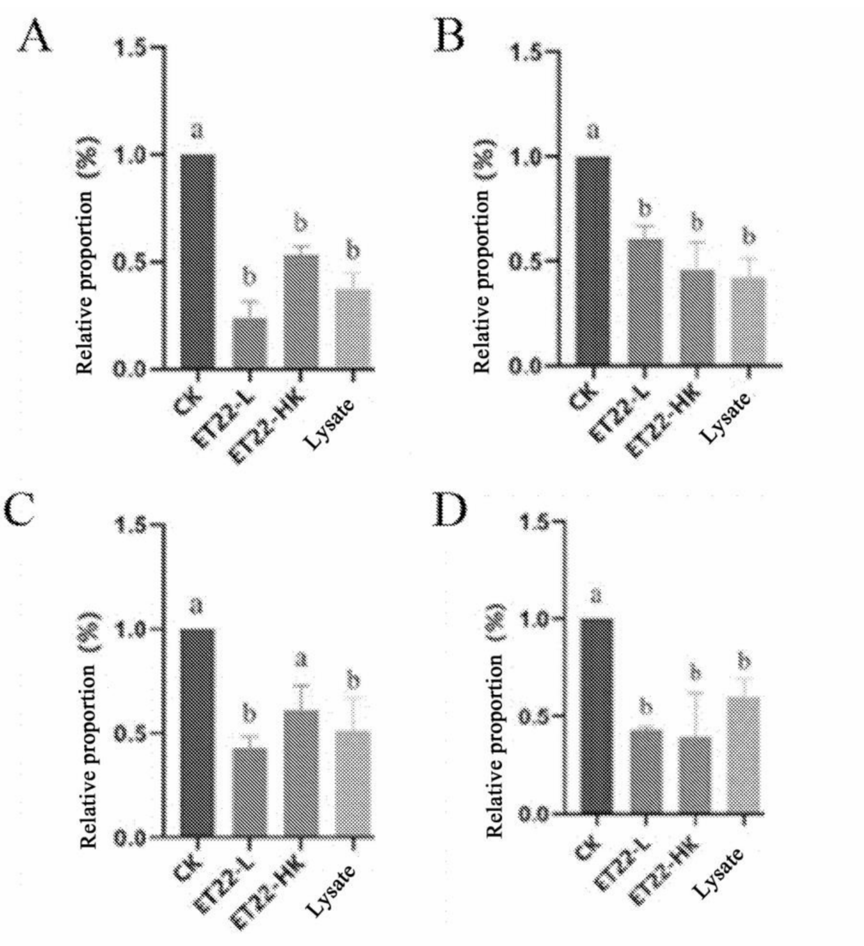
A
B
C
D
Relative proportion (%)
1.5
1.0
0.5
0.0
a
b
CK
ET22-L
ET22-HK
Lysate

FIG. 10

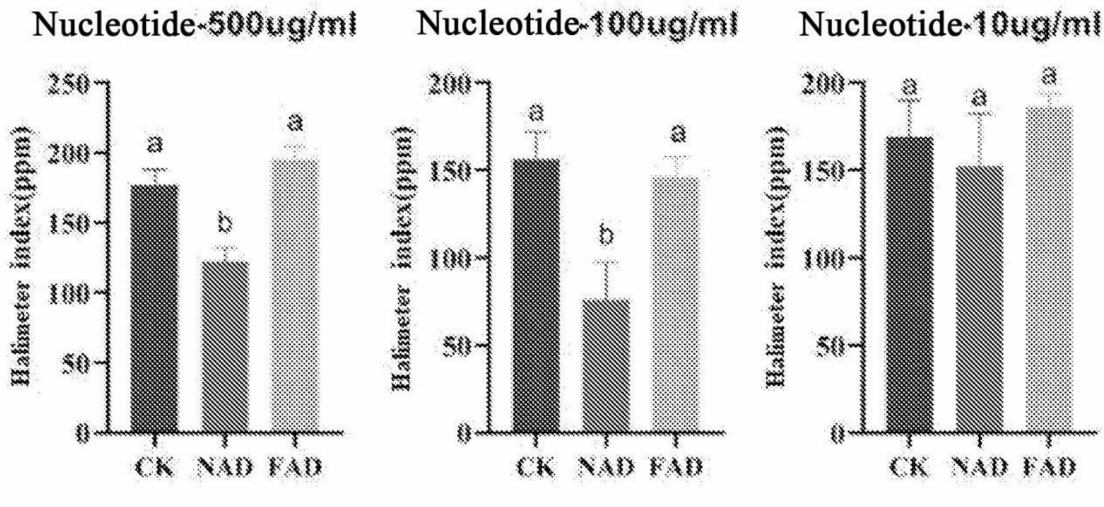
Nucleotide-500ug/ml
Nucleotide-100ug/ml
Nucleotide-10ug/ml
Halimeter index(ppm)
250
200
150
100
50
0
a
b
CK
NAD
FAD

FIG. 11

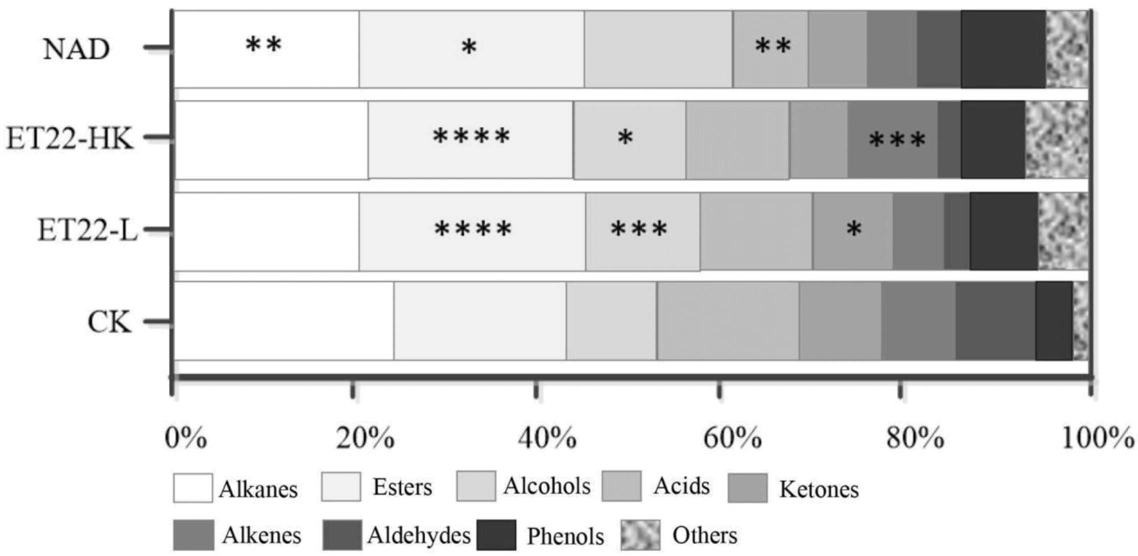


FIG. 12

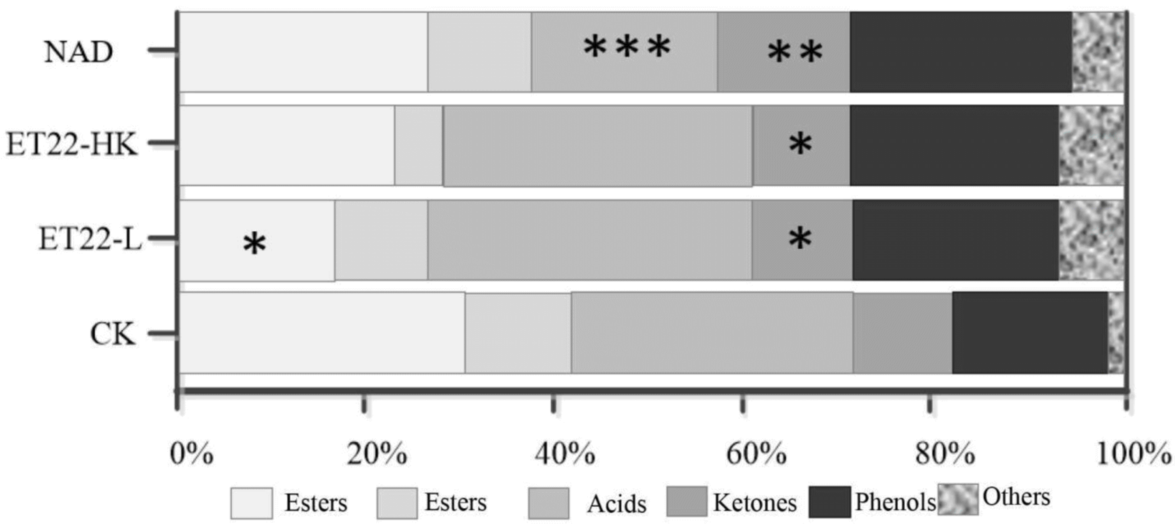


FIG. 13

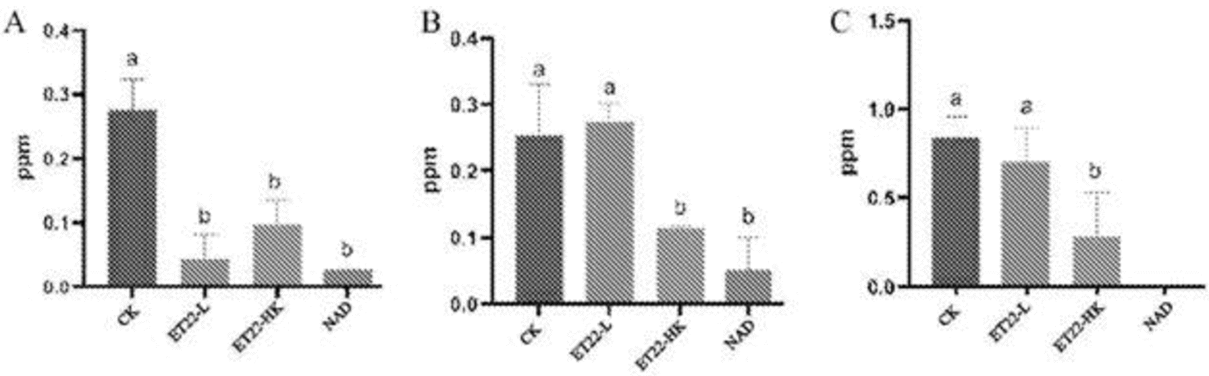


FIG. 14

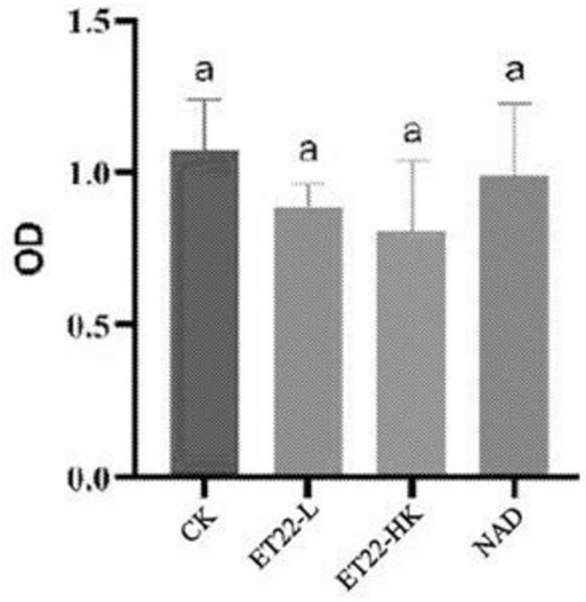


FIG. 15

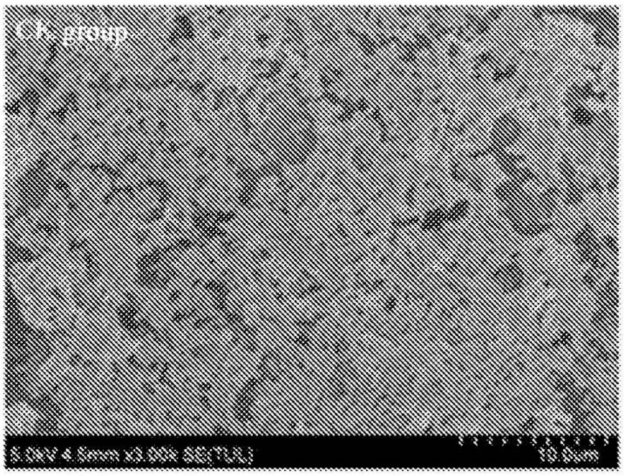

FIG. 16a

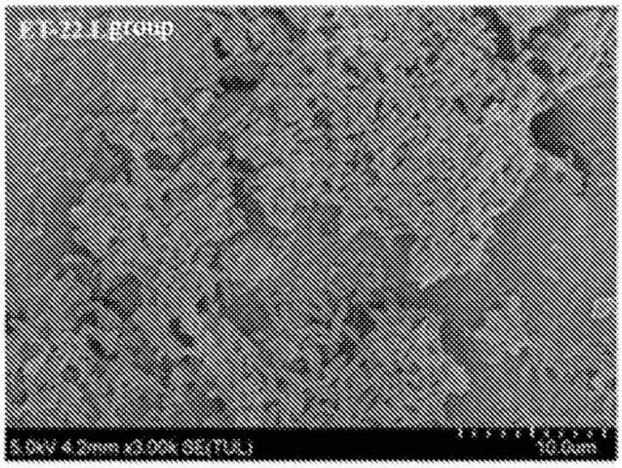

FIG. 16b

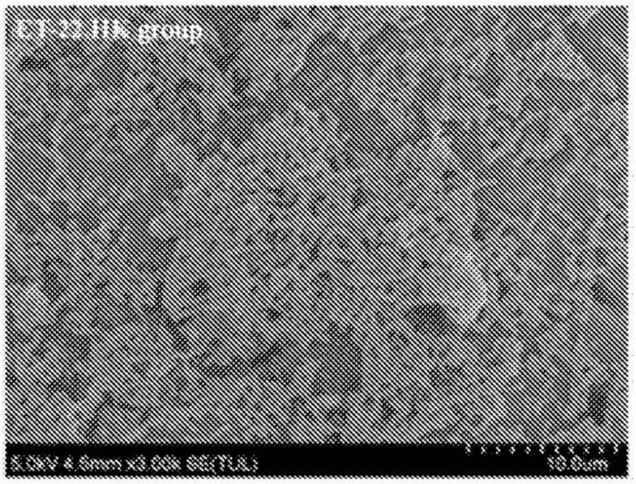

FIG. 16c

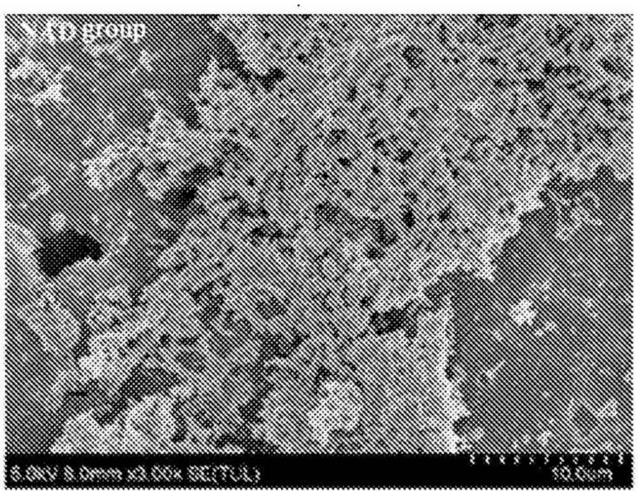

FIG. 16d

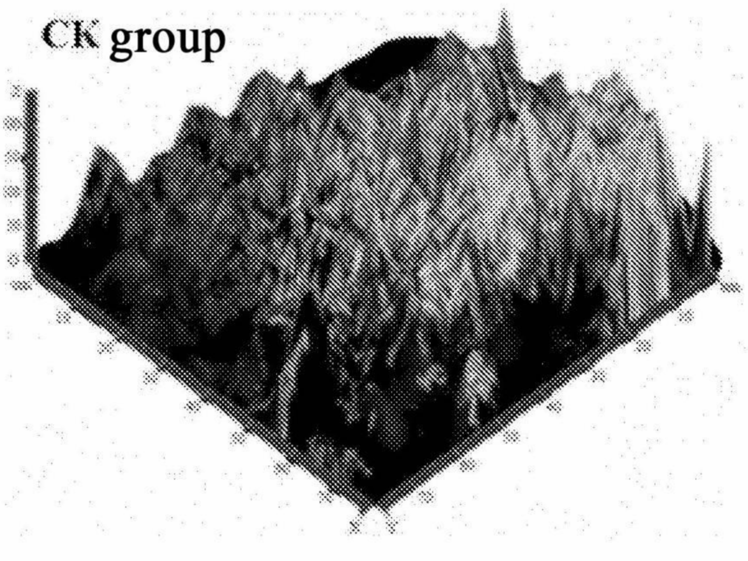


FIG. 17a

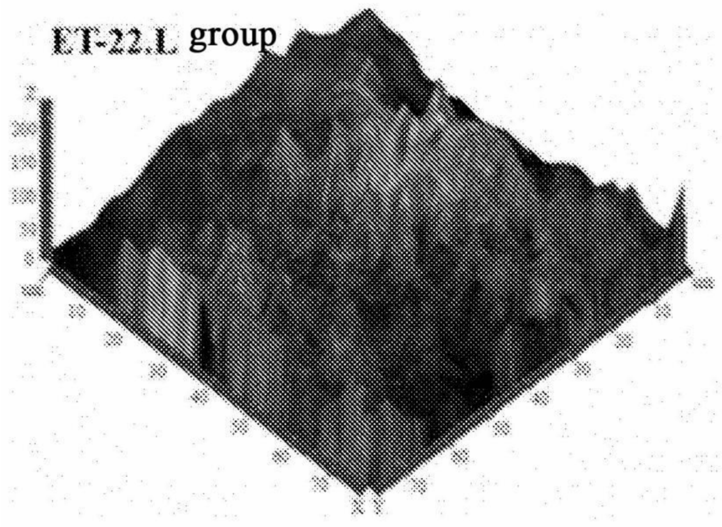


FIG. 17b

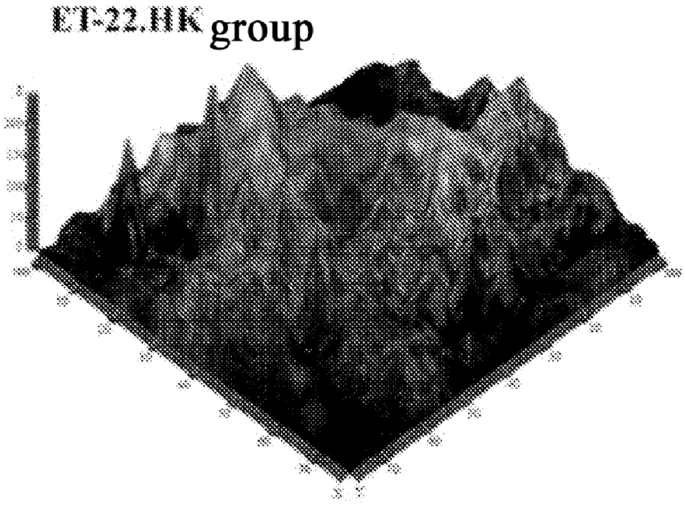


FIG. 17c

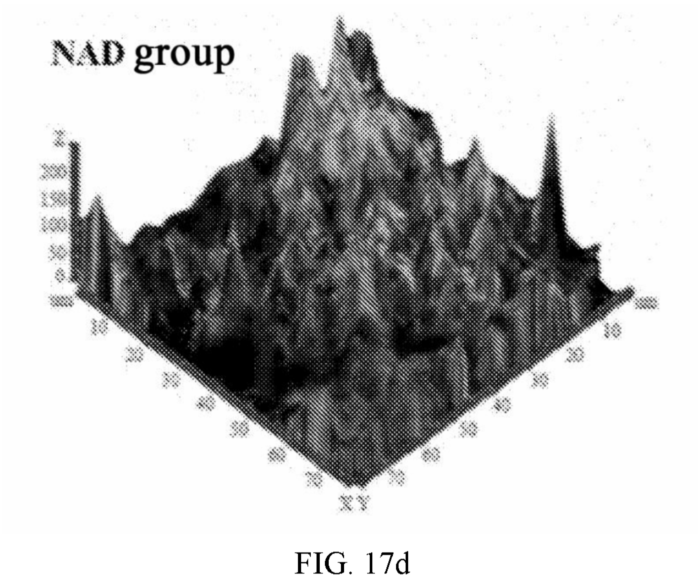


FIG. 17d

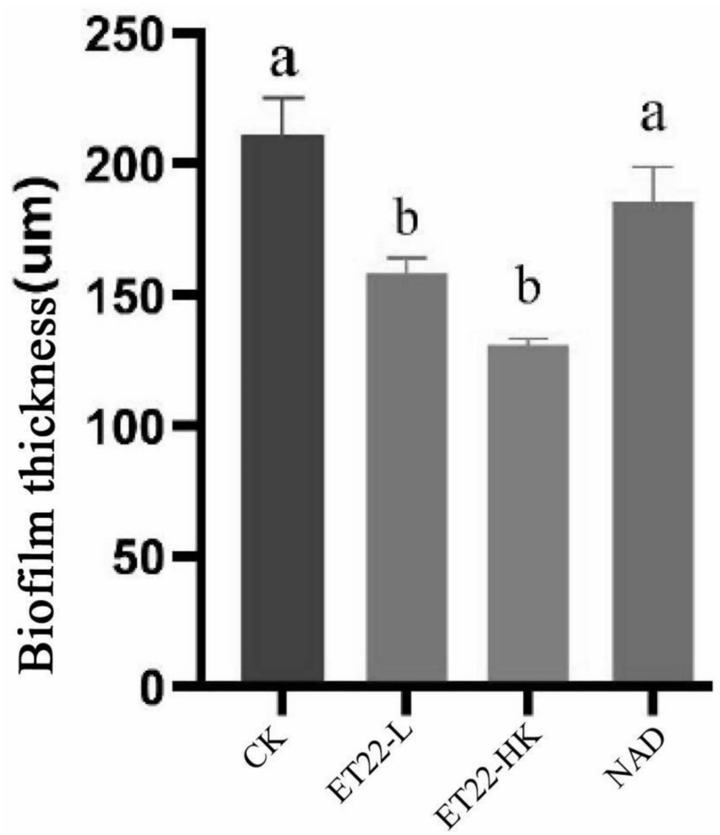
Biofilm thickness(um)
250
200
150
100
50
0
a
b
b
a
CK
ET22-L
ET22-HK
NAD

FIG. 18

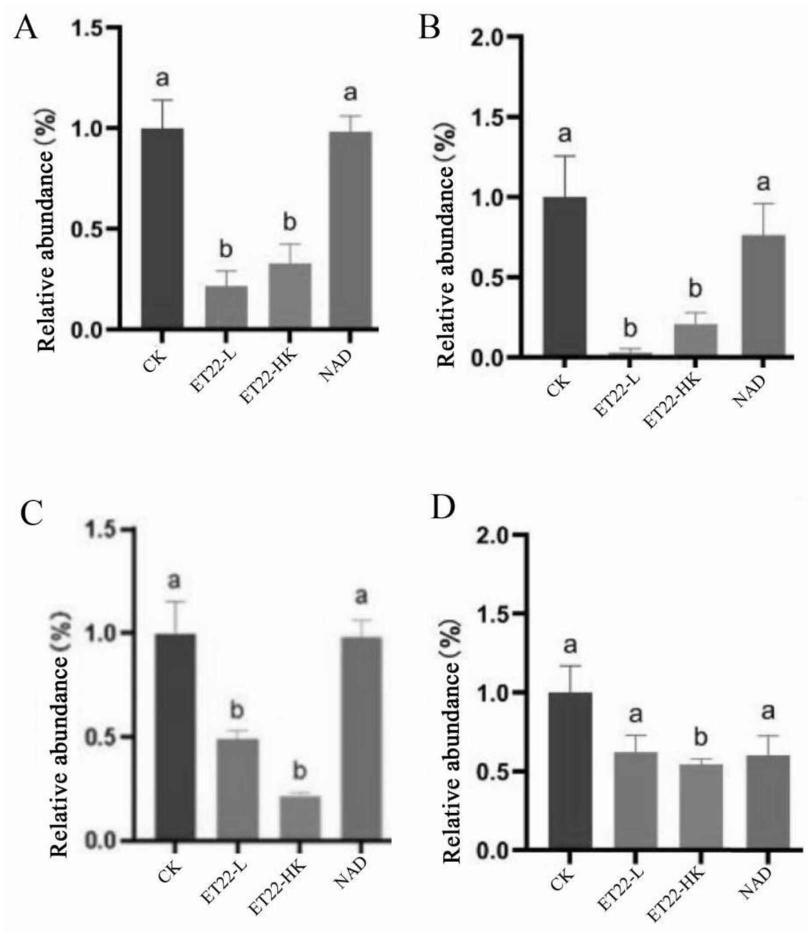


FIG. 19

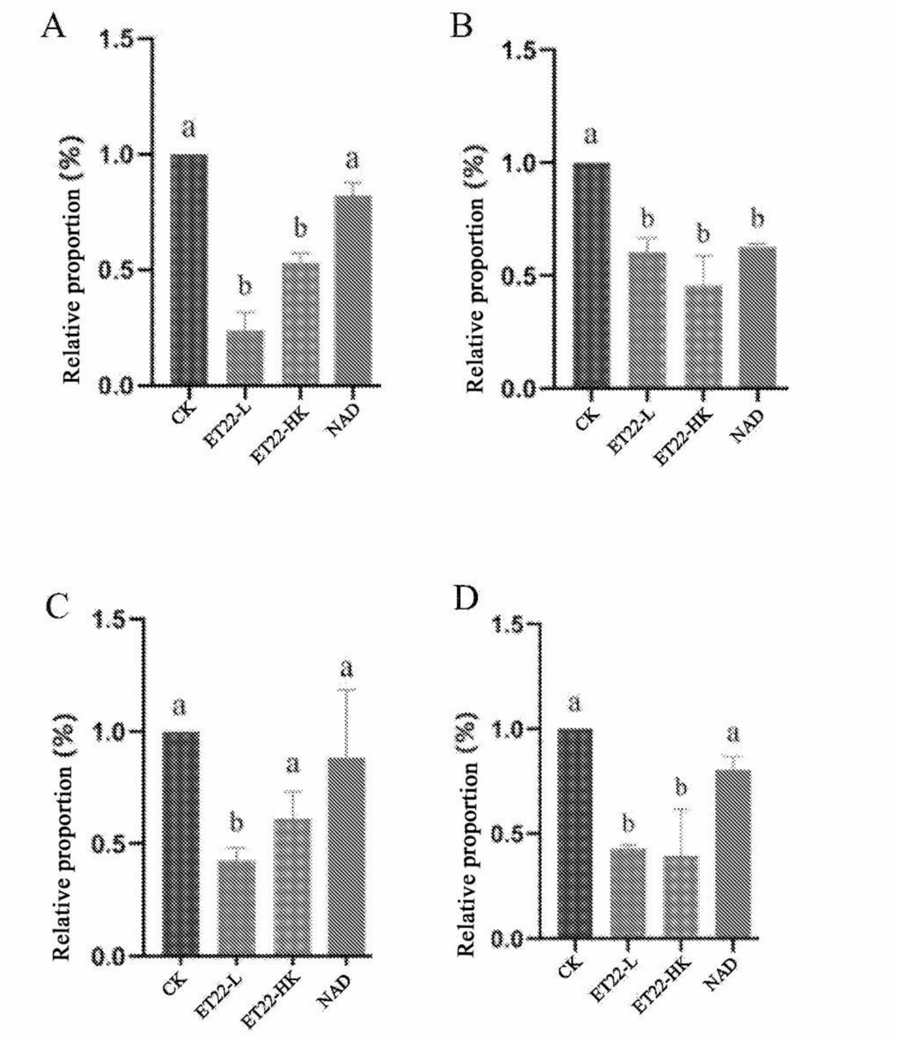
A
B
C
D
Relative proportion (%)
1.5
1.0
0.5
0.0
a
b
CK
ET22-L
ET22-HK
NAD

FIG. 20

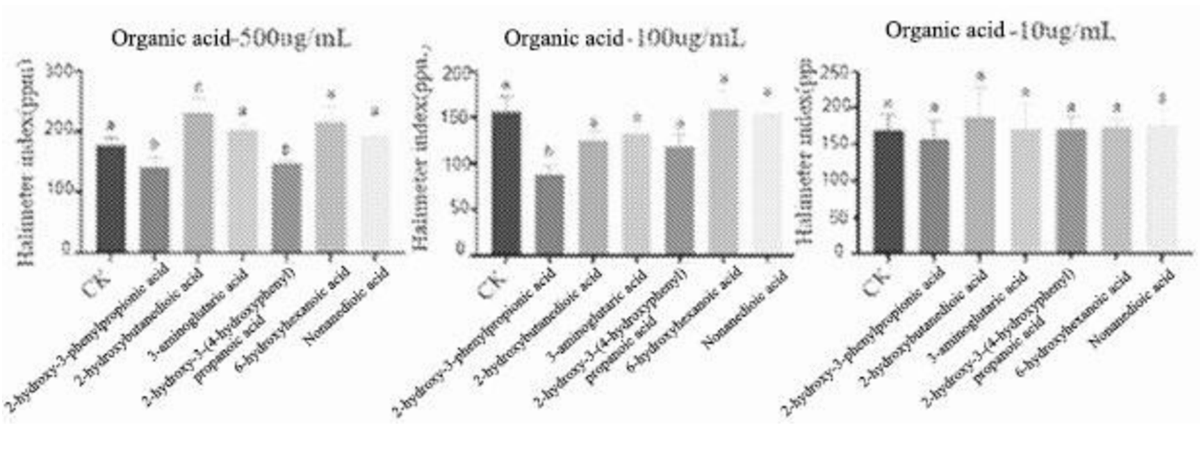
Organic acid-500ug/mL
Organic acid-100ug/mL
Organic acid-10ug/mL
Halmeter index(ppm)
CK
2-hydroxy-3-phenylpropionic acid
2-hydroxybutanedioic acid
3-aminoglutaric acid
2-hydroxy-3-(4-hydroxyphenyl) propanoic acid
6-hydroxyhexanoic acid
Nonanedioic acid

FIG. 21

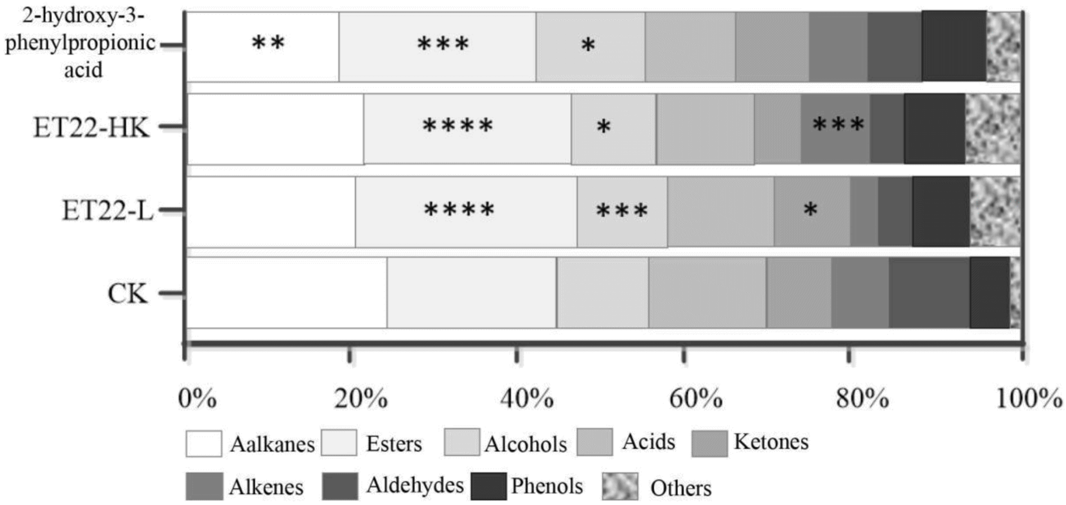


FIG. 22

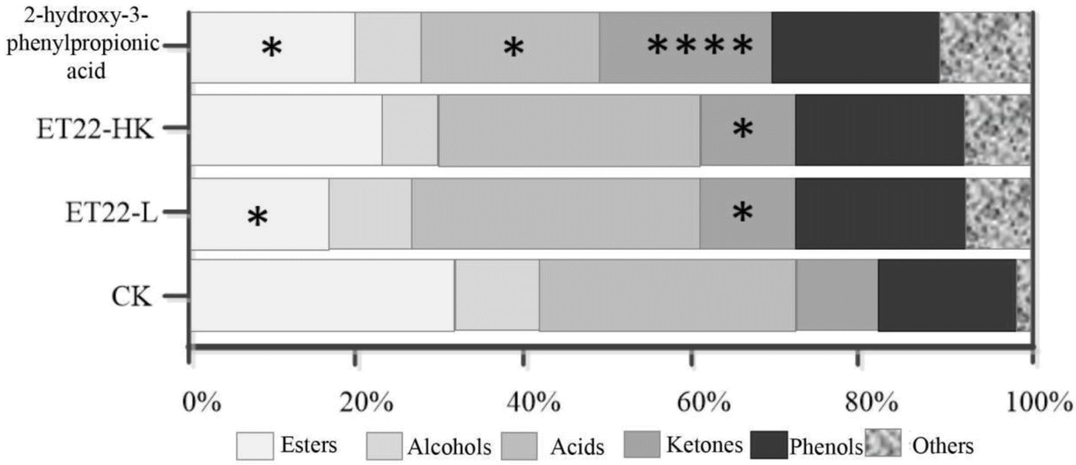


FIG. 23

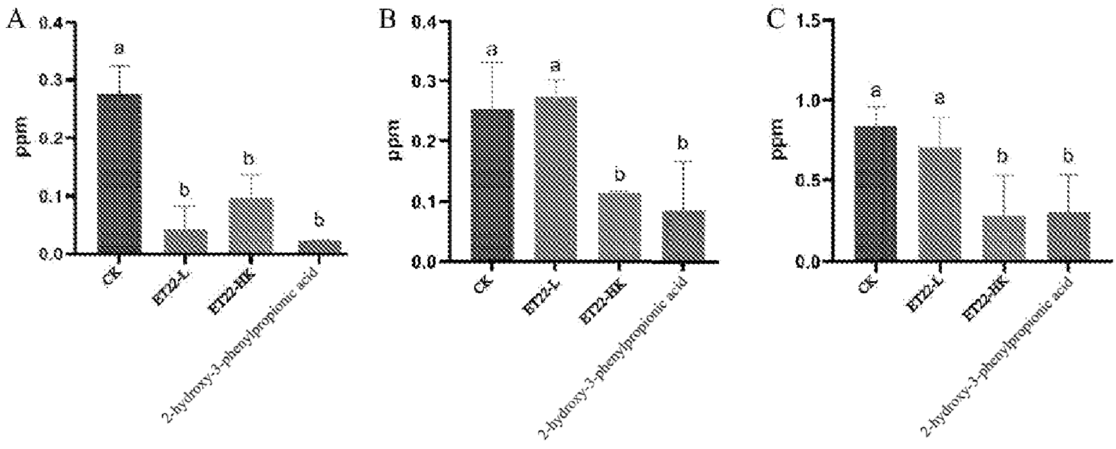


FIG. 24

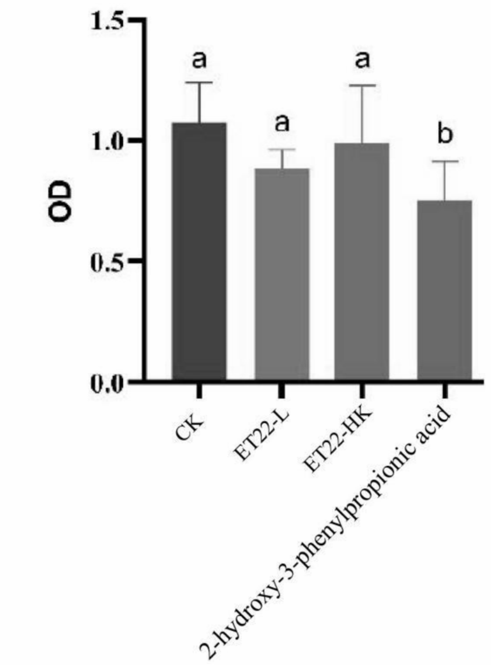


FIG. 25

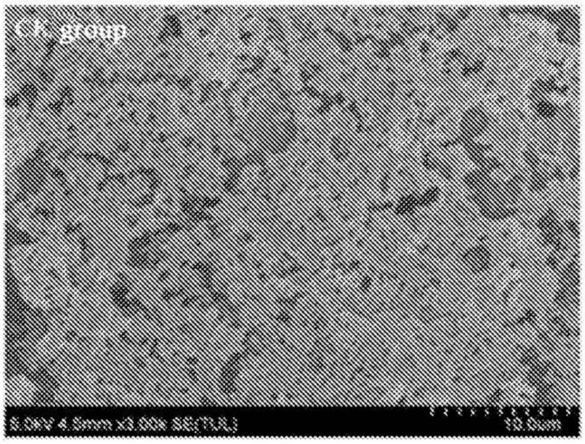


FIG. 26a

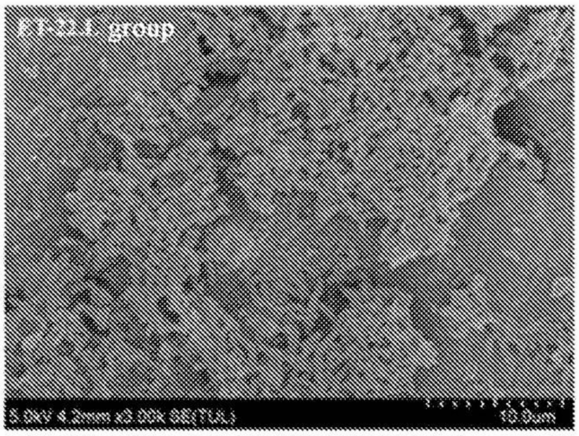


FIG. 26b

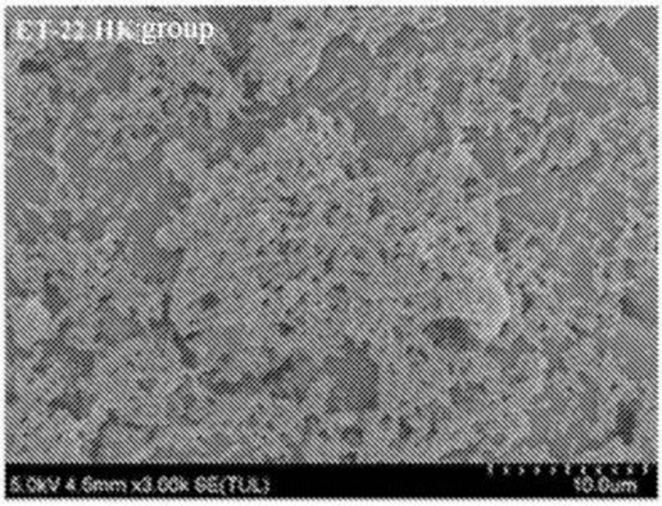


FIG. 26c

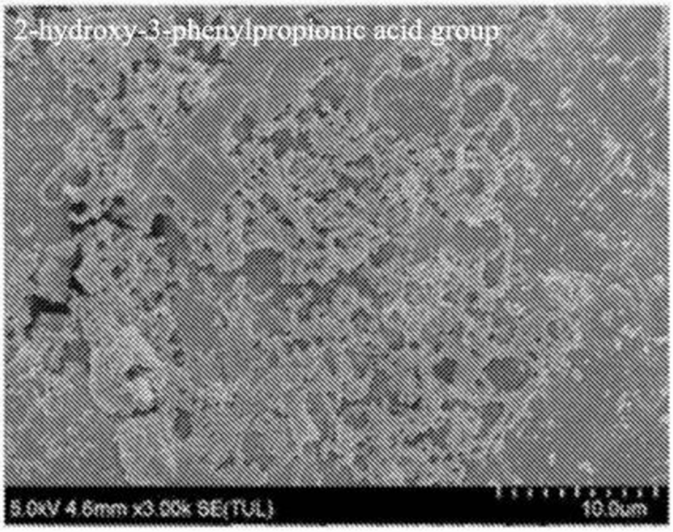


FIG. 26d

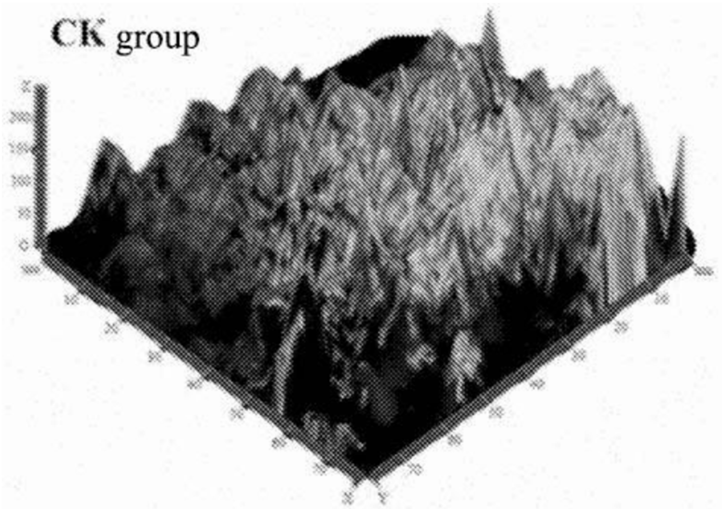


FIG. 27a

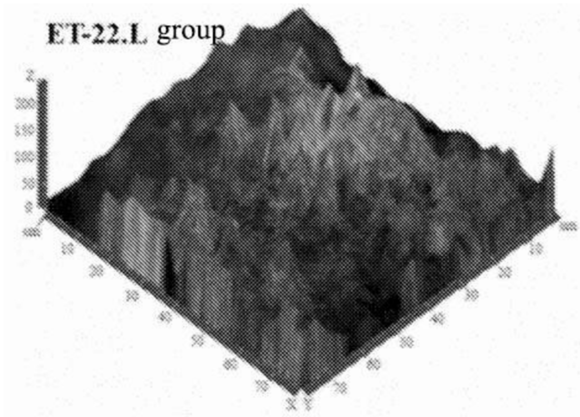


FIG. 27b

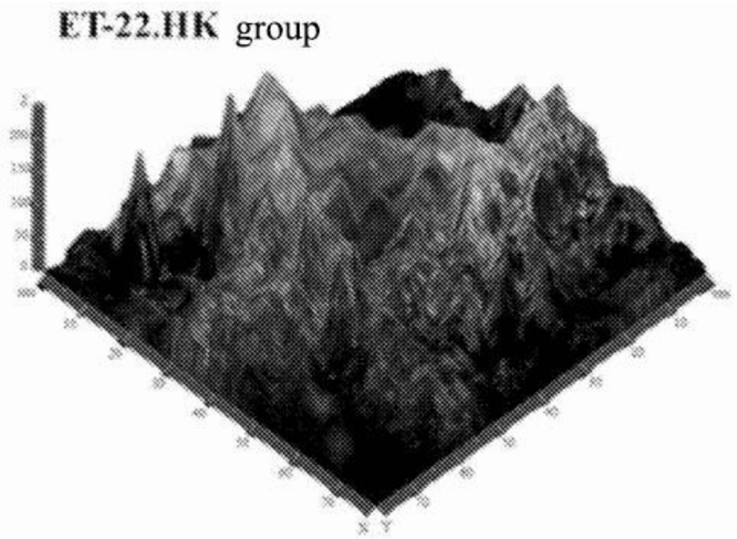


FIG. 27c

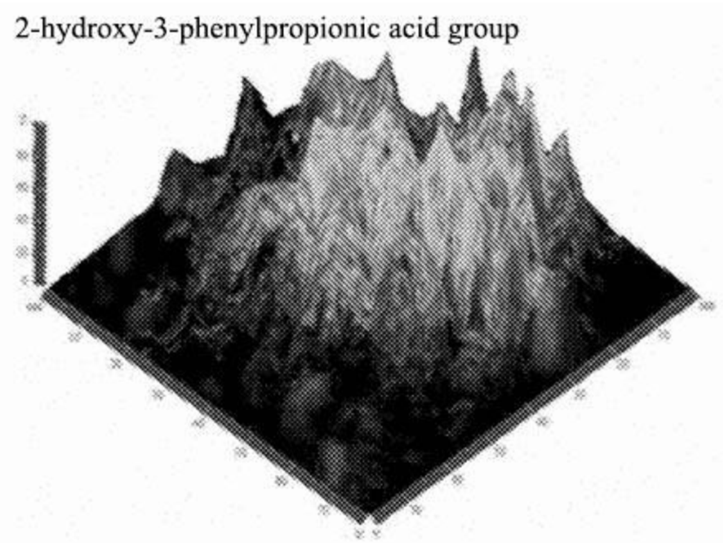


FIG. 27d

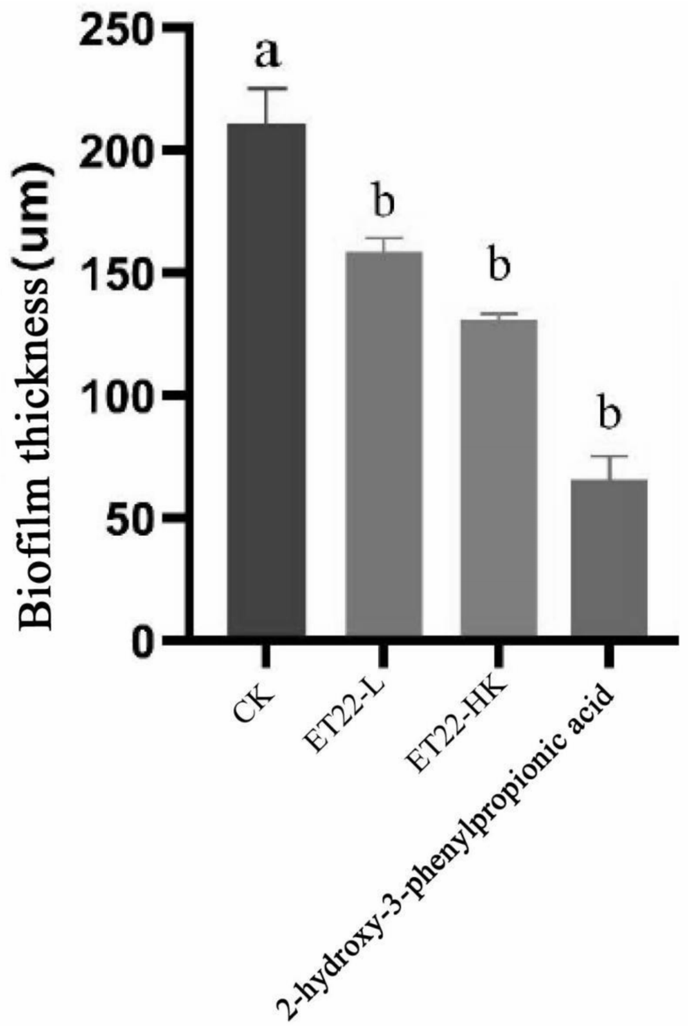
Biofilm thickness(um)
250
200
150
100
50
0
a
b
b
b
CK
ET22-L
ET22-HK
2-hydroxy-3-phenylpropionic acid

FIG. 28

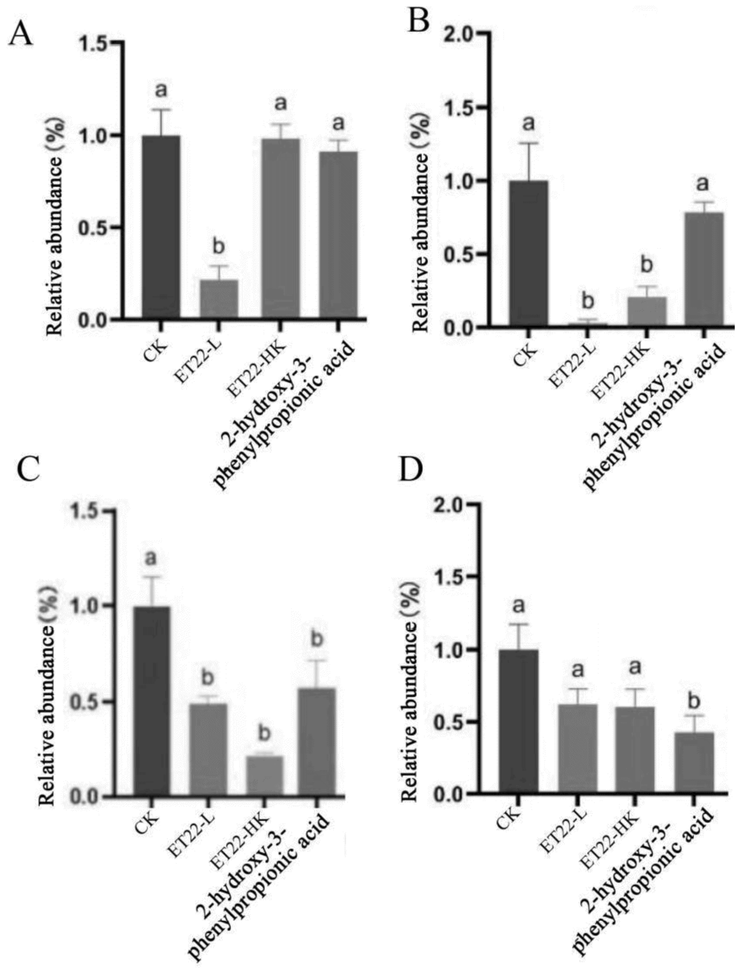
A
B
C
D
Relative abundance (%)
1.5
1.0
0.5
0.0
2.0
a
b
CK
ET22-L
ET22-HK
2-hydroxy-3-phenylpropionic acid

FIG. 29

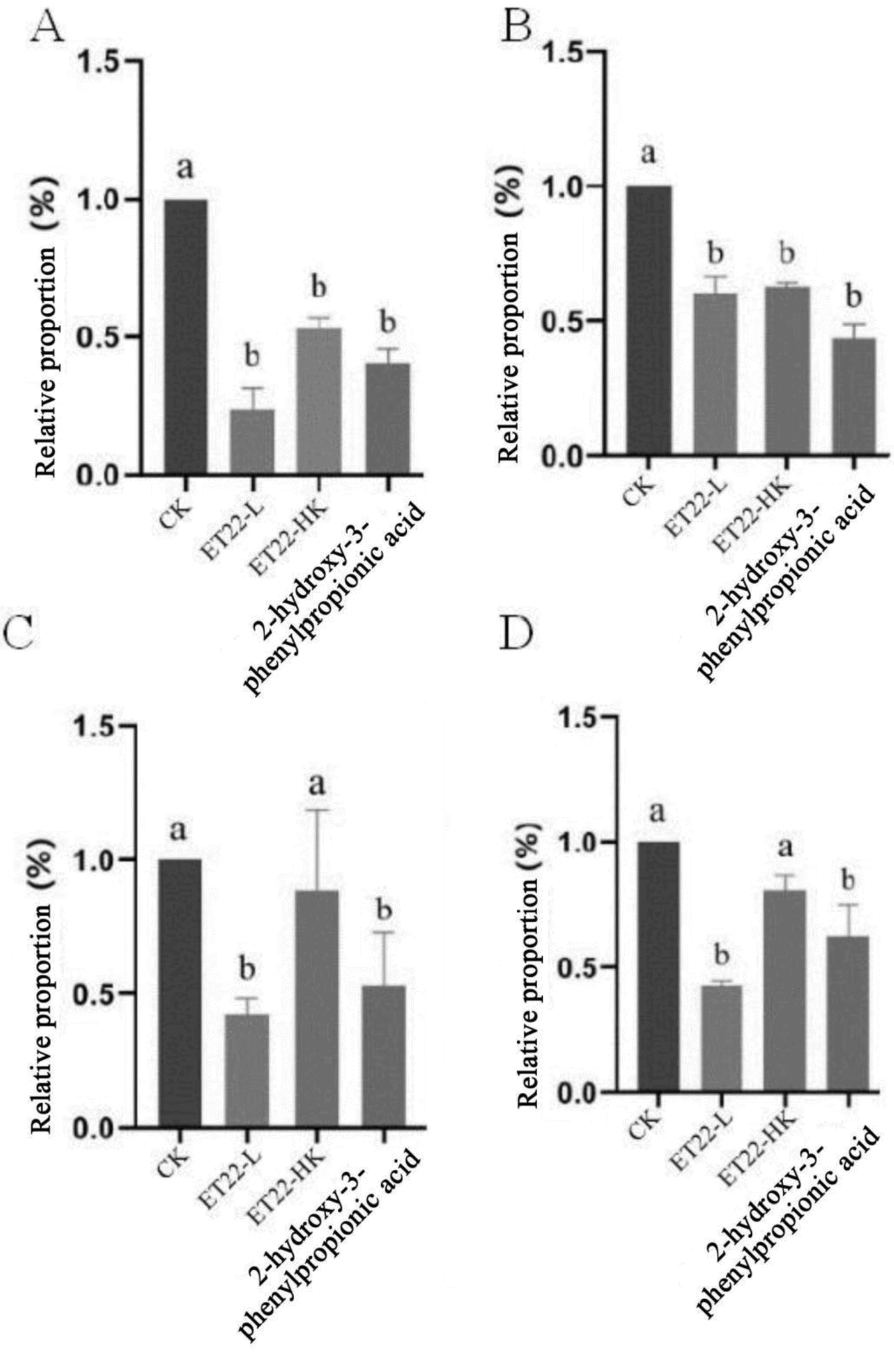
A
B
C
D
Relative proportion (%)
1.5
1.0
0.5
0.0
a
b
CK
ET22-L
ET22-HK
2-hydroxy-3-phenylpropionic acid

FIG. 30

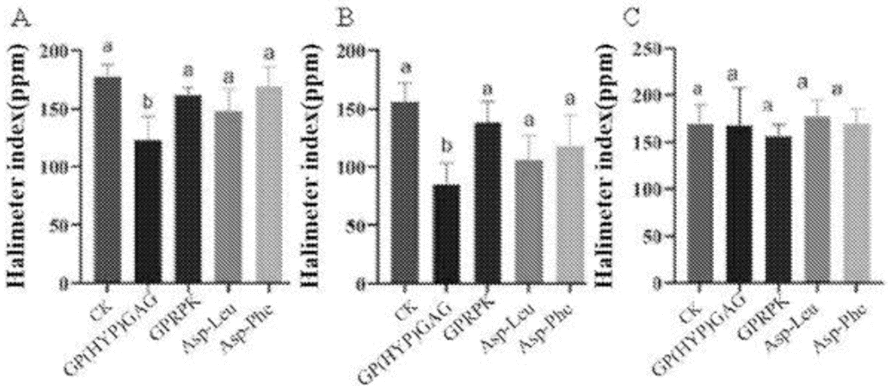


FIG. 31

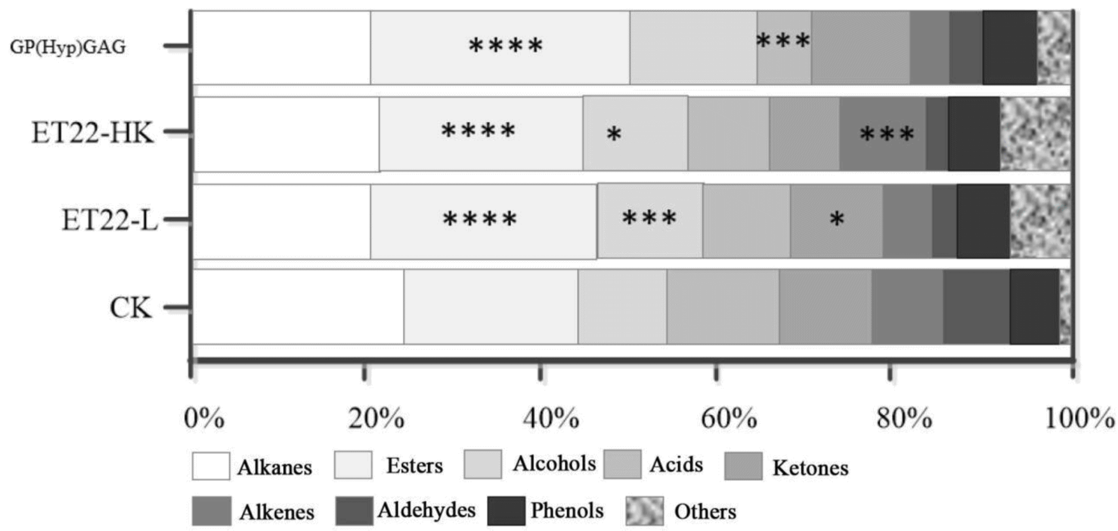


FIG. 32

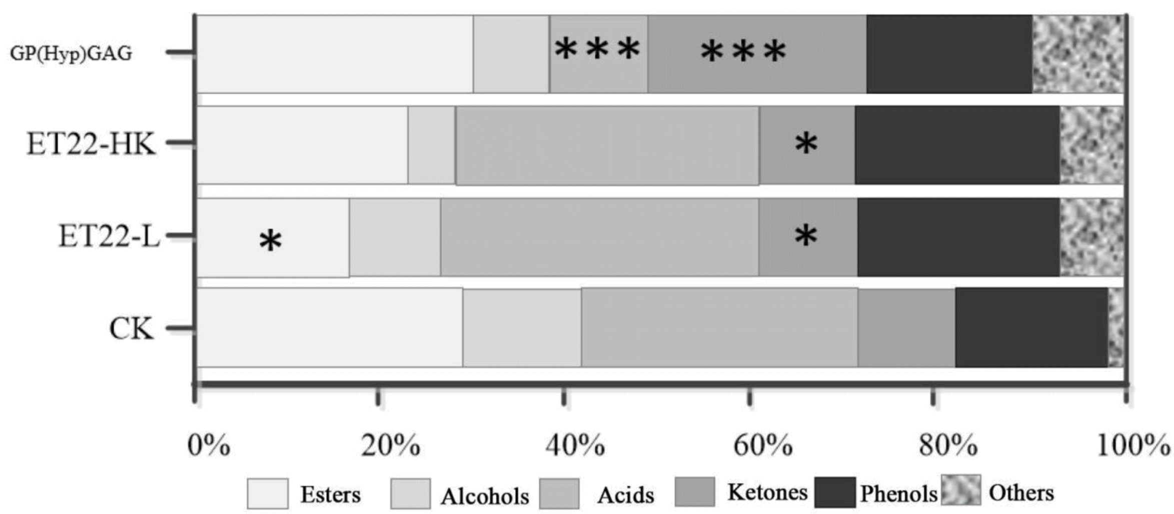


FIG. 33

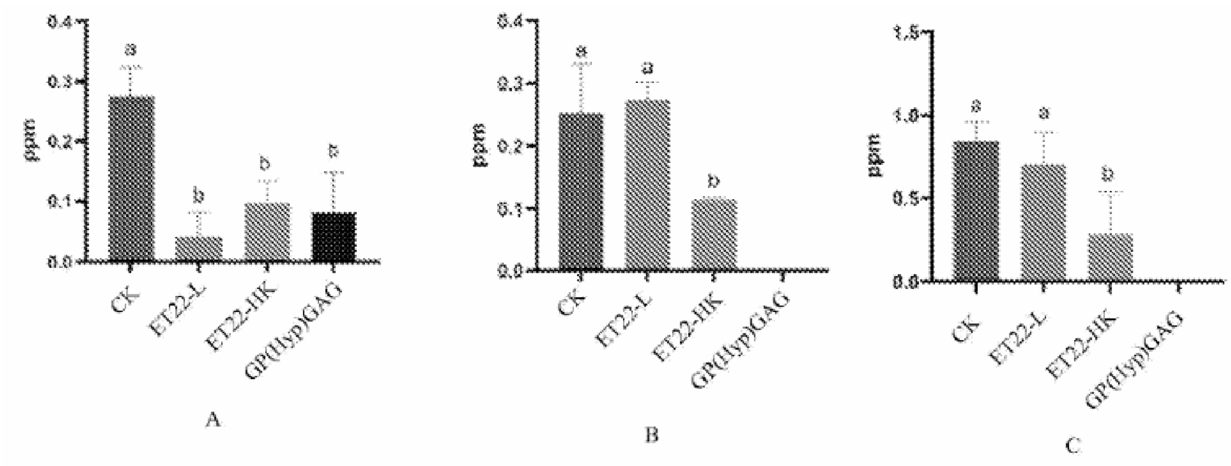


FIG. 34

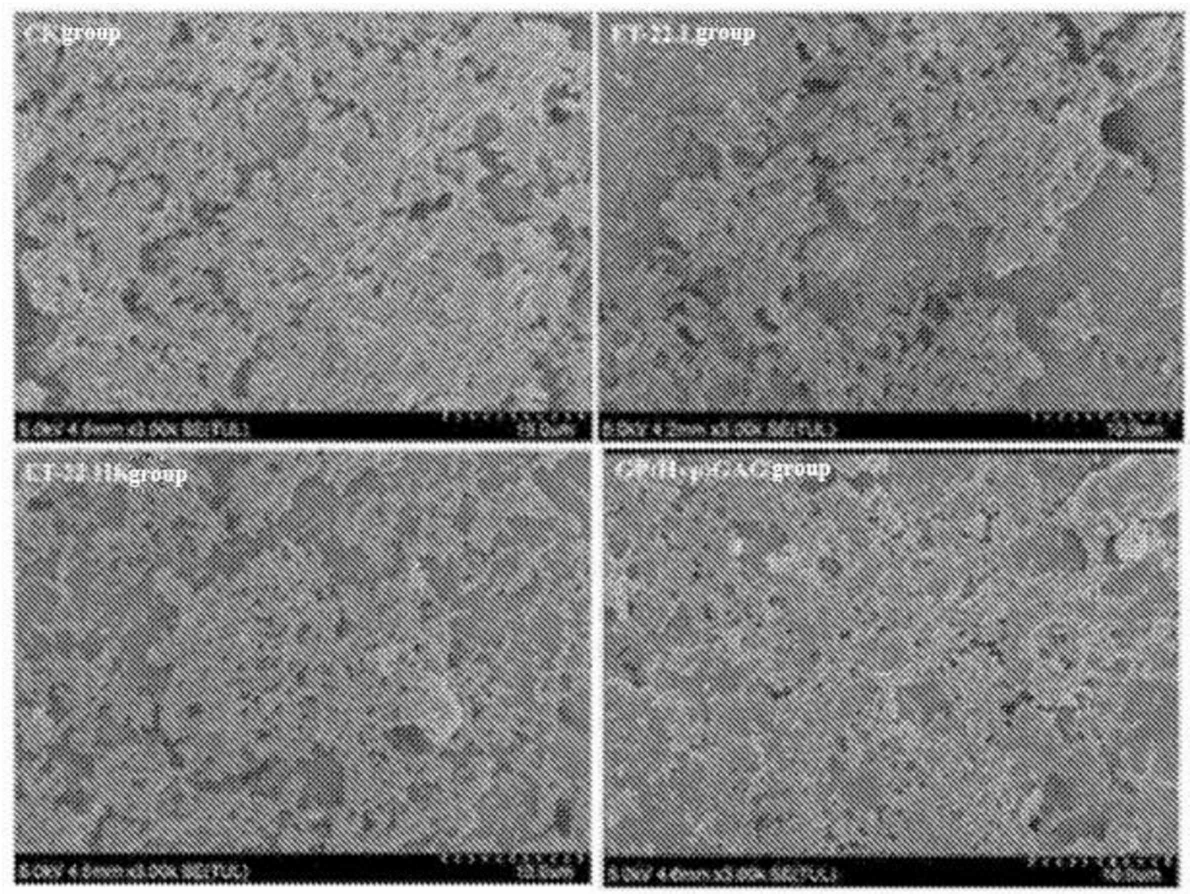

FIG. 35

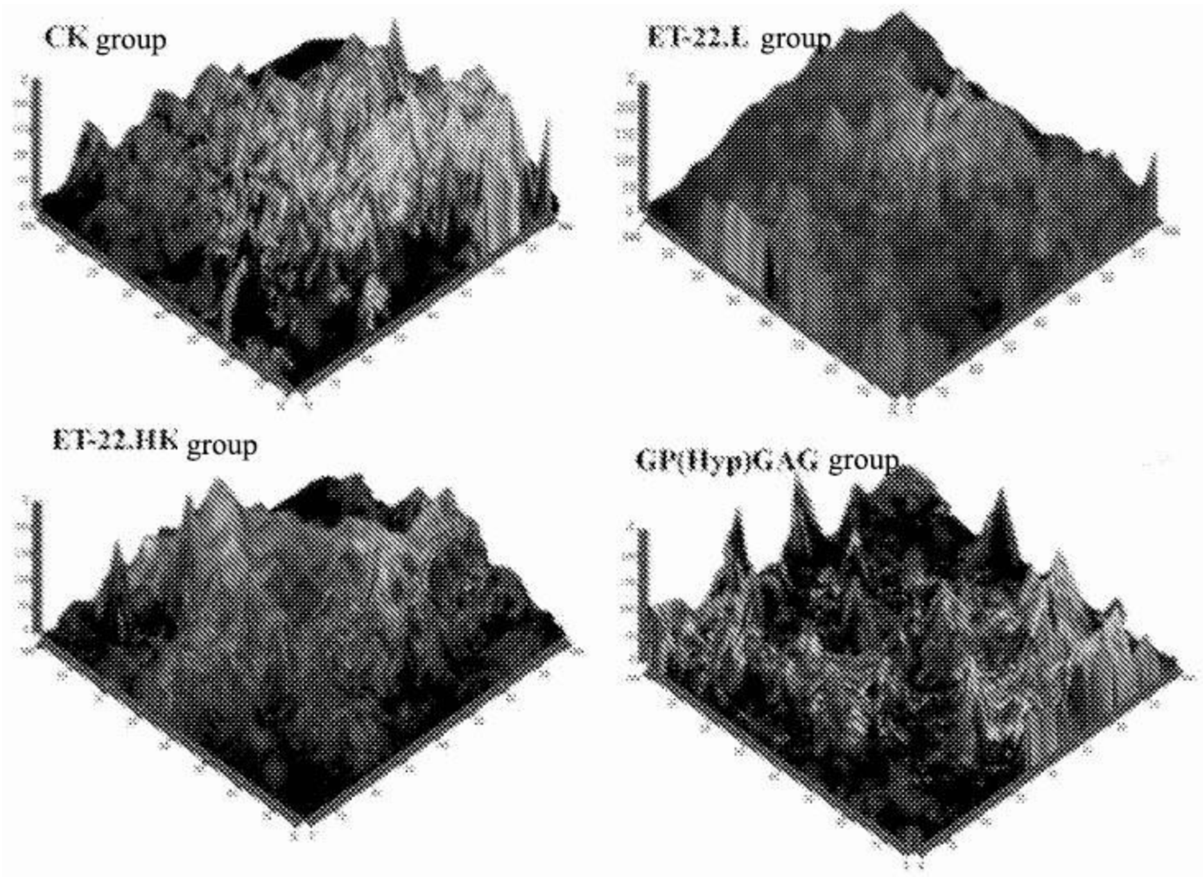
CK group
ET-22.L group
ET-22.HK group
GP(Hyp)GAG group

FIG. 36

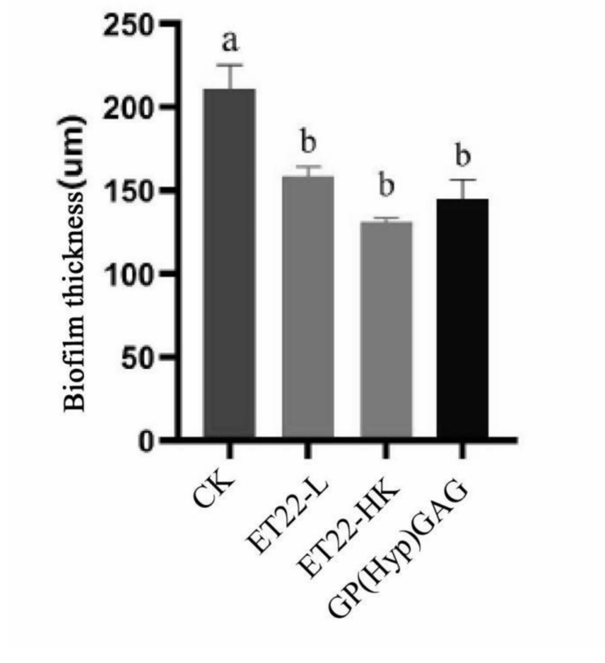
Biofilm thickness(um)
250
200
150
100
50
0
a
b
b
b
CK
ET22-L
ET22-HK
GP(Hyp)GAG

FIG. 37

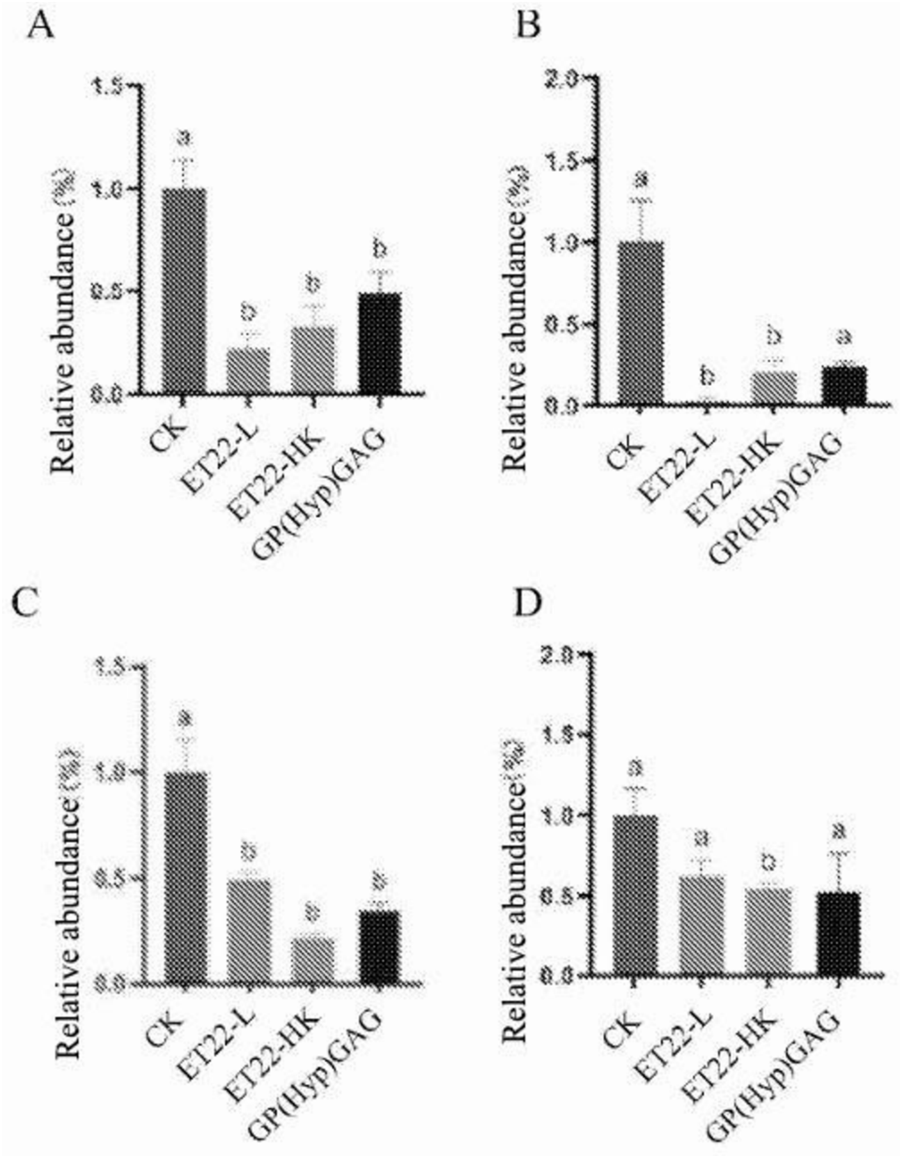


FIG. 38

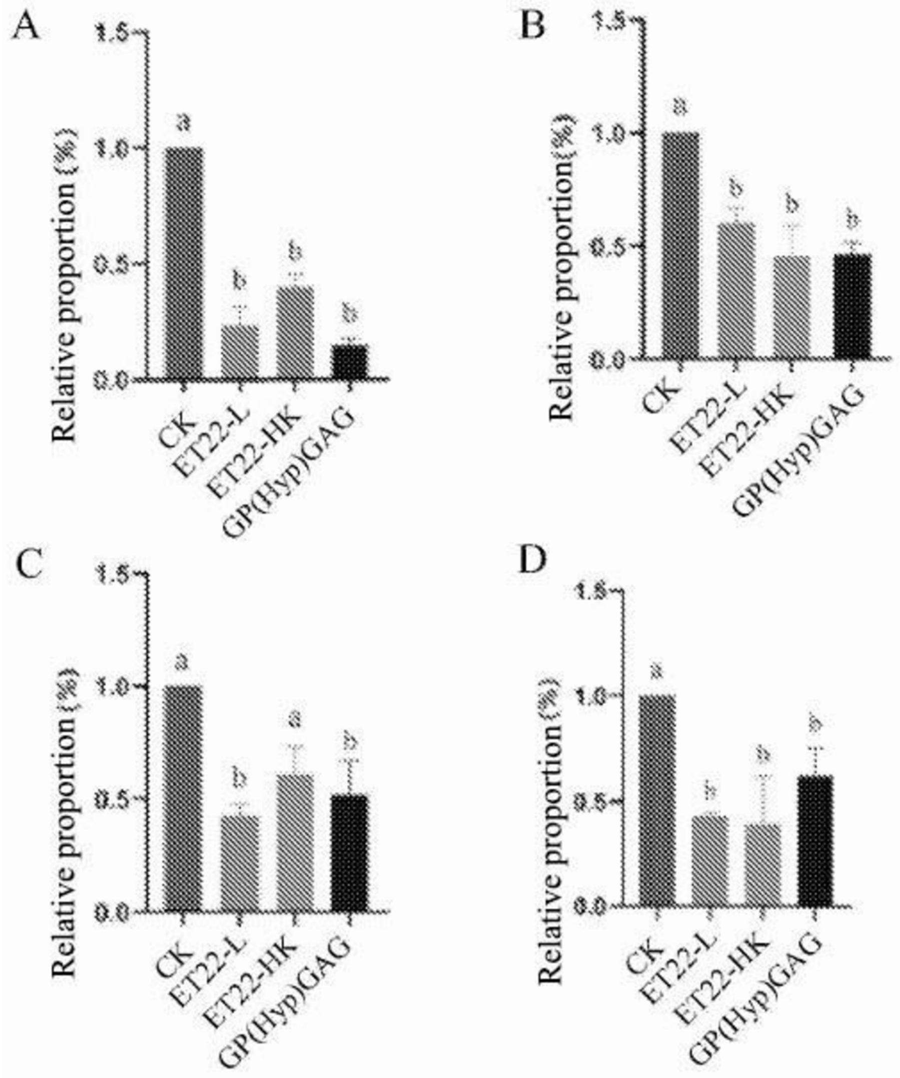
A
B
C
D
Relative proportion (%)
1.5
1.0
0.5
0.0
a
b
CK
ET22-L
ET22-HK
GP(Hyp)GAG

FIG. 39

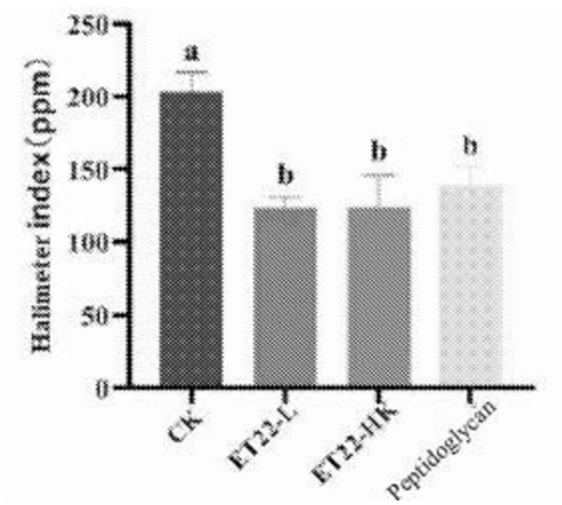
Halimeter index (ppm)
250
200
150
100
50
0
a
b
CK
ET22-L
ET22-HK
Peptidoglycan

FIG. 40

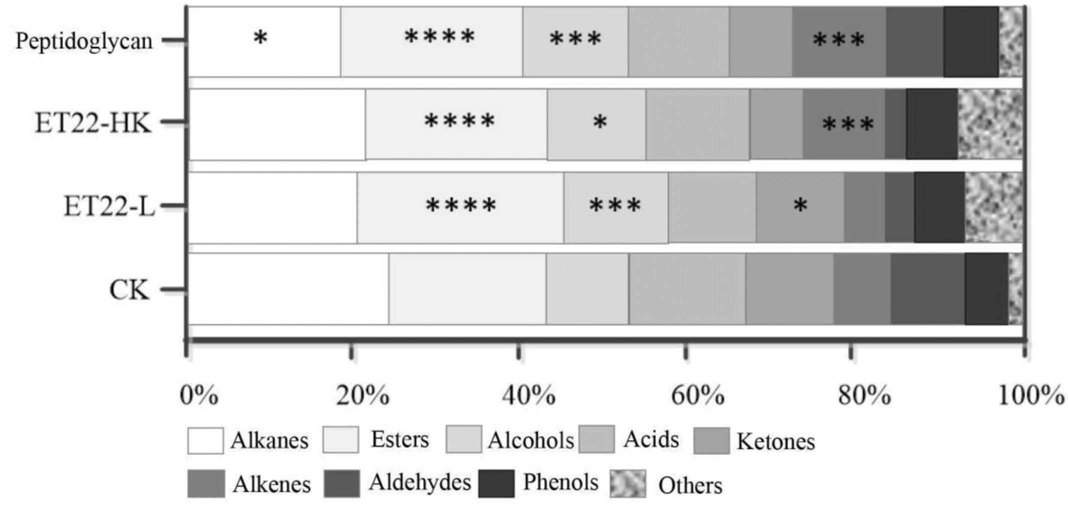


FIG. 41

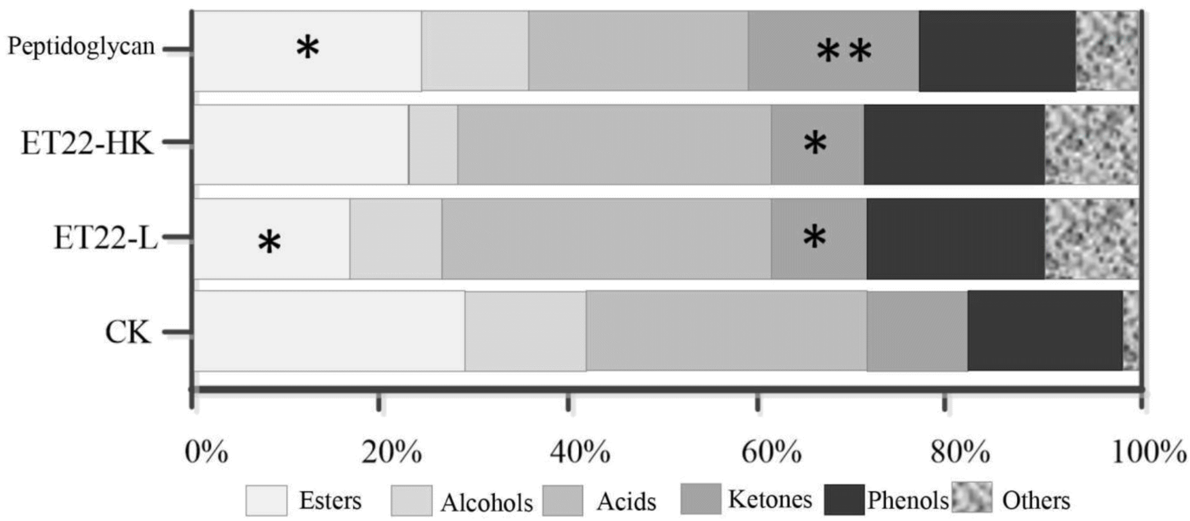


FIG. 42

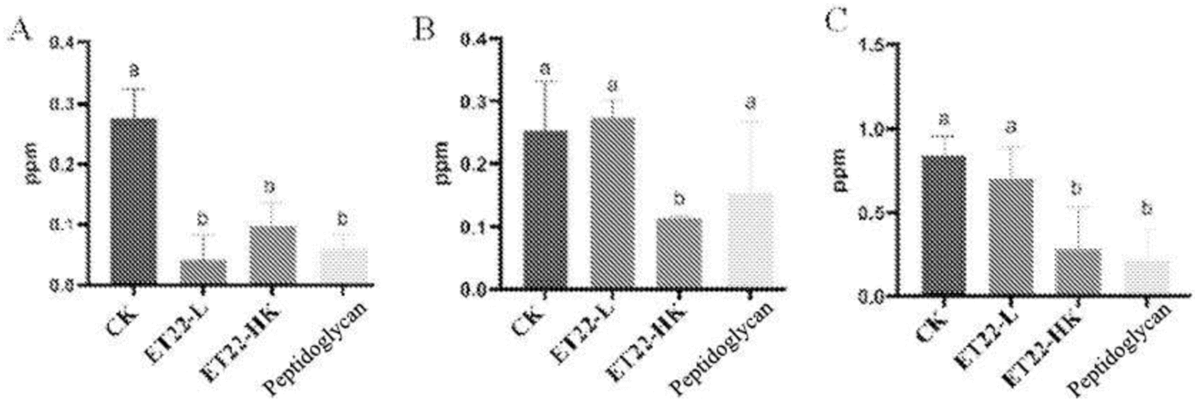


FIG. 43

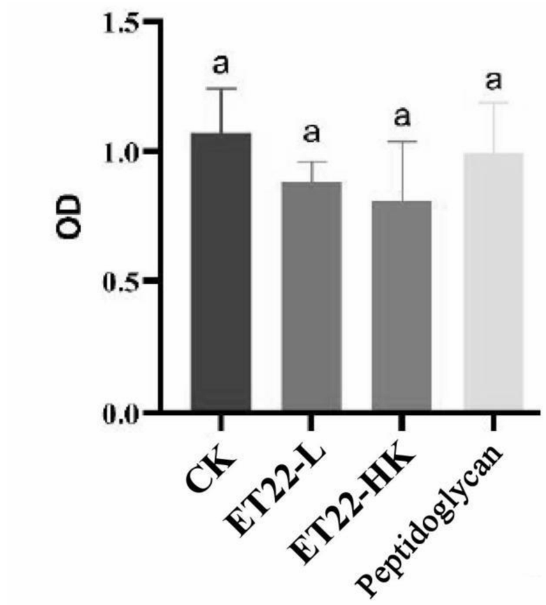


FIG. 44

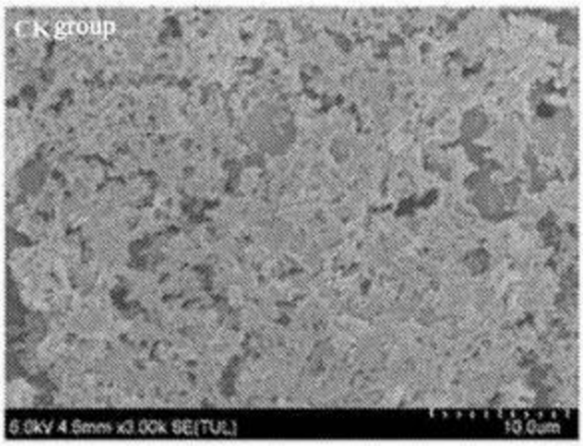


FIG. 45a

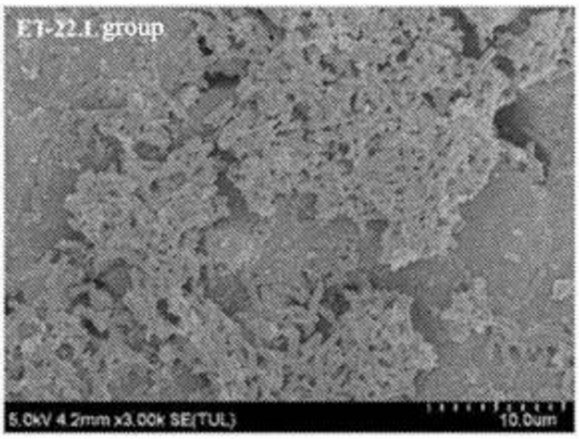


FIG. 45b

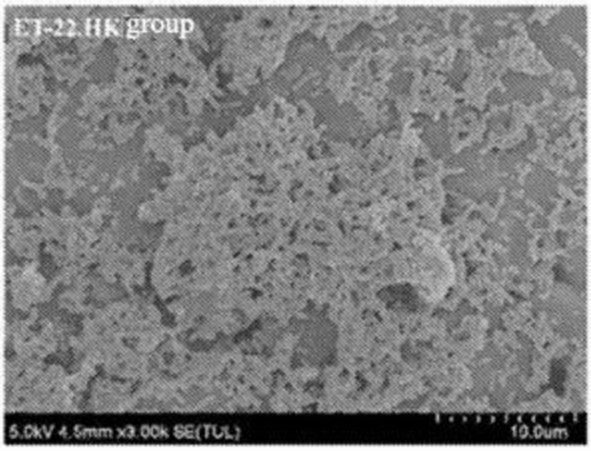


FIG. 45c

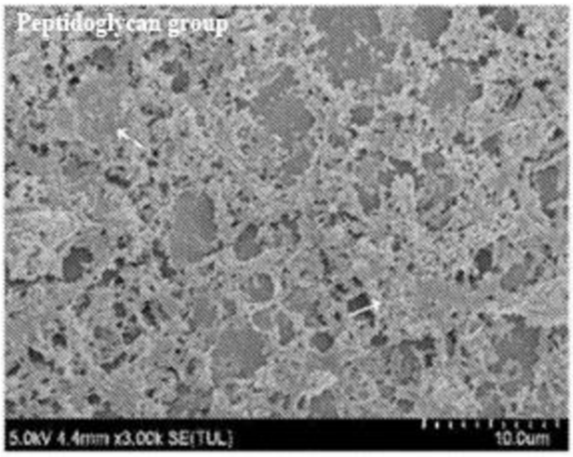


FIG. 45d

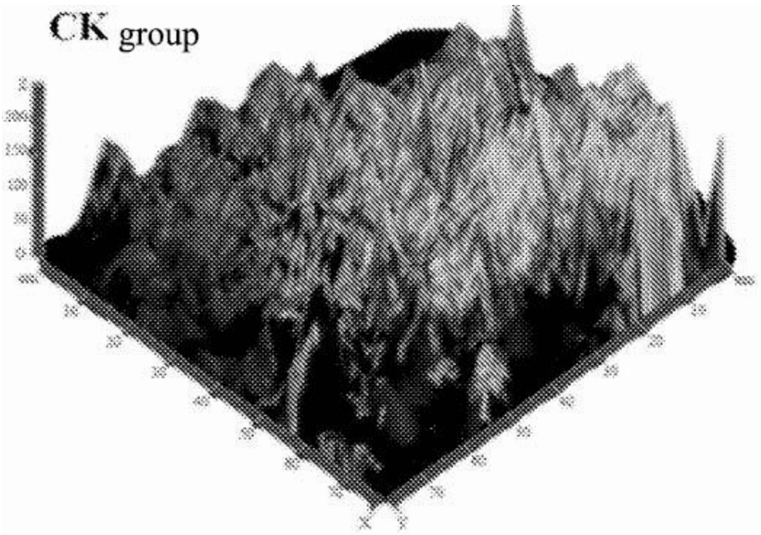


FIG. 46a

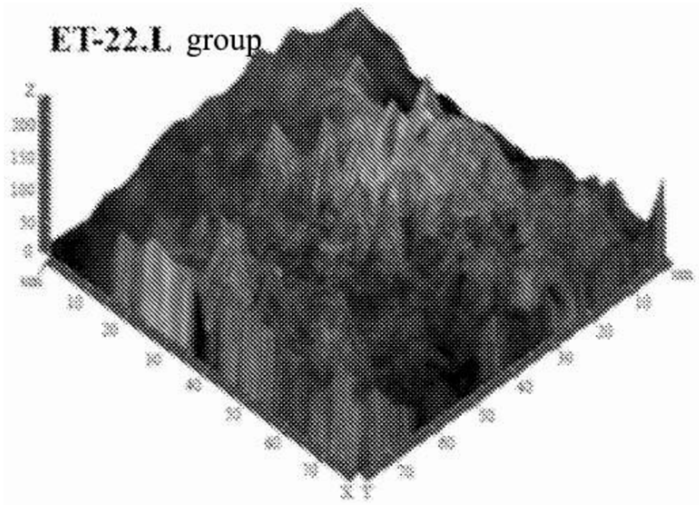


FIG. 46b

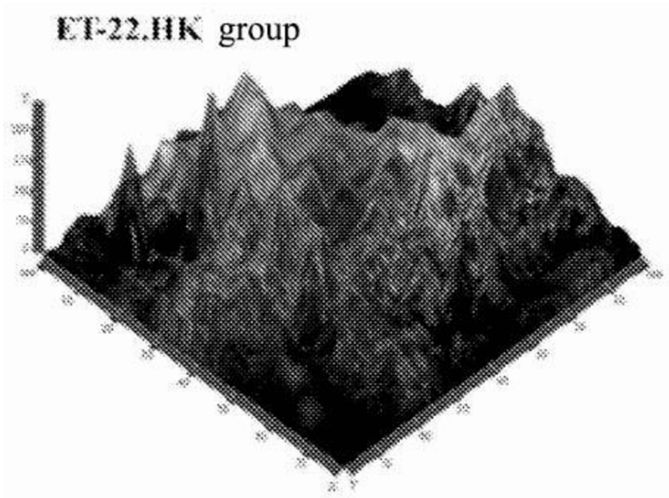


FIG. 46c

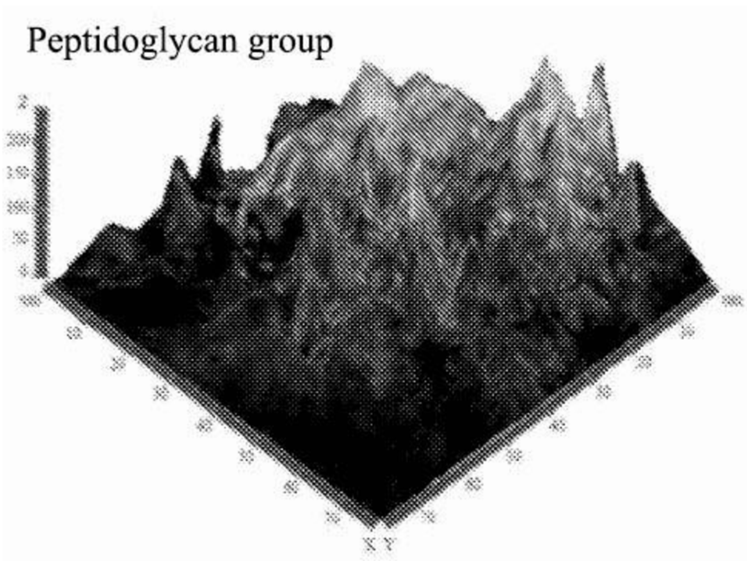


FIG. 46d

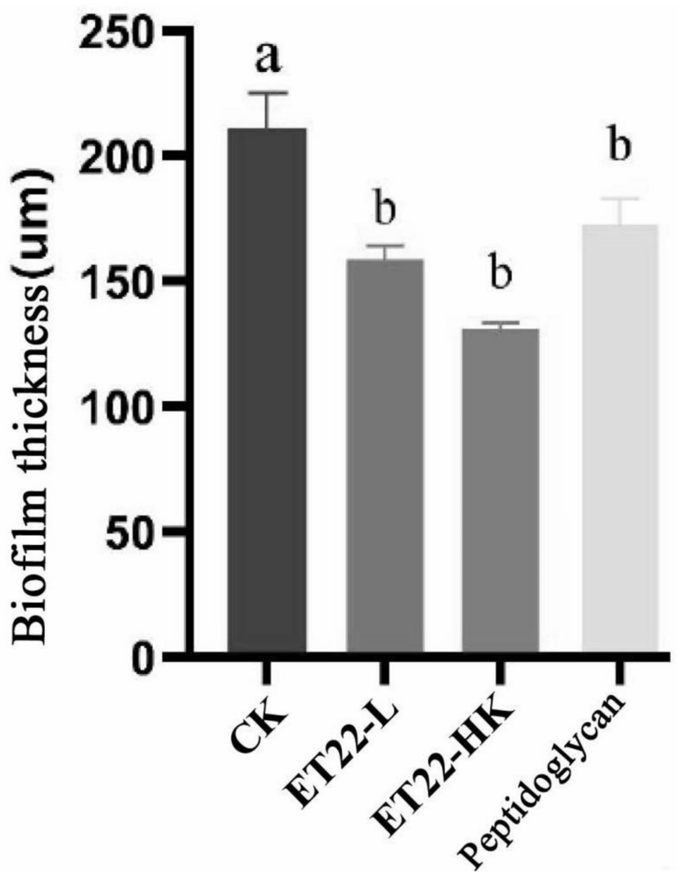
250
200
150
100
50
0
Biofilm thickness(um)
a
b
b
b
CK
ET22-L
ET22-HK
Peptidoglycan

FIG. 47

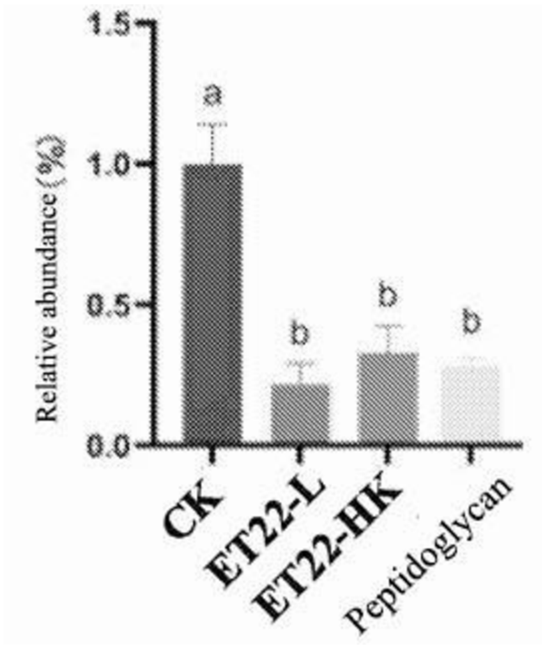
1.5
1.0
0.5
0.0
Relative abundance (%)
a
b
b
b
CK
ET22-L
ET22-HK
Peptidoglycan

FIG. 48

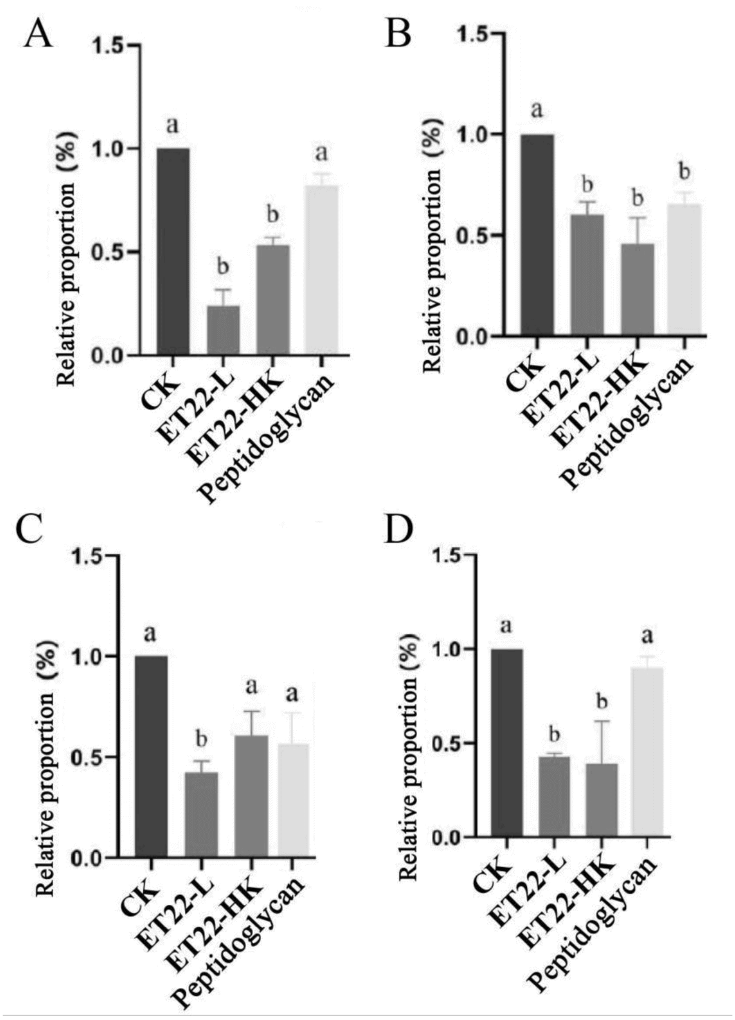
A
B
C
D
Relative proportion (%)
1.5
1.0
0.5
0.0
a
b
CK
ET22-L
ET22-HK
Peptidoglycan

FIG. 49

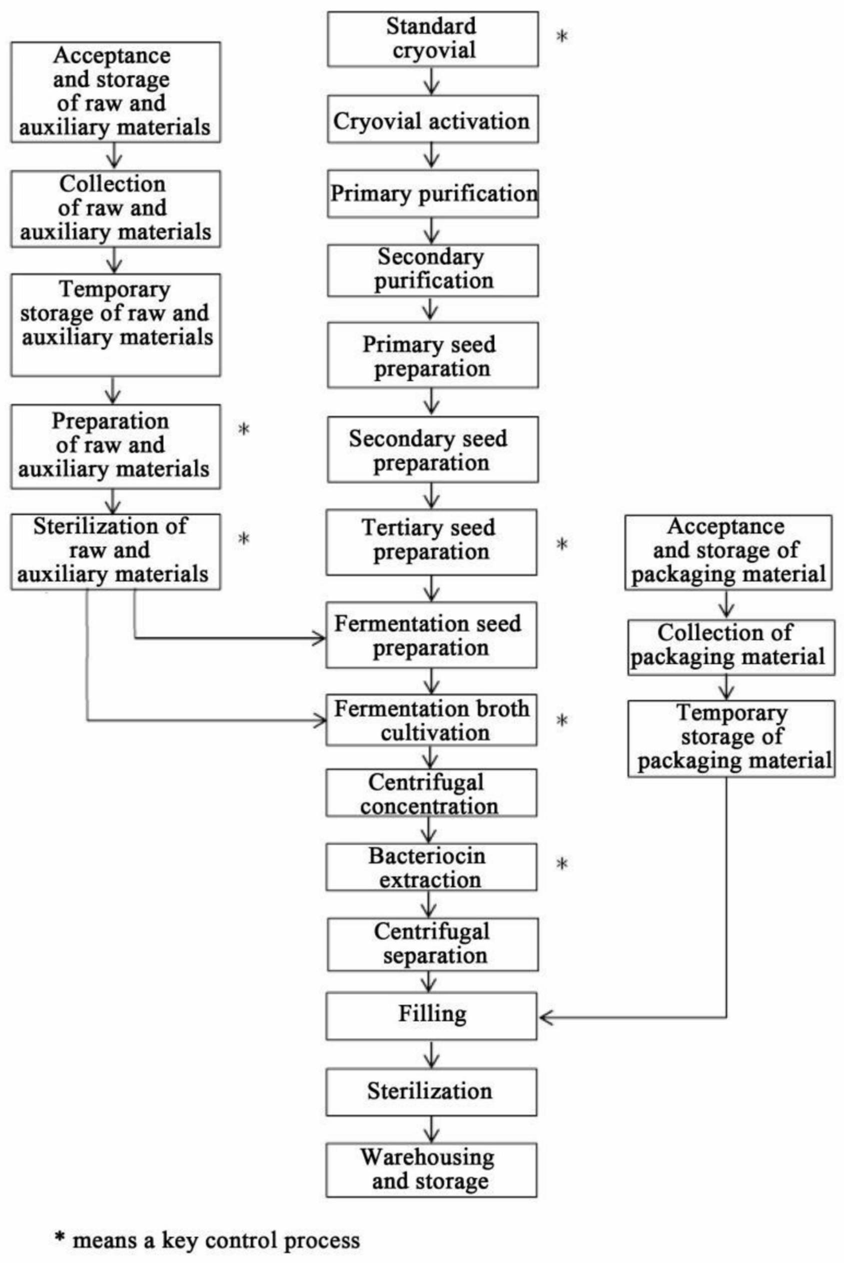
Standard cryovial *
Cryovial activation
Primary purification
Secondary purification
Primary seed preparation
Secondary seed preparation
Tertiary seed preparation *
Fermentation seed preparation
Fermentation broth cultivation *
Centrifugal concentration
Bacteriocin extraction *
Centrifugal separation
Filling
Sterilization
Warehousing and storage
Acceptance and storage of raw and auxiliary materials
Collection of raw and auxiliary materials
Temporary storage of raw and auxiliary materials
Preparation of raw and auxiliary materials *
Sterilization of raw and auxiliary materials *
Acceptance and storage of packaging material
Collection of packaging material
Temporary storage of packaging material
* means a key control process

FIG. 50

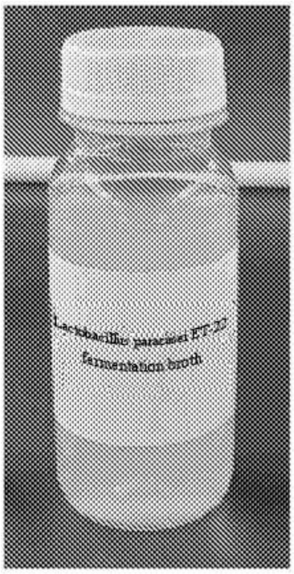
Lactobacillus paracasei ET-22
fermentation broth

FIG. 51

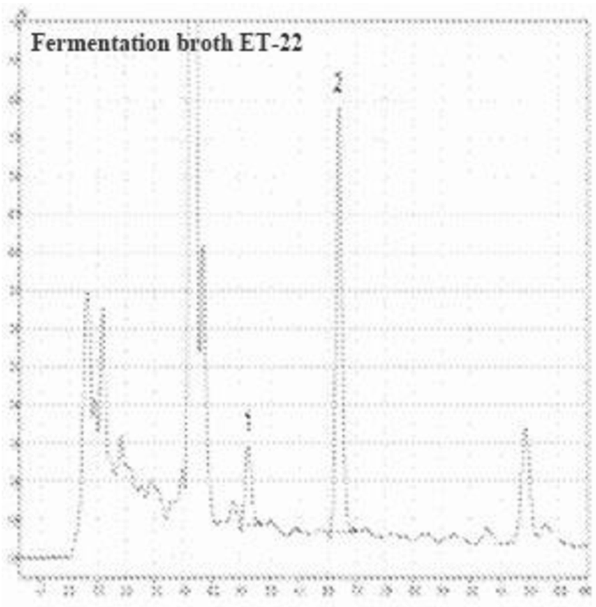
Fermentation broth ET-22

FIG. 52

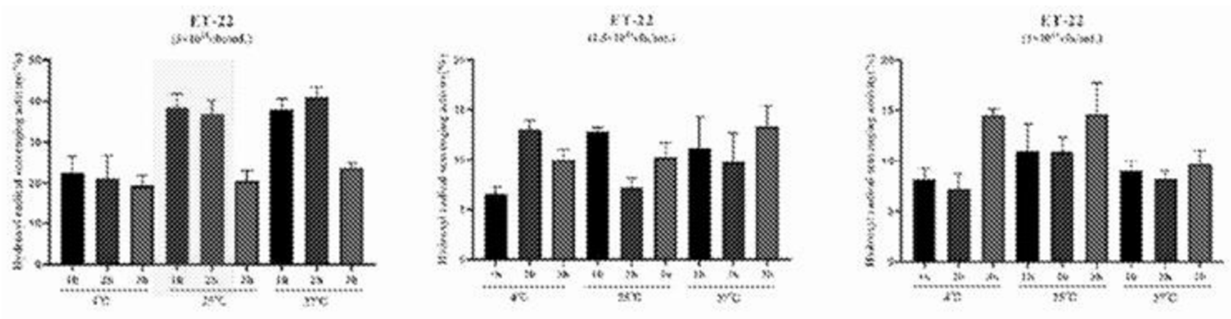

FIG. 53

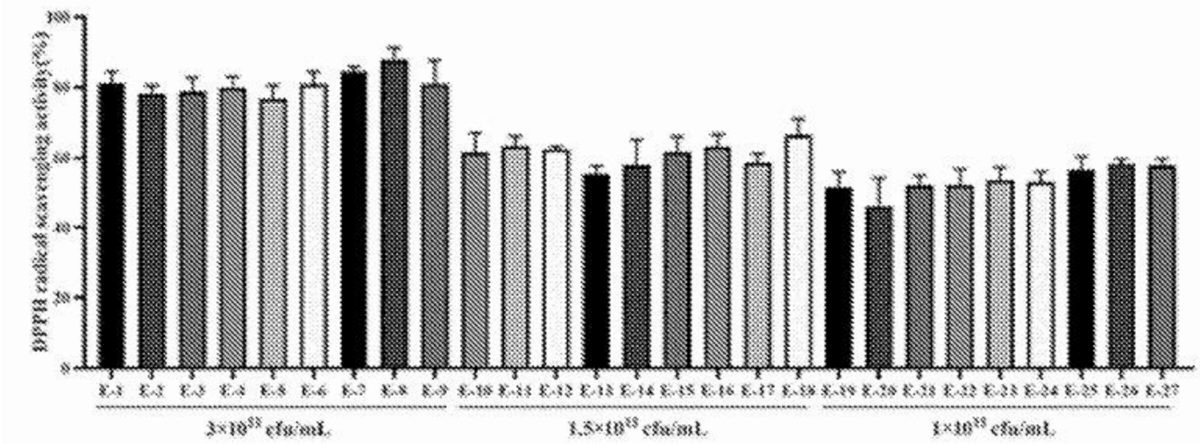
DPPH radical scavenging activity(%)
E-1 E-2 E-3 E-4 E-5 E-6 E-7 E-8 E-9 E-10 E-11 E-12 E-13 E-14 E-15 E-16 E-17 E-18 E-19 E-20 E-21 E-22 E-23 E-24 E-25 E-26 E-27

FIG. 54

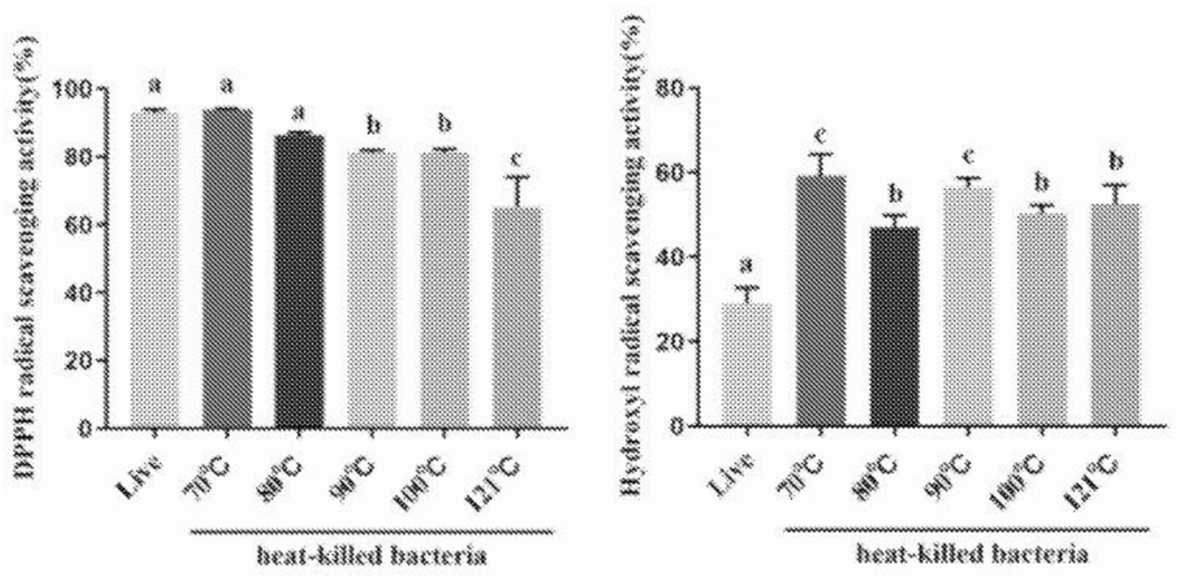
DPPH radical scavenging activity(%)
100
80
60
40
20
0
a
a
a
b
b
c
Live
70°C
80°C
90°C
100°C
121°C
heat-killed bacteria
Hydroxyl radical scavenging activity(%)
80
60
40
20
0
a
c
b
c
b
b
Live
70°C
80°C
90°C
100°C
121°C
heat-killed bacteria

FIG. 55

**INTERNATIONAL SEARCH REPORT**

International application No.
**PCT/CN2024/113210**

**A. CLASSIFICATION OF SUBJECT MATTER**

A61K35/747(2015.01)i; A61K 38/14(2006.01)i; A61K31/7084(2006.01)i; A61K 38/08(2019.01)i; A61K 31/192(2006.01)i; A23C9/13(2006.01)i; A23F3/14(2006.01)i; A23F5/14(2006.01)i; A23G3/36(2006.01)i; A23G4/12(2006.01)i; A61Q11/00(2006.01)i; A61P1/02(2006.01)i

According to International Patent Classification (IPC) or to both national classification and IPC

**B. FIELDS SEARCHED**

Minimum documentation searched (classification system followed by classification symbols)

IPC: A61K, A23C, A23F, A23G, A61Q, A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pubmed, STN on web, NCBI, 内蒙古伊利实业集团股份有限公司, 蓝航莲, 副干酪乳杆菌, Lactobacillus paracasei, ET-22, CGMCC No.15077, 2-羟基-3-苯基丙酸, 苯乳酸, 3-phenyllactic acid, NAD, 烟酰胺腺嘌呤二核苷酸, 抗心律失常肽, Gly Pro Hyp Gly Ala Gly, Antiarrhythmic peptide, 肽聚糖, peptidoglycan, 致臭菌, odorogenic bacteria, 变形链球菌, streptococcus mutans, 口臭, 口气, 口腔, oral, bad breath, oral cavity, halitosis, 抑菌, antibacterial, 53-84-9/rn, 81771-37-1/rn, 828-01-3/rn

**C. DOCUMENTS CONSIDERED TO BE RELEVANT**

| Category* | Citation of document, with indication, where appropriate, of the relevant passages | Relevant to claim No. |
|---|---|---|
| PX | CN 117137952 A (INNER MONGOLIA YILI INDUSTRIAL GROUP CO., LTD.) 01 December 2023 (2023-12-01)<br>claims 1-10 | 1-5, 8-11, 13 |
| PX | CN 117159682 A (INNER MONGOLIA YILI INDUSTRIAL GROUP CO., LTD.) 05 December 2023 (2023-12-05)<br>claims 1-10 | 8-11, 13, 24-28 |
| PX | CN 117137933 A (INNER MONGOLIA YILI INDUSTRIAL GROUP CO., LTD.) 01 December 2023 (2023-12-01)<br>claims 1-10 | 8-17 |
| PX | CN 117126240 A (INNER MONGOLIA YILI INDUSTRIAL GROUP CO., LTD.) 28 November 2023 (2023-11-28)<br>claims 1-10 | 6-13, 22-23 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 November 2024** | **21 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/113210**

**C. DOCUMENTS CONSIDERED TO BE RELEVANT**

| Category* | Citation of document, with indication, where appropriate, of the relevant passages | Relevant to claim No. |
|---|---|---|
| PX | CN 117137895 A (INNER MONGOLIA YILI INDUSTRIAL GROUP CO., LTD.) 01 December 2023 (2023-12-01)<br>claims 1-10 | 8-13, 18-21 |
| Y | CN 110960559 A (INNER MONGOLIA YILI INDUSTRIAL GROUP CO., LTD.) 07 April 2020 (2020-04-07)<br>abstract, and claims 1-38 | 1-5, 8-11, 13, 24-38 |
| X | CN 114191329 A (BAIRUI GLOBAL LIMITED COMPANY) 18 March 2022 (2022-03-18)<br>claims 1, 2 and 11-13, and description, paragraphs 38-42 and 114-115 | 8-13 |
| Y | CN 114191329 A (BAIRUI GLOBAL LIMITED COMPANY) 18 March 2022 (2022-03-18)<br>claims 1, 2 and 11-13, and description, paragraphs 38-42 and 114-115 | 14-17 |
| X | ZHAO, Z. et al. "Postbiotics Derived from L. paracasei ET-22 Inhibit the Formation of S. mutans Biofilms and Bioactive Substances: An Analysis"<br>*Molecules*, Vol. vol. 28, 27 January 2023 (2023-01-27),<br>Article 1236, page 1, Abstract, and page 15, Conclusions | 8-13, 21, 29-38 |
| Y | ZHAO, Z. et al. "Postbiotics Derived from L. paracasei ET-22 Inhibit the Formation of S. mutans Biofilms and Bioactive Substances: An Analysis"<br>*Molecules*, Vol. vol. 28, 27 January 2023 (2023-01-27),<br>Article 1236, page 1, Abstract, and page 15, Conclusions | 1-5, 18-20, 24-28 |
| Y | LIN, Y.W. et al. "Nicotinamide could Reduce Growth and Cariogenic Virulence of Streptococcus Mutans"<br>*Journal of Oral Microbiology*, 23 March 2022 (2022-03-23),<br>Article 2056291, page 1, abstract | 14-17 |
| Y | JEON, Y.M. et al. "The Effects of Oral Microorganism on Oral Malodor"<br>*International Journal of Clinical Preventive Dentistry*,<br>Vol. 11, No. (3), 30 September 2015 (2015-09-30),<br>page 171, Abstract | 1-5, 14-20, 24-28 |
| X | AONUMA, S. et al. "Studies on Heart. XXI. Amino Acid Sequence of Antiarrhythmic Peptide (AAP) Isolated from Atria"<br>*Journal of Pharmacobio-Dynamics*, 31 December 1982 (1982-12-31),<br>page 40, Abstract | 6-10, 12-13 |
| A | AONUMA, S. et al. "Studies on Heart. XXI. Amino Acid Sequence of Antiarrhythmic Peptide (AAP) Isolated from Atria"<br>*Journal of Pharmacobio-Dynamics*, 31 December 1982 (1982-12-31),<br>page 40, Abstract | 22-23 |

Form PCT/ISA/210 (second sheet) (July 2022)

**INTERNATIONAL SEARCH REPORT**

International application No.
**PCT/CN2024/113210**

**Box No. III Observations where unity of invention is lacking (Continuation of item 3 of first sheet)**

This International Searching Authority found multiple inventions in this international application, as follows:

Invention 1: a cell lysate containing a Lactobacillus paracasei ET-22 source that is involved in claims 1-5, 8-11 (in part) and 13 (in part), and the use of the cell lysate.

Invention 2: a composition of peptidoglycan containing a Lactobacillus paracasei ET-22 source that is involved in claims 8-11 (in part), 13 (in part) and 24-28 , and the use of the peptidoglycan.

Invention 3: bacteriocin of Lactobacillus paracasei ET-22 that is involved in claims 29-38, and a preparation method therefor and the use thereof.

Invention 4: a polypeptide of formula I that is involved in claims 6-7, 8-13 (in part) and 22-23 and the use thereof.

Invention 5: a composition containing nicotinamide adenine dinucleotide that is involved in claims 8-13 (in part) and 14-17, and the use of nicotinamide adenine dinucleotide.

Invention 6: a composition containing 2-hydroxy-3-phenylpropionic acid that is involved in claims 8-13 (in part) and 18-20, and the use of 2-hydroxy-3-phenylpropionic acid in freshening breath.

Invention 7: the use of 2-hydroxy-3-phenylpropionic acid involved in claim 21 in reducing the number and thickness of biofilms in an oral cavity.

The cell lysate of a Lactobacillus paracasei ET-22 source in invention 1 contains 2-hydroxy-3-phenylpropionic acid, nicotinamide adenine dinucleotide, and the polypeptide of formula I; however, as the cell lysate is a mixture, considering the content of the 2-hydroxy-3-phenylpropionic acid, nicotinamide adenine dinucleotide and polypeptide of formula I thereof, same may not be used as main components, and other components may further be contained, so that the components of the cell lysate are necessarily different from a single 2-hydroxy-3-phenylpropionic acid, nicotinamide adenine dinucleotide, and polypeptide of formula I. Therefore, the compositions or structures in inventions 1-7 are different or the uses thereof are different, and thus inventions 1-7 do not have a same or corresponding special technical feature and do not comply with PCT Rule 13.2.

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

**INTERNATIONAL SEARCH REPORT**
**Information on patent family members**

International application No.
**PCT/CN2024/113210**

| Patent document cited in search report | Publication date (day/month/year) | Patent family member(s) | Publication date (day/month/year) |
|---|---|---|---|
| CN 117137952 A | 01 December 2023 | None | |
| CN 117159682 A | 05 December 2023 | None | |
| CN 117137933 A | 01 December 2023 | None | |
| CN 117126240 A | 28 November 2023 | None | |
| CN 117137895 A | 01 December 2023 | None | |
| CN 110960559 A | 07 April 2020 | None | |
| CN 114191329 A | 18 March 2022 | None | |

Form PCT/ISA/210 (patent family annex) (July 2022)